Europäisches Patentamt
European Patent Office
Office européen des brevets

(11) Veröffentlichungsnummer: **0 074 070**
**A1**

# (12) EUROPÄISCHE PATENTANMELDUNG

(21) Anmeldenummer: 82108019.9

(22) Anmeldetag: 01.09.82

(51) Int. Cl.³: C 07 D 217/26
C 07 D 209/42, C 07 D 209/52
C 07 D 209/20, C 07 C 127/19
C 07 C 127/15, C 07 C 149/437
C 07 D 333/24, C 07 D 213/55
C 07 D 213/40, C 07 D 231/12

(30) Priorität: 03.09.81 DE 3134933

(43) Veröffentlichungstag der Anmeldung:
16.03.83 Patentblatt 83/11

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt/Main 80(DE)

(72) Erfinder: Henning, Rainer, Dr.
Völklinger Weg 56
D-6000 Frankfurt am Main 71(DE)

(72) Erfinder: Urbach, Jansjörg, Dr.
Le Lavandoustrasse 41
D-6242 Kronberg/Taunus(DE)

(72) Erfinder: Geiger, Rolf, Prof. Dr.
Heinrich-Bleicher-Strasse 33
D-6000 Frankfurt am Main 50(DE)

(72) Erfinder: Teetz, Volker, Dr.
An der Tann 20
D-6238 Hofheim am Taunus(DE)

(72) Erfinder: Schölkens, Bernward, Dr.
Am Fliedergarten 1
D-6233 Kelkheim (Taunus)(DE)

## (54) Harnstoffderivate, Verfahren zu ihrer Herstellung und diese enthaltende Medikamente sowie deren Verwendung.

(57) Verbindungen der Formel I

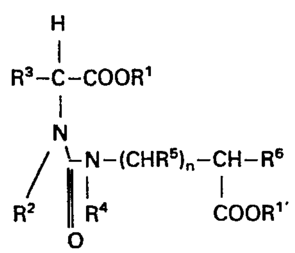

I

in der
n 0 - 3,
$R^1$ und $R^{1'}$ gleich oder verschieden sind, Wasserstoff; Alkyl oder Alkenyl, Phenyl oder Benzyl, jedes gewünschtenfalls substituiert;
$R^2$ Wasserstoff, Alkyl oder Alkenyl;
$R^3$ Wasserstoff, Alkyl, Hydroxyalkyl, Akoxyalkyl oder Aminoalkyl, Alkanoylaminoalkyl, Guanidinoalkyl, Imidazolylalkyl, Indolylalkyl, Mercaptoalkyl oder Alkylthioalkyl, Phenylalkyl, Hydroxyphenylalkyl, Phenoxyalkyl oder Phenylthioalkyl oder $R^2$ und $R^3$ gemeinsam mit den sie tragenden C- und N-Atomen ein gesättigtes oder ungesättigtes 4 bis 8-gliedrigen monocyclischen oder 8- bis 10-gliedrigen bicyclischen Isocyclus oder Heterocyclus, durch Hydroxy, Alkoxy mit 1 bis 3 C-Atomen, Alkyl ggf. mono- oder disubstituiert, $R^4$ Wasserstoff, Alkyl, Alkenyl, Alkadienyl, Alkinyl, Alkeninyl oder Alkadiinyl, Cycloalkyl, Phenyl, Benzyl, Phenethyl oder Phenylpropyl, deren jedes ggf. mono- oder disubstituiert sein kann;
$R^5$ Wasserstoff oder Alkyl, Hydroxy, Alkoxy und
$R^6$ Wasserstoff, ggf. substituiertes Alkyl, Cycloalkyl, Alkenyl, ggf. mono- oder disubstituiertes Phenyl oder Naphthyl, bedeuten
ihre Salze, Verfahren zu ihrer Herstellung und ihre Verwendung als Heilmittel.

EP 0 074 070 A1

0074070
HOECHST AKTIENGESELLSCHAFT HOE 81/F [illegible] [illegible] WS/Rü

Harnstoffderivate, Verfahren zu ihrer Herstellung und diese enthaltende Medikamente sowie deren Verwendung

Gegenstand der Erfindung sind Verbindungen der Formel (I)

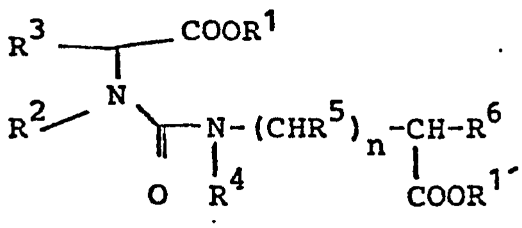

in welcher bedeutet:

n eine ganze Zahl zwischen 0 und 3 inclusiv,

$R^1$ und $R^{1'}$, gleich oder verschieden, Wasserstoff;
Alkyl oder Alkenyl mit 1 - 8 C-Atomen;
Phenyl oder Benzyl, jedes gewünschtenfalls mit Methyl, Halogen, Methoxy oder Nitro substituiert;

$R^2$ Wasserstoff, Alkyl oder Alkenyl mit 1 - 8 C-Atomen;

$R^3$ Wasserstoff;
Alkyl mit 1 - 10 C-Atomen;
Hydroxyalkyl, Alkoxyalkyl oder Aminoalkyl mit je 1 - 5 C-Atomen;
Alkanoylaminoalkyl mit 1 - 7 C-Atomen;
Guanidinoalkyl, Imidazolylalkyl, Indolylalkyl, Mercaptoalkyl oder Alkylthioalkyl mit je 1 - 6 Alkyl-C-Atomen;
Phenylalkyl mit 1 - 5 Alkyl-C-Atomen;
Hydroxyphenylalkyl mit 1 - 5 Alkyl-C-Atomen;
Phenoxyalkyl oder Phenylthioalkyl mit je 1 - 4 Alkyl-C-Atomen
oder $R^2$ und $R^3$ gemeinsam mit den sie tragenden C- und N-Atomen ein gesättigtes oder ungesättigtes 4 - 8 gliedriges monocyclisches oder 8 - 10 gliedriges bicyclisches Ringsystem bilden, das 1 - 2

Sauerstoff-, 1 - 2 Schwefel- und/oder 1 - 4 Stickstoffatome enthalten und durch Hydroxy, Alkoxy mit 1 - 3 C-Atomen, Alkyl mit 1 - 3 C-Atomen oder Phenyl mono- oder disubstituiert sein kann;

$R^4$ Wasserstoff,
Alkyl, Alkenyl, Alkadienyl, Alkinyl, Alkeninyl oder Alkadiinyl mit
1 - 8 C-Atomen,
Cycloalkyl mit 3 - 6 C-Atomen;
Phenyl, Benzyl, Phenethyl oder Phenylpropyl, deren jedes durch
Halogen, Hydroxy, Acetoxy, Carboxy, Carbonamido, Sulfonamido, Nitro, Methyl, Ethyl, Methoxy, Ethoxy oder Methylendioxy mono- oder disubstituiert sein kann;

$R^5$ Wasserstoff oder
Alkyl mit 1 - 5 C-Atomen, Hydroxy, Alkoxy mit
1 - 3 C-Atomen;

$R^6$ Wasserstoff;
Alkyl mit 1 - 12 C-Atomen;
Cycloalkyl mit 3 - 12 C-Atomen;
Alkenyl mit 1 - 12 C-Atomen;
Phenyl oder Naphthyl, deren jedes durch Halogen,
Hydroxy, Acetoxy, Carboxy, Carbonamido, Sulfonamido, Nitro, Methyl, Ethyl, Methoxy, Ethoxy oder Methylendioxy mono- oder disubstituiert sein kann;
durch Halogen, Hydroxy, Alkoxy mit 1 - 3
C-Atomen, Phenoxy, Amino,
Dialkylamino mit 1 - 6 C-Atomen, Alkanoylamino mit 1 - 3 C-Atomen, Mercapto, Alkylthio mit 1 - 3 C-Atomen, Phenylthio, Phenylsulfinyl, Phenylsulfonyl, Phenyl, Biphenylyl, Naphthyl oder Heteroaryl

substituiertes Alkyl mit 1 - 6 C-Atomen, wobei das Phenyl oder Naphthyl seinerseits mit Halogen, Methyl, Ethyl, Methoxy, Ethoxy, Nitro, Amino, Alkylamino, Dialkylamino, Acetylamino, Cyano, Methylendioxy oder Sulfonamido mono- oder disubstituiert und das Heteroaryl durch die genannten Substituenten und zusätzlich durch Phenyl substituiert sein kann.

und deren Salze.

Bevorzugt werden Verbindungen der Formel I, in welcher die Substituenten folgende Bedeutung haben:

n = 0 bis 2

$R^1$ und $R^{1'}$ Wasserstoff, Alkyl oder Alkenyl mit 1 bis 4 C-Atomen, Benzyl, ggf. im Phenylkern mit Methyl, Halogen, Methoxy- oder Nitro substituiert;

$R^2$ Wasserstoff, Alkyl, Alkenyl oder Alkinyl mit 1 bis 5 C-Atomen;

$R^3$ der Rest einer natürlichen Aminosäure, Acetylaminobutyl, Methoxymethyl, Methoxyethyl, Phenoxymethyl, Methylthiomethyl, Methylthioethyl oder Phenylthiomethyl;

$R^2$ und $R^3$ können gemeinsam mit dem sie tragenden Kohlenstoff- bzw. Stickstoffatom Teil eines gesättigten oder ungesättigten 4 bis 8-gliedrigen monocyclischen bzw. 8 bis 10-gliedrigen bicyclischen Ringsystems sein, das außer Kohlenstoff auch noch jeweils ein Sauerstoff-, Schwefel und/oder 1 bis 3 Stickstoffatome enthalten kann, als solche Ringsysteme kommen in Betracht: Azetidin, Dihydropyrrol, Pyrrolidin, Piperidin, beide ggf. durch Methoxy, Ethoxy, Methyl, Ethyl, Phenyl mono- oder disubstituiert, Hexahydroazepin, Octahydroazocin, Morpholin, N'-Alkylpiperazin mit 1 bis 3 C-Atomen, N'- Phenylpiperazin,

Thiazolidin, ggf. in 2-Stellung durch Methyl, Ethyl, Phenyl, Hydroxyphenyl oder Methoxyphenyl substituiert, als monocyclische, Tetrahydrochinolin, Tetrahydroisochinolin, Decahydrochinolin, Decahydroisochinolin, Dihydroindol, Octahydroindol, 2-Azabicyclo[3.3.0]octan, alle ggf. mono- oder disubstituiert durch Methyl oder Methoxy, Tetrahydroimidazolo-[2,3-c]pyridin, Tetrahydrothieno-[2,3-c]pyridin, Tetrahydrothieno-[3,2-c] pyridin, Tetrahydrothieno-[3,4-c]pyridin als bicyclische Systeme;

$R^4$ Wasserstoff; geradkettiges oder verzweigtes Alkyl, Alkenyl oder Alkinyl mit 1 bis 5 C-Atomen; Cycloalkyl wie Cyclopropyl, Cyclobutyl, Cyclopentyl, Cyclohexyl; Phenyl; Benzyl; Phenethyl;

$R^5$ Wasserstoff, Methyl, Ethyl, Hydroxy, Methoxy, Benzyl;

$R^6$ Wasserstoff; Alkyl mit 1 bis 8 Kohlenstoffatomen oder Phenyl, das durch Methyl, Halogen, Methoxy, Acetoxy, Nitro mono- oder disubstituiert sein kann; substituiertes Alkyl mit 1 bis 4 C-Atomen, wobei als Substituenten in Betracht kommen:
Halogen, Hydroxy, Methoxy, Ethoxy, Phenoxy, Amino, Methylamino, Dimethylamino, Anilino, Acetylamino, Benzamido, Mercapto, Phenylthio, Phenylsulfinyl, Phenylsulfonyl;
Phenyl, ggf. durch Halogen, Methyl, Ethyl, Methoxy, Ethoxy, Nitro, Amino, Methylamino, Dimethylamino, Acetylamino, Cyano, Methylendioxy, Sulfonamido mono- oder disubstituiert, Biphenylyl,
Heteroaryl, wie Pyridyl, Thienyl, Indolyl, Benzthienyl, Imidazolyl, Pyrazolyl und Thiazolyl, ggf. durch Halogen, Methyl, Methoxy und Phenyl substituiert.

Besonders bevorzugt werden Verbindungen der Formel (I), in welcher die Substituenten folgende Bedeutung haben:

n = 0 oder 1

$R^1$ und $R^{1'}$ Wasserstoff, Methyl, Ethyl, n-Butyl, t-Butyl, Benzyl, p-Nitrophenyl

$R^2$ Wasserstoff, Methyl, Ethyl, n-Butyl

$R^3$ der Rest einer natürlichen Aminosäure oder Acetylaminobutyl, Methoxymethyl, Methoxyethyl, Phenoxymethyl, Methylthiomethyl, Methylthioethyl, Phenylthiomethyl;

$R^2$ und $R^3$ können gemeinsam mit dem sie tragenden Kohlenstoff- bzw. Stickstoffatom Teil eines gesättigten oder ungesättigten 5 bis 7 gliedrigen monocyclischen bzw. 8 bis 10-gliedrigen bicyclischen Ringsystems sein, daß außer Kohlenstoff- auch noch jeweils ein Sauerstoff- oder Schwefelatom und/oder 1 bis 2 Stickstoffatome enthalten kann; als solche Ringsysteme kommen in Betracht:
Dihydropyrrol; Pyrrolidin, Piperidin, beide ggf. durch Methoxy, Methyl oder Phenyl substituiert, Hexahydroazepin, Thiazolidin, ggf. durch Methyl, Phenyl oder Hydroxyphenyl in 2-Stellung substituiert, als monocyclische,
Tetrahydroisochinolin, Decahydroisochinolin, Dihydroindol, Octahydroindol, 2-Azabicyclo[3.3.0]octan, alle ggf. durch Methyl oder Methoxy mono- oder disubstituiert, Tetrahydroimidazolo-[2,3-c]pyridin, Tetrahydrothieno-[2,3-c]-pyridin, Tetrahydrothieno-[3,2-c]-pyridin, Tetrahydrothieno-[3,4-c]-pyridin als bicyclische Systeme.

$R^4$ Methyl, Ethyl, n-Propyl, n-Butyl, Isopropyl, Isobutyl, Cyclopropyl, Cyclobutyl, Allyl, Butenyl, Propargyl, Butinyl, tert. Butyl.

$R^5$ Wasserstoff, Methyl, Benzyl.

$R^6$ Wasserstoff, geradkettiges oder verzweigtes Alkyl oder Alkenyl mit 1 bis 6 C-Atomen oder Cycloalkyl mit 3 - 6 C-Atomen; substituiertes Alkyl mit 1 bis 3 C-Atomen, wobei als Substutenten in Betracht kommen:

Methoxy, Ethoxy, Phenoxy, Dimethylamino, Anilino, Benzamido, Phenylthio, Phenylsulfinyl, Phenylsulfonyl, Phenyl, ggf.durch Halogen, Methyl, Methoxy, Nitro, Amino, Methylamino, Dimethylamino, Acetylamino, Cyano, Methylendioxy mono- oder disubstituiert;
Biphenylyl;
Heteroaryl wie Pyridyl, Thienyl, Indolyl, Benzthienyl, Imidazolyl, Thiazolyl, ggf. durch Chlor, Methyl, Methoxy oder Phenyl substituiert.

Hervorzuheben sind Verbindungen der Formel I, in der n = 1, $R^1$ Wasserstoff, $R^2$ und $R^3$ gemeinsam mit den sie tragenden C- und N-Atomen das 1,2,3,4-Tetrahydroisochinolin-System, das Octahydroindol-System oder das 2-Azabicyclo[3.3.0]octan-System, $R^4$ Ethyl, $R^5$ Wasserstoff und $R^6$ ß-Phenylethyl bedeuten.

Die Verbindungen der Formel I enthalten mehrere asymmetrische C-Atome, sie liegen daher in enantiomeren und diastereomeren Formen vor. Die Erfindung umfaßt die reinen Isomeren sowie deren Gemische. Bevorzugt sind die Verbindungen, die an dem Kohlenstoffatom, das den Substituenten $R^3$ trägt, die (S)-Konfiguration aufweisen. Besonders bevorzugt sind Verbindungen, die sowohl an dem den Substituenten $R^3$ als auch an dem die $COOR^{1'}$-Gruppe tragenden Kohlenstoffatom, jeweils die (S)-Konfiguration aufweisen. In Verbindungen der Formel I, in der $R^2$ und $R^3$ gemeinsam mit den sie tragenden C- und N-Atomen für ein gesättigtes

bicyclisches Ringsystem mit Kohlenstoffatomen als Brückenkopfatome stehen, ist die cis-Konfiguration mit einer endoständigen Orientierung der $COOR^1$-Gruppe zum bicyclischen Ringsystem bevorzugt. Besonders bevorzugte bicyclische Ringsysteme sind endo-cis-Octahydroindol und endo-cis-2-Azabicyclo[3.3.0]octan.

Die Isomeren können beispielsweise durch Kristallisation geeigneter Salze, wie der Cyclohexyl- bzw. Dicyclohexylaminsalze oder durch Chromatographie an Kieselgel oder Ionenaustauschern rein dargestellt werden. Gegebenenfalls werden die Trennungen an geeigneten Vorstufen durchgeführt.

Falls die Verbindungen der Formel I sauren Charakter haben, umfaßt die Erfindung die freien Säuren, deren Alkali- und Erdalkalisalze sowie die Salze mit pharmazeutisch unbedenklichen Aminen wie Cyclohexylamin oder Dicyclohexylamin und basischen Aminosäuren wie Lysin und Arginin.

Ein weiterer Gegenstand der Erfindung ist ein Verfahren zur Herstellung der Verbindungen der Formel I. Das Verfahren ist dadurch gekennzeichnet, daß man einen Aminosäureester der Formel II

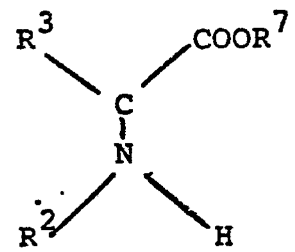

(II)

in der $R^7$ die gleiche Bedeutung wie $R^1$ hat, jedoch nicht Wasserstoff ist; mit Phosgen und danach mit einer Verbindung der Formel IV

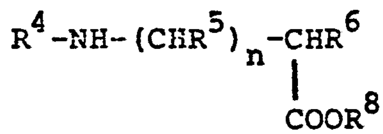

(IV)

in der $R^8$ eine der Bedeutungen von $R^7$ hat, umsetzt,

oder eine Verbindung der Formel IV mit Phosgen und danach mit einer Verbindung der Formel II umsetzt,

und gegebenenfalls das erhaltene Produkt einer Hydrolyse unterwirft.

Bei der zuerst genannten Verfahrensvariante wird eine Verbindung der Formel II in welcher $R^7$ die gleiche Bedeutung wie $R^1$ in Formel I hat, jedoch nicht Wasserstoff ist, mit Phosgen zu dem N-Chlorcarbonylderivat der Formel (III) umgesetzt.

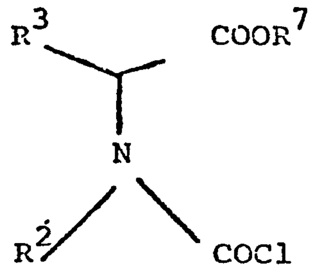

(III)

In Fällen, in denen $R^2$ Wasserstoff bedeutet, kann sich bei dieser Reaktion, vor allem bei erhöhter Temperatur, ein Isocyanat der Formel (III-a) bilden.

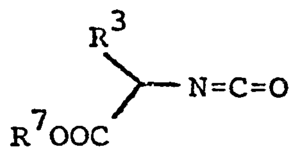

(III-a)

Die Verbindung der Formel (III) oder (III-a) wird mit einer Verbindung der Formel (IV), in welcher $R^8$ eine der Bedeutungen von $R^7$ in Formel (II) hat, zu einer Verbindung der Formel (I-a) umgesetzt

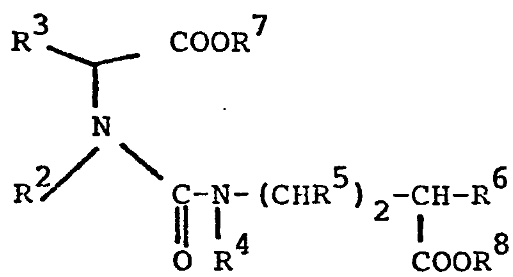

(I-a)

In Fällen, in denen $R^7$ und $R^8$ Alkyl oder Phenyl bedeuten, kann gewünschtenfalls eine Verbindung der Formel (I-a) zu einer Verbindung der Formel (I-b) hydrolysiert werden.

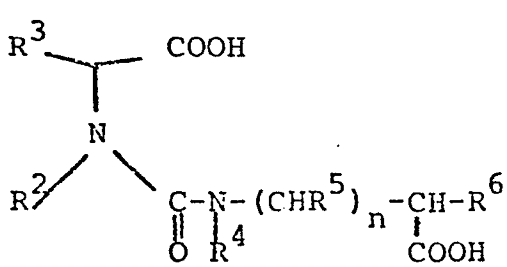

(I-b)

Falls $R^7$ in Formel (I-a) Benzyl oder 4-Nitrobenzyl bedeutet, kann man eine Verbindung der Formel (I-a) durch Hydrogenolyse in eine Verbindung der Formel (I-c) überführen.

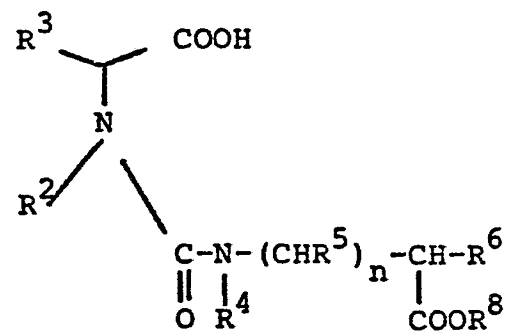

(I-c)

Die Umsetzung der Verbindung der Formel (II) mit Phosgen wird in einem aprotischen organischen Lösungsmittel mit oder ohne Zusatz eines Säurefängers durchgeführt; als Säurefänger kommen basische Verbindungen, insbesondere organische Stickstoffbasen z.B. Triethylamin, Tripropylamin, N-Methylmorpholin, Pyridin und ähnliche in Betracht. Als Lösungsmittel sind beispielsweise Methylenchlorid, Chloroform, Tetrahydrofuran und Dioxan geeignet. Die Reaktion wird bei tiefer bis leicht erhöhter Temperatur, im allgemeinen zwischen - 50°C und + 40°C, vorzugsweise bei - 30°C bis 0°C durchgeführt.
Die Reaktion einer Verbindung der Formel (III) mit einer Verbindung der Formel (IV) erfolgt unter ähnlichen Bedingungen, jedoch bei einer etwas höheren Temperatur, etwa von 0°C bis 80°C, vorzugsweise 30°C bis 50°C. Als Lösungsmittel ist außer den genannten auch Dimethylformamid gut geeignet.

Die Umsetzung einer Verbindung der Formel (IV) mit einem Isocyanat der Formel (III-a) wird in entsprechender Weise durchgeführt.

Die Hydrolyse einer Verbindung der Formel (I-a) zu einer Verbindung der Formel (I-b) kann auf verschiedenen Wegen erfolgen. In Fällen, in denen in Formel (I-a) $R^7$ und $R^8$ Alkyl, jedoch nicht t-Butyl bedeuten, kann die Umsetzung vorteilhaft mit einem Alkalihydroxid oder -carbonat in einem Gemisch aus Wasser und einem niederen Alkohol durchgeführt werden. Als Temperatur ist 0°C bis 100°C geeignet, vorzugsweise 20°C bis 40°C.
In Fällen, in denen $R^7$ und $R^8$ t-Butyl bedeuten, führt man die Umsetzung mit Hilfe einer Säure, vorzugsweise einer starken Säure wie Trifluoressigsäure, Salzsäure oder Schwefelsäure ohne Zugabe eines Lösungsmittels oder in Methanol oder Ethanol bei 0° bis 80°C, vorzugsweise bei 20°C bis 40°C durch.
In Fällen, in denen entweder $R^7$ oder $R^8$ t-Butyl und der andere Rest Alkyl oder Phenyl bedeutet, kann man auch die oben beschriebenen Verfahren sequentiell in beliebiger Reihenfolge anwenden. Die katalytische Hydrogenolyse einer Verbindung der Formel (I-a), worin $R^7$ Benzyl oder 4-Nitrobenzyl bedeutet, kann in einem niederen Alkohol als Lösungsmittel unter Zusatz eines Katalysators herbeigeführt werden.

Als Katalysatoren für die Hydrogenolyse kommen Edelmetallkatalysatoren wie Palladiumschwarz, Palladium auf Kohle oder Platindioxyd in Betracht. Die Reaktion wird bei leicht erhöhter Temperatur, etwa bei 20°C bis 80°C, vorzugsweise bei 20°C bis 40°C und unter leicht erhöhtem Wasserstoffdruck, etwa 1 bis 50 atm. durchgeführt, vorzugsweise bei 1 bis 8 atm.

Analog den zur Herstellung von Verbindungen der Formel (III) angegebenen Verfahren kann man auch eine Verbindung der Formel (IV) mit Phosgen zu einer Verbindung der Formel (V),

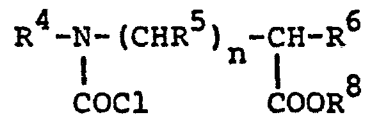

(V)

umsetzen.

In Fällen, in denen $R^4$ Wasserstoff bedeutet, kann sich bei dieser Reaktion ein Isocyanat der Formel (V-a) bilden, besonders bei erhöhter Temperatur.

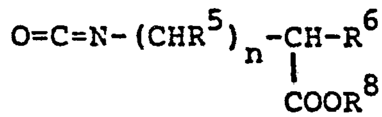

(V-a)

Eine Verbindung der Formel (V) bzw. (V-a) wird anschließend mit einer Verbindung der Formel (II) unter den oben für die Herstellung von Verbindungen der Formel (I-a) beschriebenen Bedingungen, zu einer Verbindung der Formel (I-a) umgesetzt.

Die für dieses Verfahren als Ausgangsstoffe benötigten Aminosäureester der Formel (II) werden aus den entsprechenden Aminosäuren nach üblichen Methoden hergestellt (siehe die in Houben/Weyl/Müller, Methoden der Organischen Chemie, Bd. 15/1, S. 315-370 aufgeführten Methoden). Sofern die entsprechenden Aminosäuren nicht in der Natur vorkommen, sind sie synthetisch in der Regel leicht zugänglich.
Hexahydroazepin-2-carbonsäure und deren höhere Homologe erhält man aus dem Lactam entsprechender Ringgröße durch Chlorierung und Favorskii-Reaktion mit Kalium-tert.-butylat. (J. Med. Chem. 14, 501 (1971)).
Tetrahydroisochinolin-3-carbonsäure und ihre substituierten Derivate sind durch Pictet-Spengler-Reaktion aus den entsprechenden Phenylalaninderivaten und Formaldehyd leicht zugänglich (J. Amer. Chem. Soc. 70, 180 (1948). Dihydroindol-2-carbonsäure und

deren substituierte Derivate werden nach Aust. J. Chem. 20, 1935 (1967) hergestellt.
Aus beiden erhält man durch Druckhydrierung über einem Rhodiumkatalysator die entsprechenden Decahydroisochinolin- bzw. Octahydroindol-Derivate.
Ebenfalls durch Pictet-Spengler-Cyclisierung mit Formaldehyd gewinnt man Tetrahydroimidazo-[2,3-c]-pyridin-carbonsäure aus Histidin (Hoppe-Seylers Z. physiol. Chem. 284, 131 (1949)) und die Thienopyridin-Derivate aus den entsprechenden Thienoalaninen (Heterocycles 16, 35 (1981)).
In 2-Stellung substituierte Thiazolidin-5-carbonsäuren erhält man leicht durch Ringschlußreaktion aus Cystein und dem entsprechenden Aldehyd (Jap. Pat. 5 5011-547).

Die Ausgangsstoffe der Formel (IV-a) (entspricht Formel (IV) mit n=O),

$$R^4\text{-NH-}\underset{\substack{|\\ COOR^8}}{CH}\text{-}R^6 \qquad \text{(IV-a)}$$

erhält man durch Veresterung der entsprechenden α-Aminosäuren unter üblichen Bedingungen (siehe oben).
Ausgangsstoffe der Formel (IV-b) (entspricht Formel (IV) mit n=1)

$$R^4\text{-NH-CHR}^5\text{-}\underset{\substack{|\\ COOR}}{CH}\text{-}R^6 \qquad \text{(IV-b)}$$

erhält man durch Addition eines primären Amins der Formel (VI)

$$R^4\text{-}NH_2 \qquad \text{(VI)}$$

an ein α-Alkylencarboxylat der Formel (VII)

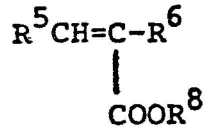

(VII)

Die α-Alkylencarboxylate der Formel (VII) sind aus den entsprechenden alkylierten Malonsäurehalbestern der Formel (VIII),

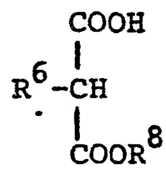

(VIII)

durch Mannich-Reaktion mit Formaldehyd und Diethylamin leicht zugänglich (Arch. Pharm. 314, 197 (1981)).

Die neuen Verbindungen der Formel (I) besitzen eine langandauernde, intensive blutdrucksenkende Wirkung. Sie entfalten diese Wirkung durch Hemmung des Angiotensin-Converting-Enzyms (ACE). Dieses Enzym wandelt das Decapeptid Angiotensin I in das pressorisch wirksame Octapeptid Angiotensin II um; die Dysregulation dieser Enzymreaktion ist ein auslösender Faktor von verschiedenen Formen der Hypertonie bei Säugern und Menschen. Weiterhin inaktiviert das ACE durch Abbau das vasodepressorisch wirksame Bradykinin; diese Inaktivierung wird durch die neuen Verbindungen ebenfalls inhibiert. Von verschiedenen Gruppen wurden in der letzten Zeit Verbindungen beschrieben, die wirksame Inhibitoren des ACE sind (Übersicht z.B. J. Med. Chem. 24, 355 (1981)). Die neuen Verbindungen konkurrieren vorteilhaft mit den dort beschriebenen Inhibitoren. Sie inhibieren in vitro das Converting-Enzyme mit $IC_{50}$-Werten von $5 \times 10^{-9}$ bis $10^{-6}$ mol/l, in vivo an normotonen Ratten wird der durch Injektion von Angiotensin I hervorgerufene Pressorreflex ab einer

Dosis von 0,1 mg/kg bei intravenöser Gabe langanhaltend inhibiert.
Aufgrund dieser Eigenschaften können die neuen Verbindungen und ihre physiologisch verträglichen Salze zur Bekämpfung des Bluthochdrucks verschiedener Genese für sich allein oder in Kombination mit anderen blutdrucksenkenden, gefäßerweiternden oder diuretisch wirkenden Verbindungen angewandt werden. Sie können entweder allein oder mit physiologisch verträglichen Hilfs- oder Trägerstoffen vermischt angewandt werden.

Die Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige alkoholische oder ölige Suspensionen oder wäßrige alkoholische öder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze gewünschtenfalls, mit den dafür üblichen Substanzen wie Lösungsvermittler, Emulgatoren oder weitere Hilfsstoffe in Lösungen, Suspensionen oder Emulsionen gebracht. Als Lösungsmittel für die neuen aktiven Verbindungen und die entsprechenden physiologisch verträglichen Salze kommen

z.B. in Frage: Wasser, physiologische Kochsalzlösungen oder Alkohole, z.B. Äthanol, Propandiol oder Glycerin, daneben auch Zuckerlösungen wie Glukose- oder Mannitlösungen, oder auch eine Mischung aus den verschiedenen genannten Lösungsmitteln.

Die tägliche Dosis für Verbindungen der Formel (I) und ihre Salze beträgt 20 mg bis 3 g, vorzugsweise 50 mg bis 1 g pro Patient. Toxische Wirkungen der Substanzen wurden bisher nicht beobachtet.

Soweit kein anderes Verfahren angegeben ist, werden die in den folgenden Beispielen beschriebenen Verbindungen zur Analyse und biologischen Bestimmung einer HPLC-Reinigung unterworfen.
Da alle erfindungsgemäßen Verbindungen nach nur zwei Methoden hergestellt werden, sollen im folgenden diese beiden Verfahren in vier Beispielen ausführlich dargestellt werden. Die weiteren analog hergestellten Derivate sind in einer Tabelle mit ihren NMR-Daten zusammengestellt.

Beispiel 1:

(3S)-⟨N-Isopropyl-N-(4-phenyl-2-carbethoxy-butyl)-carbamoyl⟩-1,2,3,4-tetrahydroisochinolin-3-carbonsäure

1.1. 1.2.3.4-Tetrahydroisochinolin-3-carbonsäure-benzylester Benzolsulfonat

53 g (0,3 Mol) 1,2,3,4-Tetrahydroisochinolin-3-carbonsäure,,150 ml Benzylalkohol und 53,3 g (0,33 Mol) Benzolsulfonsäure werden 1 h auf 140°C erhitzt, dann mit 100 ml Toluol versetzt und am Wasserabscheider gekocht, bis die theoret. Menge Wasser sich gebildet hat. Danach wird das Lösungsmittel entfernt, der Rückstand mit Ether digeriert, der Niederschlag abgesaugt und aus Ethanol/Ether umkristallisiert.
F. 165 - 166°C
$[\alpha]_D$ -42,1° c=1 (DMF)

1.2. N-Chlorcarbonyl-1,2,3,4-tetrahydroisochinolin-3-carbonsäure-benzylester

4,25 g des obigen Benzylester-Benzolsulfonats werden in 100 ml gesättigter Natriumbicarbonatlösung gelöst, mit Methylenchlorid extrahiert, über Natriumsulfat getrocknet, das Lösungsmittel entfernt. Der Rückstand (2,67 g/0,01 Mol) wird zusammen mit 1,5 g (0,o15 Mol) Triethylamin in 10 ml trockenem Methylenchlorid gelöst. Die Lösung wird bei -20° bis - 30°C zu 11,5 ml einer

15%igen Phosgenlösung in Methylenchlorid getropft, 30 min. gerührt, dann zur Trockne eingeengt.

1.3 Phenethylmalonsäurediethylester

Aus 6,5 g Natrium und 130 ml absolutem Ethanol wird eine Natriumethylatlösung bereitet, 45 g Malonsäurediethylester und 30 g Phenethylbromid werden gemischt und unter Eiskühlung zugetropft. Anschließend wird 6 Std. am Rückfluß gekocht und über Nacht abkühlen lassen. Die Hauptmenge Ethanol wird im Vakuum entfernt, der Rückstand in Wasser aufgenommen, mit Ether extrahiert, über $Na_2SO_4$ getrocknet, eingeengt und destilliert.
$Sdp_{0,1}$ 90°C

1.4 2-Methylen-4-phenylbuttersäureethylester

26,6 g (0,1 Mol) Phenethylmalonsäurediethylester werden im Verlauf einer Stunde unter Rühren zu 5,6 g KOH in 65 ml abs. Ethanol getropft, 15 Std. bei Raumtemperatur gerührt, dann 5 min. aufgekocht. Das Ethanol wird im Vakuum entfernt, Eiswasser zugesetzt und mit Ether extrahiert. Die wässrige Phase wird mit 2 N Salzsäure angesäuert und mit Ether extrahiert. Der zweite Extrakt wird getrocknet und eingeengt, dann mit 8,8 ml Diethylamin neutralisiert. 12 ml 30%iger Formaldehydlösung werden zugesetzt, 3 Std. gerührt, dann mit Kaliumcarbonat gesättigt, mit Ether extrahiert, Extrakt mit verdünnter Salzsäure gewaschen, getrocknet und eingeengt.

NMR ($CDCl_3$) $\delta$ = 7,05 s (5H); 6,02 s (1H); 5,4 s (1H); 4,15 q (4H); 2,65 bs (4H); 1,25 t (3H)

1.5 N-Isopropyl-N-(2-carbethoxy-4-phenyl-butyl)-amin

14,2 g 2-Methylen-4-phenylbuttersäureethylester und 5,7 ml Isopropylamin wurden in 25 ml absolutem Ethanol 12 Tage bei Raumtemperatur gerührt, das Lösungsmittel entfernt, in 1 N Salzsäure aufgenommen, mit Ether extrahiert, die wässrige Phase mit Natriumcarbonat alkalisch gestellt, mit Ether extrahiert, getrocknet, eingeengt.
NMR ($CDCl_3$): $\delta$ = 7,1 s (5H), 4,1 q (2H); 3,0-2,2 m (6H); 2,05 - 1,5 m (3H); 1,22 t (3H); 1,0 d (6H).

1.6 (3S)-(N-Isopropyl-N-(4-phenyl-2-carbethoxy-butyl)-carbamoyl)-1,2,3,4-tetrahydroisochinolin-3-carbonsäure-benzylester

Der rohe N-Chlorcarbonyl-1,2,3,4-tetrahydroisochinolin-3-carbonsäure-benzylester (aus 1.2) wird in 10 ml $CH_2Cl_2$ aufgenommen, eine Lösung von 2,6 g N-Isopropyl-N-(2-carbethoxy-4-phenyl-butyl)-amin und 1,2 ml Triethylamin in 10 ml Methylenchlorid wird zugetropft, die Mischung 20 Std. auf 35°C erhitzt, dann zur Trockne eingeengt, in Essigester aufgenommen, mit 1 N Salzsäure, gesättigter Natriumbicarbonatlösung und Wasser gewaschen, getrocknet und eingeengt. Das Rohprodukt wird an Kieselgel

in die beiden Diastereomeren des Produkts aufgetrennt.

Isomer 1: NMR : ($CDCl_3$) $\delta$ 7,3 - 6,8 m (14H); 5,05 s + t (3H); 4,48 s (2H), 4,2-3,5 m (4H); 3,4 -2,4 m (6H); 2,0-1,6 m (2H); 1,3 - 0,9 m (9H)

Isomer 2: NMR ($CDCl_3$) $\delta$ 7,3 - 6,9 m (14H); 5,0 s (2H); 4,8 t (1H); 4,52 s (2H); 4,2 - 3,5 m (4H); 3,3 - 2,4 m (6H); 2,0-1,6 m (2H), 1,3 - 1,0 m (9H).

1.7 <u>3-(S)-(N-Isopropyl-N-(4-phenyl-2-carbethoxy-butyl)-carbamoyl)-1,2,3,4-tetrahydroisochinolin-3-carbonsäure</u>

1.7.1. <u>Isomer 1</u>

1,35 g 1.6 (Isomer 1) werden in 30 ml absolutem Ethanol mit 0,7 g Pd/C (10%ig) 4 h bei 1 atm. Wasserstoffdruck hydriert. Nach Ende der Wasserstoffaufnahme wird filtriert und eingeengt.

NMR $\delta$=7,4 - 6,9 m (9H); 6,5 bs (1H); 4,66 t (1H); 4,4 s (2H); 4,2 - 3,5 m (3H); 3,4 - 2,3 m (7H); 2,0 - 1,6 m (2H); 1,3 - 0,9 m (9H).

Natriumsalz: 0,35 g 1.7.1 werden in 10 ml $H_2O$ aufgenommen, mit 63 mg Natriumbicarbonat 30 min. erhitzt, eingeengt,

mit Ether verfestigt; farbloses Pulver
IR 1730, 1620 $cm^{-1}$

1.7.2. Isomer 2

1,62 g 1,6 (Isomer 2) werden in 30 ml absolutem Ethanol mit 0,7 g Pd/c (10%ig) 1,5 h bei 1 atm Wasserstoffdruck hydriert. Nach Ende der Wasserstoffaufnahme wird filtriert und eingeengt.
NMR = $\delta$ 7,3 - 6,9 m (9H); 5,1 bs (1H); 4,60 t (1H); 4,30 s (2H); 4,2 - 3,5 m (3H); 3,3 - 2,4 m (7H); 2,0 - 1,5 m (2H); 1,4 - 1,0 m (9H).

Lysinsalz: 0,57 g 1.7.2. werden in 10 ml Methanol gelöst, 0,21 g Lysin in 5 ml Wasser werden zugesetzt, zur Trockne eingeengt, mit Ether verfestigt, farbloses Pulver
IR 1730, 1610 $cm^{-1}$

Beispiel 2:

3(S)-(N-Isopropyl-N-(4-phenyl-2-carboxy-butyl)-carbamoyl)-1,2,3,4-tetrahydro-isochinolin-3-carbonsäure

2.1. Isomer 1

0,55 g 1.7.1 werden in 6 ml Ethanol gelöst, 6 ml 6N Natronlauge zugegeben, über Nacht stehen gelassen, das Ethanol entfernt, mit 1N Salzsäure

angesäuert, mit Methylenchlorid extrahiert, über Magnesiumsulfat getrocknet, eingeengt, kristallisiert aus Chloroform/Petrolether.
F. 118 - 120°C
NMR (DMSO) = $\delta$ 7,1 s (9H); 4,5 t (1H); 4,43 s (2H); 4,0-2,8 m (7H); 1,9 - 1,5 m (2H), 1,05 dd (6H).

2.2 Isomer 2

0,68 g 1.7.2 werden in 10 ml Ethanol gelöst, 10 ml 6 N Natronlauge zugesetzt, 2 Std. gerührt, das Ethanol entfernt, mit 1 N Salzsäure angesäuert, mit Methylenchlorid extrahiert, getrocknet, eingeengt, farbloser Schaum.
NMR ($CDCl_3$) = $\delta$ 7,0 s (9H); 4,65 t (1H); 4,44 s (2H); 4,0 - 3,0 m (7H), 2,1 - 1,6 m (2H); 1,1 dd (6H).

Bis-Dicyclohexylaminsalz: 0,65 g 2,2 werden in 10 ml Methylenchlorid gelöst, 0,6 ml Dicyclohexylamin zugegeben, eingeengt, mit n-Hexan verrieben; farblose Kristalle F. 67 - 70°C (Zers.).
IR 1630 $cm^{-1}$

Beispiel 3:

3(S)-(N-Methyl-N-(4-phenyl-2-carbethoxy-butyl)-carbamoyl)-1,2,3,4-tetrahydroisochinolin-3-carbonsäure

3.1. N-Methyl-N -(2-carbethoxy-4-phenyl-butyl)-amin

26,3 g 2-Methylen-4-phenylbuttersäureethylester (1,4) und 4 g Methylamin werden im Autoklaven in 150 ml Ethanol 10 Std. auf 80°C erhitzt. Nach Abkühlen wird das Ethanol entfernt, mit 1N HCl aufgenommen, mit Ether extrahiert, mit Natriumcarbonat alkalisch gestellt und erneut extrahiert, getrocknet, eingeengt.

NMR ($CDCl_3$) = 7,1 s (5H); 4,1 q (2H); 3,1 s (3H); 3,0-2,2 m (6H); 2,0 - 1,5 m (2H); 1,22 t (3H).

3.2. N-Methyl-N-chlorcarbonyl-N-(2-carbethoxy-4-phenyl-butyl)-amin

2,35 g 3.1 werden zusammen mit 1,5 g Triethylamin in 10 ml trockenem Methylenchlorid gelöst. Diese Lösung wird bei -20° bis -30°C zu 11,5 ml einer 15%igen Phosgenlösung in Methylenchlorid getropft, 30 min. gerührt, dann zur Trockne eingeengt.

3.3. 2-Carbobenzoxy-3-carboxy-1,2,3,4-tetrahydroisochinolin (Z-Tic)

188 g (1,05 Mol) 1,2,3,4-Tetrahydroisochinolin-3-carbonsäure gibt man bei 0° zu 1050 ml 1N NaOH und tropft bei dieser Temperatur anschließend gleichzeitig 100 ml Chlorkohlensäurebenzylester und weitere 1050 ml 1N NaOH ein. Anschließend rührt man 2 Std. bei Raumtemperatur. Man extrahiert dreimal mit Ether und säuert mit konz. HCl auf pH 1 an. Das ausgefallene Öl wird in Essigester extrahiert. Man wäscht die Essigesterlösung mit Wasser, bis die Wasserphase einen pH-Wert von 3 zeigt. Nach Trocknenkristallisiert das Produkt beim Einengen und Anreiben. Man setzt 1,5 Liter Diisopropylether zu und rührt eine Stunde bei Raumtemperatur. Dann wird das Produkt abgesaugt; F. 138 - 139°.

3.4. 2-Carbobenzoxy-3-carboxy-1,2,3,4-tetrahydroisochinolin-tert.-butylester

Zur Lösung von 248,8 g (0,8 Mol) 3,3 in 1,6 Litern Methylenchlorid gibt man 312 ml tert.Butanol und 8 g 4-Dimethylaminopyridin. Man kühlt auf -5°C und setzt portionsweise eine Lösung von 176 g Dicyclohexylcarbodiimid in 350 ml Methylenchlorid zu. Nach 21 Stunden bei Raumtemperatur wird der ausgefallene Dicyclohexylharnstoff abgesaugt. Das Filtrat extrahiert man dreimal mit gesättigter Natriumbicarbonatlösung. Man trocknet über Magnesiumsulfat, engt im Vakuum bei Raumtemperatur ein. Es verbleibt ein gelbliches Öl.
NMR: 7,3 s (5H); 7,2 s (4H); 5,1 - 4,3 m (3H);

5,0 s (2H); 1,46 s (9H)

3.5. 3-Carboxy-1,2,3,4-tetrahydroisochinolin-tert.-butylester · Hydrochlorid

284 g 3.4. (0,775 Mol) löst man in 3 Litern Methanol, gibt 15 g 10% Pd-Bariumsulfatkatalysator zu und hydriert mit Wasserstoff bei Normaldruck. Der pH-Wert wird durch Zutropfen von 1N methanolischer HCl auf 4,0 gehalten. Nach beendeter Wasserstoffaufnahme saugt man ab, engt ein und verreibt mit Ether.
F. 180°C (Zers.)

3.6. 3(S)-(N-Methyl-N-(4-phenyl-2-carbethoxy-butyl)-carbamoyl)-1,2,3,4-tetrahydroisochinolin-3-carbonsäure-tert.butylester

2,7 g 3.5. werden in 30 ml gesättigter Natriumbicarbonatlösung gelöst, mit Methylenchlorid extrahiert, der Extrakt getrocknet und eingeengt. Der Rückstand wird in 10 ml Methylenchlorid und 1,2 g Triethylamin gelöst und zu 3.2. in 10 ml Methylenchlorid getropft. Die Mischung wird 20 Stunden auf 35°C erwärmt, dann zur Trockne eingedampft, der Rückstand wird in Essigester aufgenommen, mit gesättigter Natriumbicarbonatlösung, 1 N HCl und Wasser gewaschen, über Magnesiumsulfat getrocknet und eingeengt; blaßgelbes Harz.

NMR: 7,3 - 6,8 m (14H); 5,0 s (2H); 5,0 - 4,8 m (1H); 4,5 s (2H), 4,2 - 3,5 m (3H); 3,0 s (3H); 3,3 - 2,4 m (6H); 2,0 - 1,6 m (2H); 1,1 t (3H).

3.7. 3(S)-⟨N-Methyl-N-(2-carbethoxy-4-phenyl-butyl)-carbamoyl⟩-1,2,3,4-tetrahydroisochinolin-3-carbonsäure

3 g 3.6. werden mit 40 ml Trifluoressigsäure 2 Stunden bei Raumtemperatur gerührt, dann zur Trockne eingeengt. Der Rückstand wird in Essigester aufgenommen, mit Wasser dreimal gewaschen, getrocknet und eingeengt.

NMR 7,4 - 6,9 m (9H); 6,8 bs (1H); 4,6 t (1H); 4,4 s (2H); 4,2 - 3,5 m (2H); 3,4 - 2,3 m (7H); 3,0 s (3H); 2,0 - 1,6 m (2H); 1,1 t (3H)

Lysinsalz: farbloses Pulver
IR 1730, 1610 $cm^{-1}$

Beispiel 4:

3(S)-⟨N-Methyl-N-(2-carboxy-4-phenyl-butyl)-carbamoyl⟩-1,2,3,4-tetrahydroisochinolin-3-carbonsäure

1,3 g 3.7. werden in 20 ml Ethanol gelöst, 20 ml 6 N Natronlauge zugegeben, 3 Stunden bei Raumtemperatur gerührt, das Ethanol i.V. entfernt, mit 1 N Salzsäure

angesäuert, mit Methylenchlorid extrahiert und über Magnesiumsulfat getrocknet, dann eingeengt.

NMR 7,6 - 6,9 m(9H); 6,0 bs (2H); 4,6 t (1H); 4,4 s (2H); 3,4 - 2,3 m (7H); 3,0 s (3H); 2,0 - 1,6 m (2H).

Beispiel 5

(3S)-〈N-Ethyl-N-(4-phenyl-2S-carbethoxy-butyl)-carbamoyl〉-1,2,3,4-tetrahydroisochinolin-3-carbonsäure

5.1. N-Ethyl-N-(2-carbethoxy-4-phenyl-butyl)-amin

17 g 2-Methylen-4-phenylbuttersäureethylester (Beispiel 1.4) und 3.6 g Ethylamin werden in 50 ml abs. Ethanol gelöst und unter 40 Atmosphären Stickstoff 20 Stunden auf 105°C erhitzt. Nach Entfernen des Lösungsmittels wird mit 5-normaler Salzsäure aufgenommen, mit Ether extrahiert und die wäßrige Lösung mit Kaliumcarbonat auf pH 9.5 gestellt, erneut mit Ether extrahiert, mit Kaliumcarbonat getrocknet und eingeengt.

NMR ($CDCl_3$): 7,1 s (5H); 4,1 q (2H); 3,0-2,2 m (6H); 2,0-1,4 m (2H); 1,1 d+t (6H).

5.2. (3S)-〈N-Ethyl-N-(4-phenyl-2-carbethoxy-butyl)-carbamoyl〉-1,2,3,4-tetrahydroisochinolin-3-carbonsäurebenzylester

Nach dem in Beispiel 1.2. und 1.6. beschriebenen Verfahren werden 4,25 g 1,2,3,4-Tetrahydroisochinolin-3-carbonsäurebenzylester-Benzolsulfonat mit Phosgen und 2,5 g N-Ethyl-N-(2-carbethoxy-4-phenyl-butyl)-amin umgesetzt. Nach Chromatographie an Kieselgel (Laufmittel Essigester/ Cyclohexan 1:5) erhält man 1,99 g Isomer 1 und 2,45 g Isomer 2.

Isomer 1: NMR ($CDCl_3$) 7,3-6,8 m (14H); 5,05 s (2H); 4,95 t (1H); 4,48 s (2H); 4,2-3,5 m (4H); 3,4-2,4 m (6H); 2,0-1,6 m (3H); 1,1 t (6H);

Isomer 2: NMR ($CDCl_3$) 7,3-6,8 m (14H); 5,05 s (2H); 4,82 t (1H); 4,4 s (2H); 4,2-3,5 m (4H); 3,4-2,4 m (6H); 2,0-1,5 m (3H); 1,1 t ( 6H)

5.3. (3S)-⟨N-Ethyl-N-(4-phenyl-2S-carbethoxy-butyl)-carbamoyl⟩-1,2,3,4-tetrahydroisochinolin-3-carbonsäure

1,9 g des Isomer 1 aus Beispiel 5.2. werden nach dem in Beispiel 1.7 beschriebenen Verfahren hydriert.

1H-NMR 7,4-6,9 m (9H); 6.5 bs (1H); 4,6 t (1H); 4,4 s (2H); 4,2-3,5 m (3H); 3,4-2,3 m (6H); 2,0-1,6 m (2H); 1,3-0,9 m (6H).

Lysinsalz: 0,87 g 5.3 werden in 10 ml Methanol gelöst und mit 0,28 g Lysin in 5 ml Wasser versetzt. Das Lösungsmittel wird entfernt, der Rückstand mit Ether verrieben; farbloses Pulver.

Beispiel 6

(2S)-⟨N-Ethyl-N-(4-phenyl-2S-carbethoxy-butyl)-carbamoyl⟩-cis-endo-octahydroindol-2-carbonsäure

6.1. (2S)-cis-endo-Octahydroindol-2-carbonsäurebenzylester-Hydrochlorid

3 g (2S)-cis-endo-Octahydroindol-2-carbonsäure (hergestellt nach Eur.Pat.Appl. 37 231) werden in einer bei -10°C hergestellten Lösung von 3 ml Thionylchlorid

in 28,5 ml Benzylalkohol gegeben. Nach 15 Stunden wird der Benzylalkohol abdestilliert und das Produkt mit Diisopropylether verrieben, F. 140°C

6.2. (2S)-⟨N-Ethyl-N-(4-phenyl-2-carbethoxy-butyl)-carbamoyl⟩-cis-endo-octahydroindol-2-carbonsäure-benzylester

2,96 g der Verbindung aus Beispiel 6.1 werden mit Phosgen und 2,5 g der Verbindung aus Beispiel 5.1 nach dem in Beispiel 1.2 und 1.6 beschriebenen Verfahren umgesetzt. Die Trennung der Diastereomeren erfolgt an Kieselgel mit Essigester/Cyclohexan 1:4 als Laufmittel.

Isomer 1: $[\alpha]_D^{20}$ + 7,0° (c = 1, $CH_3OH$)

$^1$H-NMR ($CDCl_3$) 7,3 s (5H); 7,2 s (5H); 5,2-4,7 m (3H); 4,1 q (2H); 3,9-1,4 m (20H); 1,2 t (3H); 1,0 t (3H).

Isomer 2: $[\alpha]_D^{20}$ - 4,6°C (c = 1, $CH_3OH$)

$^1$H-NMR ($CDCl_3$): 7,3 s (5H); 7,15 s (5H); 5,1 s (2H); 5,0-4,6 m (1H); 4,1 q (2H); 3,9-1,4 m (20H); 1,2 t (3H); 1,0 t (3H).

6.3. (2S)-⟨N-Ethyl-N-(4-phenyl-2S-carbethoxy-butyl)-carbamoyl⟩-cis-endo-octahydroindol-2-carbonsäure

1,5 g des Isomer 2 aus Beispiel 6.2 werden nach dem in Beispiel 1.7 beschriebenen Verfahren hydriert.

$[\alpha]_D^{20}$ + 23,3° (c = 1, $CH_3OH$)

$^1$H-NMR ($CDCl_3$) 7,15 s (5H); 4,5 m (1H); 4,1 q (2H); 3,9-1,4 m (20H); 1,0 t (6H).

Natriumsalz: 0,876 g 6.3 werden in 10 ml Ethanol gelöst, mit 1,9 ml 1N Natronlauge versetzt, eingeengt und mit Ether verrieben; farbloses Pulver.

Beispiel 7

(3S)-〈N-Ethyl-N-(4-phenyl-2S-carboxy-butyl)-carbamoyl〉-1,2,3,4-tetrahydroisochinolin-3-carbonsäure

0,72 g der Verbindung aus Beispiel 5.3 werden mit 10 ml 6 N Natronlauge nach dem in Beispiel 2 beschriebenen Verfahren verseift,

$^{1}H$-NMR: 7,1 s (9H); 4,5 t (1H); 4,4 s (2H); 4,0-2,8 m (8H); 1,9-1,5 m (2H); 1.05 t (3H).

Beispiel 8

(2S)-〈N-Ethyl-N-(4-phenyl-2S-carboxy-butyl)-carbamoyl〉-cis-endo-octahydroindol-2-carbonsäure

0,39 g der Verbindung aus Beispiel 6.3 werden mit Natronlauge nach dem im Beispiel 2 beschriebenen Verfahren verseift.

$[\alpha]_D^{20}$ + 15,8° (c = 1, $CH_3OH$)

$^{1}H$-NMR ($CDCl_3$) 7,15 s (5H); 4,5 m (1H); 3,9-1,4 m (20H) 1,0 t (3H)

Bis-Dicyclohexylaminsalz: 0,31 g werden in 10 ml Methylenchlorid gelöst, mit 0,29 ml Dicyclohexylamin versetzt, eingeengt und mit Diisopropylether verrieben; farbloses Pulver.

Beispiel 9

〈N-Ethyl-N-(4-phenyl-2-carbethoxy-butyl)-carbamoyl〉-cis-endo-2-aza-bicyclo〈3,3,0〉octan-3-carbonsäure

9.1. Cis-endo-2-Aza-bicyclo ⟨3,3,0⟩ octan-3-carbonsäure-benzylester-Hydrochlorid

Hergestellt aus Cis-endo-2-Aza-bicyclo ⟨3,3,0⟩ octan-3-carbonsäure nach dem in Beispiel 6.1 beschriebenen Verfahren.

9.2. ⟨N-Ethyl-N-(4-phenyl-2-carbethoxy-butyl)-carbamoyl⟩-2-aza-cis-endo-bicyclo ⟨3,3,0⟩ octan-3-carbonsäure-benzylester

2,82 g der Verbindung aus Beispiel 9.1 werden mit Phosgen und 2,5 g der Verbindung aus Beispiel 5.1 nach den in Beispiel 1.2 und 1.6 beschriebenen Verfahren umgesetzt. Die Trennung der Diastereomeren erfolgt am Kieselgel mit Essigester/Cyclohexan (1:3) als Laufmittel,

Isomer 1 $^{1}H$-NMR ($CDCl_3$): 7,3 s (5H); 7,2 s (5H); 5,3-4,7 m (3H); 4,1 q (2H); 3,9-1,4 m (18H); 1,2 t (3H); 1,05 t (3H),

Isomer 2 $^{1}H$-NMR ($CDCl_3$): 7,3 s (5H); 7,15 s (5H); 5,1 s (2H); 4,8 m (1H); 4,15 q (2H); 4,0-1,5 m (18H); 1,15 t (3H); 1,0 t (3H).

9.3. ⟨N-Ethyl-N-(4-phenyl-2-carbethoxy-butyl)-carbamoyl⟩-cis-endo-2-azabicyclo ⟨3,3,0⟩ octan-3-carbonsäure

Hergestellt aus 1 g Isomer 2 aus Beispiel 9.2 nach dem in Beispiel 6.2 beschriebenen Verfahren.

$^{1}H$-NMR ($CDCl_3$) 7,1 s (5H); 4,4 m (1H); 4,1 q (2H); 3,8-1,4 m (18H); 1,0 t (6H).

Beispiel 10

⟨N-Ethyl-N-(4-phenyl-2-carboxy-butyl)-carbamoyl⟩-cis-endo-2-azabicyclo⟨3,3,0⟩octan-3-carbonsäure

Hergestellt aus 0,32 g der Verbindung aus Beispiel 9.3 nach dem in Beispiel 5.3 beschriebenen Verfahren.

$^{1}H$-NMR ($CDCl_3$) 7,1 s (5H); 4,5 m (1H); 3,8-1,4 m (18H); 1,0 t (3H).

Die in derfolgenden Tabelle aufgeführten Verbindungen werden nach analogen Verfahren unter Verwendung der entsprechenden Ausgangsmaterialien hergestellt.

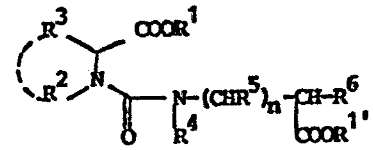

| | n | $R^1$ | $R^{1'}$ | $R^2$ | $R^3$ | $R^4$ | $R^5$ | $R^6$ | |
|---|---|---|---|---|---|---|---|---|---|
| 11 | 0 | H | H | H | $CH_3$ | $CH_3$ | – | $CH_2CH_2C_6H_5$ | 7.2 s (5H); 4.7-4,4 m (1H); 3.8-2.8 m (3H); 3.0 2 s (3H); 2.0-1.7 m (2H); 1.2 d (5H) |
| 12 | 1 | H | H | H | $CH_3$ | $C_2H_5$ | H | $CH_2CH_2C_6H_5$ | 7.2 s (5H); 4.7-4.4 m (1H); 3.5-2.8 m (7H); 2.0-1.7 m (2H); 1.2 d + t (6H) |
| 13 | 1 | H | $C_2H_5$ | H | $CH_3$ | $C_2H_5$ | H | $CH_2CH_2C_6H_5$ | 7.2 s (5H); 4.7-4.4 m (1H); 4.2 q (2H) 3.5-2.8 m (7H); 2.0-1.7 m (2H); 1.2 d + t (9H) |
| 14 | 1 | H | H | H | $(CH_3)_2CH$ | $CH_3$ | H | $CH_2CH_2C_6H_4$-4-$OCH_3$ | 7.0-6.5 m (4H); 4.8-4.4 m (1H); 3.5 - 2.8 m (5H); 3.0 2 s (3H); 1.9-1.3 m (3H); 1.2-0.9 d (6H) |
| 15 | 0 | H | H | H | $(CH_3)_2CH$ | $C_2H_5$ | – | $CH_2CH_2C_6H_5$ | 7.2 s (5H); 4.8-4.3 m (2H); 3.8-2.9 m (2H); 2.8-2.0 m (2H); 1.8-1.3 m (3H); 1.3-0.9 m (9H) |
| 16 | 0 | H | $C_2H_5$ | H | $(CH_3)_2CH$ | $C_2H_5$ | – | $CH_2CH_2C_6H_5$ | 7.2 s (5H); 4.8-4.3 m (2H); 4.1 q (2H) 3.9-2.9 m (2H); 2.8-2.0 m (2H); 1.8-1.3 m (3H); 1.3-0.9 m (12H) |
| 17 | 1 | H | H | $CH_3$ | $(CH_3)_2CH$ | $CH(CH_3)_2$ | H | $CH_2CH_2C_6H_5$ | 7.2 s (5H); 4.7-4.4 m (1H); 3.8-2.8 m (4H); 3.0 s (3H); 2.0-1.7 m (2H); 1.6-1.3 m (3H); 1.2-0.9 m (12H) |
| 18 | 1 | H | H | H | $(CH_3)_2CHCH_2$ | 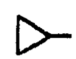 | H | $CH_2CH_2$-$C_6H_4$-4-F | 7.2-6.9 m (4H); 4.7-4.4 m (1H); 3.8 - 2.6 m (4H); 2.8-2.0 m (2H); 1.9-1.4 m (5H); 1.2-0.6 m (10H) |

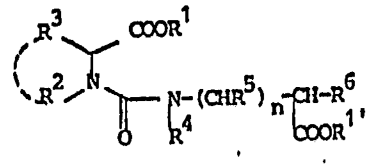

| | n | $R^1$ | $R^{1'}$ | $R^2$ | $R^3$ | $R^4$ | $R^5$ | $R^6$ | |
|---|---|---|---|---|---|---|---|---|---|
| 19 | 1 | H | $C_2H_5$ | H | $(CH_3)_2CHCH_2$ | 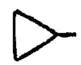 | H | $CH_2-CH_2-C_6H_4-4-F$ | 7.2-6.9 m (4H); 4.7-4.4 m (1H); 4.2 q (2H) 3.8-2.6 m (4H); 2.8-2.0 m (2H); 1.9-1.4 m (5H); 1.2-0.6 m + t (13H) |
| 20 | 1 | H | H | $CH_3$ | $(CH_3)_2CHCH_2$ | $HC\equiv C-CH_2$ | H | $CH_2CH_2-C_6H_3-(OCH_3)_2-3.4$ | 6.8-6.4 m (3H); 4.7-4.4 m (1H); 3.8-2.6 m (5H); 3.9 s (6H); 3.0 s (3H); 2.8-2.0 m (2H); 1.9-1.4 m (6H); 1.1-0.9 m (6H) |
| 21 | 0 | H | H | H | $(CH_3)_2CHCH_2$ | $CH_3CH_2CH_2$ | - | $CH_2-CH_2-C_6H_4-4-F$ | 7.2-6.9 m (4H); 4.7-4.4 m (2H); 3.8-2.6 m (2H); 2.8-2.0 m (2H); 2.0-1.4 m (7H); 1.1 d + t (9H) |
| 22 | 1 | H | H | H | $CH(CH_3)CH_2CH_3$ | $C_2H_5$ | H | $CH_2-CH_2-C_6H_4-2-CH_3$ | 7.2 s (4H); 4.7-4.4 m (1H); 3.8-2.6 m (5H); 2.8-2.0 m (2H); 2.2 s (3H); 1.9-1.4 m (5H); 1.1 d + 2t (9H) |
| 23 | 1 | H | $C_2H_5$ | H | $CH(CH_3)CH_2CH_3$ | $C_2H_5$ | H | $CH_2-CH_2-C_6H_4-2-CH_3$ | 7.2 s (4H); 4.7-4.4 m (1H); 4.2 q (2H); 3.8-2.6 m (5H); 2.8-2.0 m (2H); 2.2 s (3H); 1.9-1.4 m (5H); 1.3-0.9 m (12H) |
| 24 | 1 | H | H | H | $CH_2CONH_2$ | $C_2H_5$ | H | $CH_2-CH_2-$ 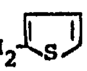 | 7.3-6.9 m (3H); 4.7-4.4 m (1H); 3.8-2.6 m (5H); 2.8-2.0 m (4H); 1.9-1.4 m (2H); 1,0 t (3H) |
| 25 | 1 | H | $C_2H_5$ | H | $CH_2CONH_2$ | $C_2H_5$ | H | $CH_2-CH_2-$ 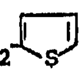 | 7.3-6.9 m (3H); 4.7-4.4 m (1H); 4.2 q (2H); 3.8-2.6 m (5H); 2.8-2.0 m (4H); 1.9-1.4 m (2H) 1,2 t (3H); 1.0 t (3H) |

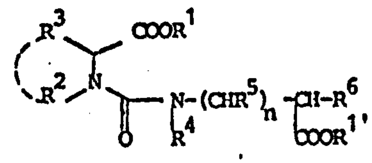

| | n | $R^1$ | $R^{1'}$ | $R^2$ | $R^3$ | $R^4$ | $R^5$ | $R^6$ | |
|---|---|---|---|---|---|---|---|---|---|
| 26 | 0 | H | $C_2H_5$ | H | $CH_2CONH_2$ | $C_2H_5$ | - | $CH_2CH_2-C_6H_4-3-CN$ | 7.6-7.0 m (4H); 4.8-4.3 m (2H); 4.2 q (1H); 2.8-2.0 m (4H); 1.9-1.4 m (2H); 1.2 t (3H) |
| 27 | 1 | H | H | H | $CH_2CH_2COOH$ | $CH_3$ | H | $CH_2-CH_2-C_6H_5$ | 7.2 s (5H); 4.7-4.4 m (1H); 3.6-2.8 m (3H); 2.8-2.0 m (4H); 2.0 s (3H); 1.8-1.4 m (4H) |
| 28 | 1 | H | H | H | $CH_2CH_2CONH_2$ | $CH_3$ | H | $CH_2CH_2-C_6H_5$ | 7.2 s (5H); 4.7-4.4 m (1H); 3.6-2.8 m (3H); 2.8-2.0 m (4H); 2.0 s (3H) 1.8-1.4 m (4H) |
| 29 | 1 | H | $C_2H_5$ | H | $CH_2CH_2CONH_2$ | $C_2H_5$ | H | $CH_2-CH_2-$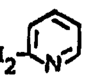 | 8.6-7.2 m (4H); 4.7-4.4 m (1H); 4.2 q (2H); 3.6-2.8 m (5H); 2.8-2.0 m (4H); 1.8-1.4 m (4H); 1.2 2 t (6H) |
| 30 | 1 | H | H | H | $(CH_2)_4NH_2$ | $C_2H_5$ | H | $CH_2CH_2-C_6H_5$ | 7.25 (5H); 4.7-4.4 m (1H); 3.6-2.8 m (5H); 2.8-2.0 m (4H); 1.9-1.3 m (8H); 1.1 t (3H) |
| 31 | 1 | H | $C_2H_5$ | H | $(CH_2)_4NH_2$ | 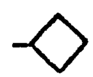 | $CH_3$ | $CH_2CH_2C_6H_5$ | 7.2 s (5H); 4.7-4.4 m (1H); 3.6-2.8 m (4H); 2.8-2.0 m (4H); 4.2 (2H); 1.9-1.1 m (14H) 1.2-1.0 2 t + a (9H) |
| 32 | 1 | H | H | $CH_3$ | $CH_2SH$ | $-CH_2-CH=CH_2$ | H | $CH_2-CH_2-C_6H_4-2-CH_3$ | 7.2 s (4H); .8 m (1H); 5.0-4.3 m (5H); 3.6-2.7 m (3H); 2.8-2.0 m (4H) 2.1 s (3H); 1.9-1.4 m (2H) |
| 33 | 1 | H | H | H | $CH_2SH$ | $-CH_2CH_2CH_3$ | H | $CH_2-CH_2-C_6H_4-4-NH-COCH_3$ | 7.1-6.7 m (4H); 4.8-4.3 m (1H); 3.6-2.7 m (5H); 2,8-2.0 m (4H); 2.0 s (3H); 1.9-1.4 m (4H); 1.1 t (3H) |

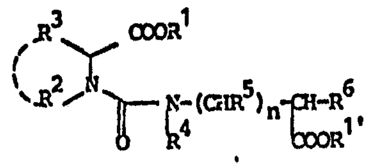

| | n | $R^1$ | $R^{1'}$ | $R^2$ | $R^3$ | $R^4$ | $R^5$ | $R^6$ | |
|---|---|---|---|---|---|---|---|---|---|
| 34 | 0 | H | $C_2H_5$ | H | $CH_2SH$ | $-C_2H_5$ | - | $CH_2-CH_2-C_6H_5$ | 7.2 s (5H); 4.8-4.3 m (2H); 4.2 q 3.8-2.8 m (2H); 2.8-2.0 m (4H); 1.9-1.4 m (2H); 1.2 t (3H) |
| 35 | 1 | H | H | H | $CH_2SC_6H_5$ | $-C_2H_5$ | H | $CH_2CH_2C_6H_5$ | 7.2 s (10H); 4.8-4.3 m (1H); 3.8-2.8 m (6H); 3.4-2.8 m (1H); 1.9-1.4 m (2H) 1.1 t (3H) |
| 36 | 1 | H | $C_2H_5$ | H | $CH_2SC_5H_5$ | $-C_2H_5$ | H | $CH_2CH_2C_6H_5$ | 7.2 s (10H); 4.8-4.3 m (1H); 4.2 q (2H); 3.8-2.8 m (6H); 3,4-2.8 m (1H); 1.9-1.4 m (2H); 1.3-1.0 m (6H) |
| 37 | 1 | H | H | H | $CH_2CH_2SCH_3$ | $-CH_3$ | H | $CH_2CH_2C_6H_5$ | 7.2 s (5H); 4.7-4.4 m (1H); 3.8-2.8 m (3H); 2.8-2.0 m (4H); 2.2 s (3H); 2.1 s (3H); 1.9-1.4 m (4H) |
| 38 | 1 | H | $C_2H_5$ | H | $CH_2CH_2SCH_3$ | $-CH\ (CH_3)_2$ | H | $CH_2CH_2-C_6H_5-(OCH_3)_2-3.4$ | 6.8-6.3 m (3H); 4.7-4.4 m (1H); 4.2 q (2H); 4.0 s (6H); 3.8-2.8 m (4H), 2.8-2.0 m (4H); 2.2 s (3H); 1.9-1.4 m (4H); 1.0 d (6H) |
| 39 | 1 | H | H | $CH_3$ | $CH_2CH_2SCH_3$ | $-CH_2CH_2CH_3$ | $CH_3$ | $CH_2-CH_2-C_6H_5$ | 7.2 s (5H); 4.7-4.4 m (1H); 3.8-2.8 m (3H); 2.8-2.0 m (4H); 2.2 s (3H) 2.0 s (3H) 1.9-1.4 m (6H); 1.0 d + t (6H) |
| 40 | 0 | H | $C_2H_5$ | H | $CH_2CH_2SCH_3$ | $-CH_2CH_2CH_3$ | - | $CH_2-CH_2-C_6H_5$ | 7.2 s (5H); 4.8-4.3 m (2H); 4.2 q (2H); 3.8-2.8 m (2H); 2.8-2.0 m (4H) 2.2 s (3H); 1.9-1.4 m (6H); 1.2 t (3H) 1,0 t (3H) |

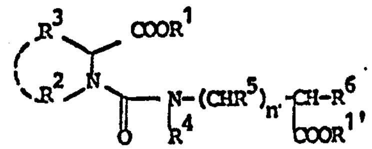

| | n | $R^1$ | $R^{1'}$ | $R^2$ | $R^3$ | $R^4$ | $R^5$ | $R^6$ | |
|---|---|---|---|---|---|---|---|---|---|
| 41 | 1 | H | H | H | $CH_2C_6H_5$ | $CH_3$ | H | $(CH_2)_3-CH_3$ | 7.2 s (5H); 4.7-4.3 m (1H); 3.8-2.6 m (3H); 2.8-2.0 m (2H); 2.0 s (3H); 1.9-1.3 m (6H); 1.2 t (3H) |
| 42 | 1 | H | H | H | $CH_2C_6H_5$ | $C_2H_5$ | H | $CH_2-S-C_6H_5$ | 7.2-6.9 m (10H); 4.7-4.3 m (1H); 3.8-2.6 m (6H), 2.8-2.0 m (2H); 1.0t (3H) |
| 43 | 1 | H | H | H | $CH_2C_6H_5$ | $C_2H_5$ | H | $CH_2-SOC_6H_5$ | 7.4-6.9 m (10H); 4.7-4.3 m (1H); 3.8-2.6 m (6H); 2.8-2.0 m (2H); 1.0 t (3H) |
| 44 | 1 | H | $C_2H_5$ | H | $CH_2C_6H_5$ | $CH_2-CH=CH_2$ | $CH_3$ | $CH_2-NH-C_6H_5$ | 7.1-6.4 m (10H); 5.8 m (1H); 4.8-4.3 m (5H); 4.2 q (2H); 3.8-2.6 m (5H); 2.8-2.0 m (2H); 1.3 t (3H) |
| 45 | 1 | H | H | $CH_3$ | $CH_2C_6H_5$ | 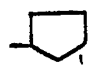 | H | $CH_2-CH_2-$ 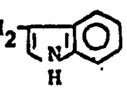 | 8.0 s (1H); 7.8-6.9 m (10H); 4.7-4.3 m (1H); 3.9-2.8 m (4H); 2.8-2.0 m (4H); 2.1 s (3H); 1.9-1.4 m (10H) |
| 46 | 1 | H | $C_2H_5$ | H | $CH_2C_6H_5$ | $CH(CH_3)_2$ | H | $CH_2-CH_2-$ 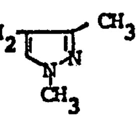 | 7.3-6.9 m (6H); 4.7-4.4 m (1H); 4.2 q (2H); 3.8-2.6 m (4H); 2.7 s (3H); 2.3 s (3H); 2.8-2.0 m (4H); 1.3 t (3H); 1.1 d (6H) |
| 47 | 1 | H | H | H | $CH_2C_6H_5$ | $C_2H_5$ | H | $CH_2CH_2-C_6H_5$ | 7.2 s (10H); 4.7-4.4 m (1H); 3.8-2.6 m (5H); 2.8-2.0 m (4H); 1.9-1.4 m (2H); 1.1 t (3H) |
| 48 | 0 | H | H | H | $CH_2C_6H_5$ | $C_2H_5$ | H | $CH_2-CH_2-C_6H_5$ | 7.2 s (10H); 4.8-4.3 m (2H); 3.8-2.6 m (2H); 1.9-1.4 m (2H); 1.2 t (3H) |

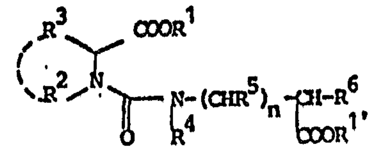

| | n | $R^1$ | $R^{1'}$ | $R^2$ | $R^3$ | $R^4$ | $R^5$ | $R^6$ | |
|---|---|---|---|---|---|---|---|---|---|
| 49 | 0 | H | $C_2H_5$ | H | $CH_2C_6H_5$ | $CH_2CH_2CH_3$ | H | $CH_2$-$CH_2$-$C_6H_4$-4-F | 7.3-6.9 m (9H); 4.8-4.3 m (2H); 3.8-2.6 m (2H); 4.2 q (2H); 1.9-1.4 m (4H); 1.2 t (3H); 1.0 t (3H) |
| 50 | 0 | H | $C_2H_5$ | H | $CH_2C_6H_5$ | $CH_2$-CH=CH-$CH_3$ | H | $CH_2$-$CH_2$-$C_6H_5$ | 7.2 s (10H); 5.5-5.1 m (2H); 4.8-4.1m (4H); 4.2 q (2H);2.8 - 2.0 m (4H); 2.2 d (3H); 1.9-1.4 m (2H); 1.2 t (3H) |
| 51 | 1 | H | H | H | $CH_2$- 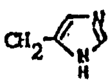 | $CH_3$ | H | $CH_2CH_2C_6H_5$ | 7.6 d (1H); 6.7 d (1H); 7.2 s (5H); 4.7-4.3 m (1H); 3.8-2.6 m (3H); 2.3 s (3H); 2.8-2.0 m (4H);1.9-1.4 m (2H) |
| 52 | 1 | H | $C_2H_5$ | H | " | $C_2H_5$ | H | $CH_2CH_2C_6H_5$ | 7.6 d (1H); 7.2 s (5H); 6.7 d (1H); 4.7-4.3 m (1H); 4.2 q (2H); 3.8-2.6 m (5H); 2.8-2.0 m (4H); 1.9-1.4 m (2H); 1.2 t (3H) |
| 53 | 1 | H | H | H | $CH_2OH$ | $CH_3$ | H | $CH_2CH_2C_6H_5$ | 7.2 s (5H); 4.7-4.3 m (1H); 3.8-2.6 m (5H); 2.8-2.0 m (2H); 2.2 s (3H); 1.9-1.4 m (2H); |
| 54 | 1 | H | $C_2H_5$ | H | $CH_2OH$ | $C_2H_5$ | H | $CH_2CH_2$-$C_6H_4$-4-$OCH_3$ | 6.9-6.5 m (4H); 4.7-4.3 m (1H); 4.2 q (3H); 4.0 s (3H); 3.8-2.6 m (7H); 2.8-2.0 m (2H); 1.9-1.4 m (2H); 1.5 t (3H); 1.1 t (3H) |
| 55 | 1 | H | H | H | $CH_2OCH_3$ | $CH(CH_3)_2$ | H | $CH_2C_6H_5$ | 7.2 s (5H); 4.7-4.3 m (1H); 3.9-2.6 m + s (9H); 2.8-2.0 m (2H); 1.1 d (6H) |
| 56 | 1 | H | H | H | $CH_2OCH_3$ | $CH(CH_3)_2$ | H | $CH_3$ | 4.7-4.3 m (1H); 3.9-2.6 m + s (9H); 1.2 d (9H) |

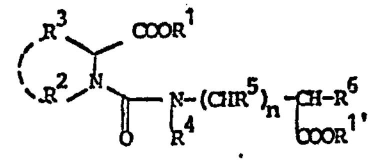

| | n | $R^1$ | $R^{1'}$ | $R^2$ | $R^3$ | $R^4$ | $R^5$ | $R^6$ | |
|---|---|---|---|---|---|---|---|---|---|
| 57 | 1 | H | H | H | $CH_2OCH_3$ | $CH(CH_3)_2$ | H | H | 4.7–4.3 m (1H); 3.9–2.6 m + s (9H); 2.4–2.0 m (2H); 1.2 d (6H) |
| 58 | 1 | H | $C_2H_5$ | H | 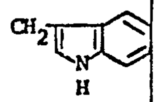 | $CH_3$ | H | $CH_2CH_2C_6H_5$ | 8.0 s (1H); 7.8–6.8 m (10H); 4.7–4.3 m (1H); 4.2 q (2H); 3.8–2.7 m (3H); 2.8–2.0 m (4H); 1.9–1.3 m (2H); 2.0 s (3H) 1.2 t (3H) |
| 59 | 1 | H | H | H | " | $C_2H_5$ | H | 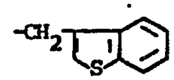 | 8.0 s (1H); 7.8–6.8 m (10H); 4.7–4.3 m (1H); 3.8–2.7 m (5H); 2.8–2.0 m (4H); 1.9–1.3 m (2H); 1.1 t (3H) |
| 60 | 1 | H | H | H | $CH_2CH_2OCH_3$ | $CH_3$ | H | $CH_2CH_3$ | 4.7–4.3 m (1H); 3.8–2.7 m + s (8H); 2.3 s (3H); 1.9–1.4 m (4H); 1.1 t(3H) |
| 61 | 1 | H | $C_2H_5$ | $CH_3$ | $CH_2CH_2OCH_3$ | $CH_2-CH=CH_2$ | H | $CH_2CH(CH_3)_2$ | 5.8 m (1H); 4.9–4.1 m (5H); 3.7–2.9 m + s (8H); 4.2 q (2H) 1.9–1.4 m (5H); 1.0 d (9H) |
| 62 | 0 | H | $C_2H_5$ | H | $CH_2CH_2OCH_3$ | $C_2H_5$ | – | $CH_2CH_2C_6H_5$ | 7.2 m (5H); 4.9–4.3 m (2H); 3.7–2.9 m + s (7H); 4.2 q (2H); 2.8–2.0 m (2H) 1.9–1.4 m (4H); 1.3 t (3H); 1.0 t(3H) |
| 63 | 1 | H | H | $CH_3$ | $(CH_2)_4NHCOCH_3$ | $CH_3$ | H | $CH_2CH_2C_6H_5$ | 7.2 s (5H); 4.7–4.4 m (1H); 3.7–2.9 m (2H); 2.8–2.0 m (4H); 2.1 s (9H); 1.9–1.3 m (8H); 1.1 d (3H) |
| 64 | 1 | H | $C_2H_5$ | H | $(CH_2)_4NHCOCH_3$ | $CH(CH_3)_2$ | H | $(CH_2)_4CH_3$ | 4.7–4.4 m (1H); 4.2 q (2H); 3.7–2.9 m (6H); 2.0–1.2 m (14H); 1.3 t (3H); 1.1–0.9 m (9H) |

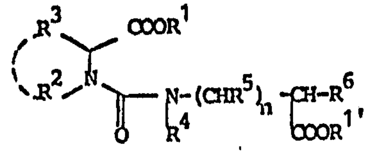

| | n | $R^1$ | $R^{1'}$ | $R^2$ — $R^3$ | $R^4$ | $R^5$ | $R^6$ | |
|---|---|---|---|---|---|---|---|---|
| 65 | 1 | H | H | $(CH_2)_3$ | $CH_3$ | H | $CH_2CH_2C_6H_5$ | 7.2. s (5H); 4.9-4.4 m (1H); 3.8-2.9m (5H); 2.8-2.0 m (2H); 2.0 s (3H); 1,9-1.4 m (6H) |
| 66 | 1 | H | H | $(CH_2)_3$ | $C_2H_5$ | H | $CH_2C_6H_5$ | 7.2 s (5H); 4.9-4.4 m (1H); 3.8-2.9 m (7H); 2.8-2.0 m (2H); 1.9-1.4 m (4H) 1.1 t (3H) |
| 67 | 1 | H | H | $(CH_2)_3$ | $CH(CH_3)_2$ | H | $CH_2CH_2CH_2CH_3$ | 4.8-4.4 m (1H); 3.8-2.9 m (6H); 1.9-1.3 m (10H); 1.2-0.9 m (9H) |
| 68 | 1 | H | H | $(CH_2)_3$ | $CH_2-CH=CH_2$ | H | $CH_2-CH_2-CH(CH_3)_2$ | 5.8 m (1H); 4.8-4.2 m (5H); 3.8-2.9m (5H); 1.9-1.3 m (9H); 1.0 d (6H) |
| 69 | 1 | H | H | $(CH_2)_3$ | $CH_2-C\equiv CH$ | H | $CH_2CH_2-C(CH_3)_3$ | 4.8-4.2 m (1H); 3.8-2.9 m (7H); 1.9-1.3 m (9H); 0.9 s (9H) |
| 70 | 1 | H | H | $(CH_2)_3$ | 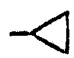 | H | $CH_2CH_2-C_6H_4-4-F$ | 7.3-6.8 m (4H); 4.7-4.3 m (1H); 3.8-2.7 m (6H); 2.8-2.0 m (2H); 1.9-1.3 m (6H); 1.0-0.6 m (4H) |
| 71 | 1 | H | H | $(CH_2)_3$ | $-C_2H_5$ | H | $CH_2-CH_2-C_6H_3-2.6-Cl_2$ | 7.3 s (3H); 4.7-4.3 m (1H); 3.7-2.7m (7H); 2.8-2.0 m (2H); 1.9-1.3 m (6H) 1.1 t (3H) |
| 72 | 1 | H | H | $(CH_2)_3$ | $-C_2H_5$ | H | $CH_2$ 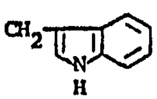 | 8.0 s (1H); 7.6-6.7 m (5H) 4.7-4.3 m (1H); 3.7-2.7 m (7H); 2.8-2.0 m (2H); 1.1 t (3H) |

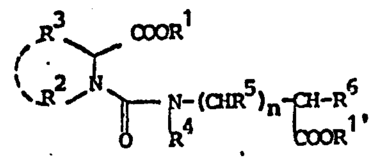

| | n | $R^1$ | $R^{1'}$ | $R^2$ — $R^3$ | $R^4$ | $R^5$ | $R^6$ | |
|---|---|---|---|---|---|---|---|---|
| 73 | 1 | H | $C_2H_5$ | $(CH_2)_3$ | $CH_3$ | H | $CH_2CH_2C_6H_5$ | 7.2 s (5H); 4.7-4.3 m (1H); 3.7-2.7 m (5H); 4.2 q (2H); 2.8-2.0 m (2H); 2.1s (3H); 1.9-1.4 m (6H); 1.3 t (3H) |
| 74 | 1 | H | $C_2H_5$ | $(CH_2)_3$ | $C_2H_5$ | H | $CH_2CH_2C_6H_5$ | 7.2 s (5H); 4.7-4.3 m (1H); 4.2 q (2H) 3.7-2.7 m (7H); 2.8-2.0 m (2H); 1.9-1.4 m (6H); 1.3 t (3H); 1.0 t (3H) |
| 75 | 1 | H | $C_2H_5$ | $(CH_2)_3$ | $CH(CH_3)_2$ | H | $CH_2CH_2C_6H_5$ | 7.2 s (5H); 4.7-4.3 m (1H); 4.2 q (2H) 3.7-2.7 m (6H); 2.8-2.0 m (2H); 1.9-1.3 m (6H); 1.3 t (3H); 0.9 d (6H) |
| 76 | 1 | H | $C_2H_5$ | $(CH_2)_3$ | $CH_2-CH=CH_2$ | H | $CH_2CH_2C_6H_5$ | 7.2 s (5H); 5.8 m (1H); 4.8-4.1 m (5H) 4.2 q (2H); 3.7-2.7 m (5H); 2.8-2.0 m (2H); 1.9-1.4 m (6H); 1.3 t (3H) |
| 77 | 1 | H | $C_2H_5$ | $(CH_2)_3$ | 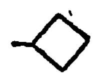 | H | $CH_2CH_2C_6H_5$ | 7.2 s (5H); 4.6-4.2 m (1H); 4.2 q (2H) 3.8-2.7 m (6H); 2.8-2.0 m (2H); 1.9-1.4 m (12H); 1.3 t (3H) |
| 78 | 1 | H | $C_2H_5$ | $(CH_2)_3$ | $CH_2-C≡CH$ | H | $CH_2CH_2C_6H_5$ | 7.2 s (5H); 4.7-4.3 m (1H); 4.2 q (2H) 3.8-2.7 m (7H); 2.8-2.0 m (2H); 1.9-1.4 m (7H); 1.3 t (3H) |
| 79 | 0 | H | H | $(CH_2)_3$ | $CH_3$ | - | $CH_2CH_2C_6H_5$ | 7.2 s (5H); 4.9-4.3 m (2H); 3.8-2.7 m (2H); 2.3 s (3H); 2.8-2.0 m (2H); 1.9-1.4 m(6H) |
| 80 | 0 | H | $C_2H_5$ | $(CH_2)_3$ | $C_2H_5$ | - | $CH_2CH_2-C_6H_4-4-F$ | 7.2-6.8 m (4H); 4.9-4.3 m (2H); 4.2 q (2H); 3.8-2.7 m (4H); 2.8-2.0 m (2H); 1.9-1.4 m (6H); 1.3 t (3H); 1.1 t (3H) |

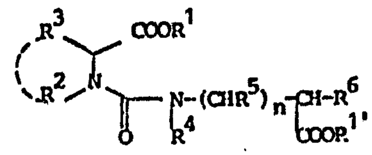

| | n | $R^1$ | $R^{1'}$ | $R^2$ — $R^3$ | $R^4$ | $R^5$ | $R^6$ | |
|---|---|---|---|---|---|---|---|---|
| 81 | 0 | H | $C_2H_5$ | $(CH_2)_3$ | $C_2H_5$ | - | $CH_2-CH_2-CH_3$ | 4.9-4.3 m (2H); 4.2 q (2H); 3.8-2.7 m (4H); 1.9-1.4 m (8H); 1.1 t (6H) |
| 82 | 0 | H | H | $(CH_2)_3$ | $(CH(CH_3)_2$ | - | $CH_2CH_2-C_6H_4-4-OCH_3$ | 6.9-6.3 m (4H); 4.9-4.3 m (2H); 3.8-2.7 m (3H); 3.9 s (3H); 2.8-2.0 m (2H); 1.9-1.4 m (6H); 1.0 d (6H) |
| 83 | 0 | H | H | $(CH_2)_3$ | $CH_2-CH=CH_2$ | - | $CH_2-CH_2-C_6H_3-(OCH_3)_2-3.4$ | 6.8-6.0 m (3H); 5.8 m (1H); 4.9-4.3 m (6H); 4.0 s (6H), 3.8-3.1 m (2H); 2.8-2.0 m (2H); 1.9-1.4 m (6H) |
| 84 | 0 | H | H | $(CH_2)_3$ | $CH_2-C\equiv C-CH_3$ | - | $CH_2CH_2C_6H_5$ | 7.2 s (5H); 4.9-4.3 m (2H); 3.8-2.7 m (4H); 2.8-2.0 m (2H); 1.8 s (3H); 1.8-1.3 m (6H) |
| 85 | 1 | H | H | $-CH_2-CH(OCH_3)-CH_2-$ | $C_2H_5$ | H | $CH_2CH_2C_6H_5$ | 7.2 s (5H); 4.8-4.3 m (1H); 4.1-2.9 m (8H); 3.2 s (3H); 2.8-2.0 m (2H); 2.0-1.3 m (6H); 1.0 t (3H) |
| 86 | 1 | H | $C_2H_5$ | $-CH_2-CH(OCH_3)-CH_2-$ | $CH_3$ | H | $CH_2CH_2C_6H_5$ | 7.2 s (5H); 4.8-4.3 m (1H); 4.1-2.9 m (6H); 4.2 q (2H); 3.2 s (3H); 2.4 s (3H); 2.8-2.0 m (2H); 2.0-1.4 m (6H); 1.3 t (3H) |
| 87 | 1 | H | H | $CH_2-CH=CH-$ | $C_2H_5$ | $CH_3$ | $CH_2CH_2C_6H_5$ | 7.2 s (5H); 6.4-5.5 m (3H); 4.3-2.9 m (6H); 2.8-2.0 m (2H); 1.9-1.4 m (2H); 1.2 d + t (6H) |
| 88 | 1 | H | $C_2H_5$ | $CH_2-CH=CH$ | $CH(CH_3)_2$ | H | $CH_2CH_2C_6H_5$ | 7.2 s (5H); 6.4-5.5 m (3H); 4.3-2.9 m (8H); 2.8-2.0 m (2H); 1.6-1.2 m (2H); 1.3 t (3H); 1.0 d (6H) |

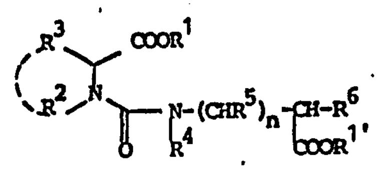

| | n | $R^1$ | $R^{1'}$ | $R^2$ — $R^3$ | $R^4$ | $R^5$ | $R^6$ | |
|---|---|---|---|---|---|---|---|---|
| 89 | 1 | H | H | $CH(CH_3)CH_2-CH_2$ | $CH_3$ | H | $CH_2CH_2C_6H_5$ | 7.2 s (5H); 4.7-4.3 m (1H); 3.7-2.8 m (5H); 2.8-2.0 m (2H); 1.6-1.3 m (5H); 2.1 s (3H); 1.0 d (3H) |
| 90 | 1 | H | H | $CH_2-CH(CH_3)-CH_2$ | $C_2H_5$ | H | $CH_2CH_2C_6H_5$ | 7.2 s (5H); 4.7-4.3 m (1H); 3.7-2.8 m (7H); 2.8-2.0 m (2H); 1.7-1.3 m (5H); 1.0 d + t (6H) |
| 91 | 1 | H | $C_2H_5$ | $CH_2-CH(C_6H_5)-CH_2$ | $C_2H_5$ | H | $CH_2CH_2C_6H_5$ | 7.2 s (10H); 4.7-4.3 m (1H); 4.2q (2H); 3.7-2.8 m (8H); 2.8-2.0 m (2H); 1.8-1.4 m (6H); 1.3 t (3H); 1.0 t (3H) |
| 92 | 1 | H | H | $CHC_6H_5-CH_2-CH_2$ | $C_3H_7$ | H | $CH_2CH_2C_6H_5$ | 7.2 s (10H); 4.7-4.3 m (1H); 3.7-2.8 m (8H); 2.8-2.0 m (2H); 1.8-1.4 m (8H); 1.0 t (3H) |
| 93 | 1 | H | H | $(CH_2)_4$ | $CH_3$ | H | $CH_2-CH_2-$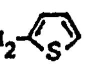 | 7.3-6.9 m (3H); 4.7-4.4 m (1H); 3.6-2.8 m (5H); 2.2 s (3H); 2.8-2.0 m (2H) 1.9-1.4 m (8H) |
| 94 | 1 | H | $C_2H_5$ | $(CH_2)_4$ | $CH_3$ | H | $CH_3$ | 4.7-4.4 m (1H); 4.2 q (2H); 3.6-2.8 m (5H); 2.2 s (3H); 1.9-1.4 m (6H); 1.1 d (3H) |
| 95 | 1 | H | $C_2H_5$ | $(CH_2)_4$ | $C_2H_5$ | $CH_3$ | $CH_2-CH_3$ | 4.7-4.4 m (1H); 4.2 q (2H); 3.6-2.8 m (6H); 1.9-1.4 m (8H); 1.3 t (3H); 1.0 m (9H) |
| 96 | 1 | H | H | $(CH_2)_4$ | $CH(CH_3)_2$ | H | $CH_2-CH_2-C_6H_5$ | 7.2 s (5H); 4.7-4.4 m (1H); 3.6-2.8 m (6H); 2.8-2.0 m (2H); 1.9-1.4 m (8H); 1.0 d (6H) |

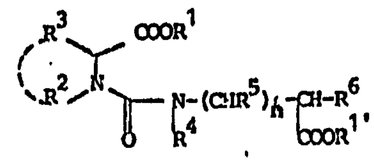

| | n | $R^1$ | $R^{1'}$ | $R^2$ — $R^3$ | $R^4$ | $R^5$ | $R^6$ | |
|---|---|---|---|---|---|---|---|---|
| 97 | 1 | H | H | $(CH_2)_4$ | $C_2H_5$ | H | 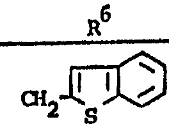 | 7.4-6.8 m (5H); 4.7-4.4 m (1H); 3.6-2.8 m (7H); 2.8-2.0 m (2H); 1.9-1.4 m (6H); 1.0 t (3H) |
| 98 | 1 | H | H | $(CH_2)_4$ | $C_2H_5$ | H | 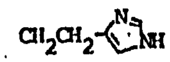 | 7.6 d (1H); 6.7 d(1H); 4.7-4.4 m (1H) 3.6-2.8 m (7H); 2.8-2.0 m (2H); 1.9-1.4 m (8H); 1.0 t (3H) |
| 99 | 1 | H | $C_2H_5$ | $(CH_2)_4$ | $CH_2-CH=CH_2$ | H | $CH_2-CH_2-C_6H_3-2.6-Cl_2$ | 7.3 s (3H); 5.8 m (1H); 4.9-4.3 m (5H); 4.2 q (2H); 3.6-2.8 m (5H); 2.8-2.0 m (2H); 1.9-1.5 m (8H) |
| 100 | 1 | H | H | $(CH_2)_4$ | $CH_2-C\equiv CH$ | H | $CH_2-CH_2-C_6H_3-Cl-2-OCH_3-4$ | 7.3-6.6 m (3H); 4.7-4.3 m (1H); 3.8-2.8 m (7H); 4.0 s (3H); 2.8-2.0 m (2H) 1.9-1.5 m (9H) |
| 101 | 1 | H | $C_2H_5$ | $(CH_2)_4$ | $CH_2-CH_3$ | $CH_3$ | 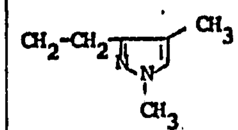 | 7.4 s (1H); 4.7-4.3 m (1H); 4.2 q (2H) 3.8-2.8 m (6H); 2.3 s (3H); 2.1 s (3H) 2.8-2.0 m (2H); 1.9-1.4 m (8H); 1.3 t (3H); 1.0 d (3H) |
| 102 | 1 | H | H | $(CH_2)_4$ | $CH_3$ | H | $CH_2-C_6H_5-C_6H_5$ | 7.4.-6.8 m (9H); 4.7-4.3 m (1H); 3.8-2.8 m (5H); 2.2 s (3H); 2.8-2.0 m (2H) 1.9-1.4 m (6H) |
| 103 | 1 | H | $C_2H_5$ | $(CH_2)_4$ | 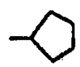 | H | $CH_3$ | 4.7-4.3 m (1H); 4.2 q (2H); 3.8-2.8 m (6H); 1.9-1.3 m (14H); 1.3 t (3H); 1.0 d (3H) |

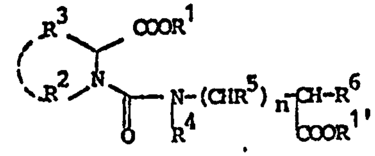

| | n | $R^1$ | $R^{1'}$ | $R^2$ — $R^3$ | $R^4$ | $R^5$ | $R^6$ | |
|---|---|---|---|---|---|---|---|---|
| 104 | 1 | H | H | $(CH_2)_4$ | $C_2H_5$ | H | $CH_2CH_2$- 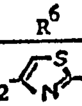 -$C_6H_5$ | 7.5 s (1H); 7.1 s (5H); 4.7–4.3 m (1H); 3.8–2.8 m (7H); 2.8–2.0 m (2H); 1.9–1.4 m (8H); 1.0 t (3H) |
| 105 | 1 | H | H | $(CH_2)_4$ | $C_2H_5$ | H | $CH_2CH_2CH_2C_6H_5$ | 7.2 s (5H); 4.7–4.3 m (1H); 3.8–2.9 m (7H); 2.8–2.0 m (2H); 1.9–1.4 m (10H); 1.0 t (3H) |
| 106 | 0 | H | H | $(CH_2)_4$ | $CH_3$ | – | $CH_2CH_2C_6H_5$ | 7.2 s (5H); 4.9–4.2 m (2H); 3.8–2.9 m (2H); 2.8–2.0 m (2H); 2.2 s (3H); 1.9–1.5 m (8H) |
| 107 | 0 | H | H | $(CH_2)_4$ | $C_2H_5$ | – | $CH_2$-$CH_2$-$C_6H_4$-4-F | 7.2–6.8 m (4H); 4.9–4.2 m (2H); 3.8–2.9 m (4H); 2.8–2.0 m (2H); 2.0–1.5 m (8H); 1.0 t (3H) |
| 108 | 0 | H | $C_2H_5$ | $(CH_2)_4$ | $CH(CH_3)_2$ | – | $CH_2$-$CH_2$-$C_6H_3$-$(OCH_3)_2$-3.4 | 6.9–6.2 m (3H); 4.9–4.2 m (2H); 4.2 q (2H); 4.0 s (6H); 3.8–2.9 m (3H); 2.8–2.0 m (2H); 1.9–1.4 m (8H); 1.0 d (6H); 1.3 t (3H) |
| 109 | 0 | H | $C_2H_5$ | $(CH_2)_4$ | -$CH_2$-$CH$=$CH_2$ | – | $CH_2$-$CH_2$-$C_6H_5$ | 7.2 s (5H); 4.9–4.2 m (6H); 4.2 q (2H) 5.8 m (1H); 2.8–2.1 m (2H); 1.9–1.4 m (8H); 1.3 t (3H) |
| 110 | 1 | H | H | $(CH_2)_5$ | $CH_3$ | H | $CH_2CH_2C_6H_5$ | 7.2 s (5H); 4.7–4.3 m (1H); 3.8–2.8 m (5H); 2.8–2.0 m (2H); 2.3 s (3H); 1.9–1.4 m (10H) |
| 111 | 1 | H | $C_2H_5$ | $(CH_2)_5$ | $C_2H_5$ | H | $CH_2$-$CH_2$-$C_6H_4$-2-$CH_3$ | 7.2 s (4H); 4.7–4.3 m (1H); 4.2 q (2H) 3.8–2.8 m (7H); 2.2 s (3H); 2.8–2.0 m (2H); 1.9–1.4 m (10H); 1.3 t (3H) 1.1 t (3H) |

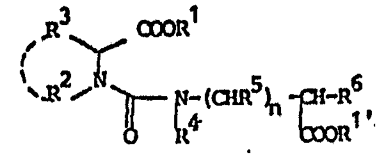

| | n | $R^{1}$ | $R^{1'}$ | $R^2$ — $R^3$ | $R^4$ | $R^5$ | $R^6$ | |
|---|---|---|---|---|---|---|---|---|
| 112 | 1 | H | H | $(CH_2)_5$ | $CH(CH_3)_2$ | H | $CH_2-CH_2-C_6H_3-2-Cl-4-OCH_3$ | 7.1-6.5 m (3H); 4.7-4.3 m (1H); 3.8-2.8 m (6H); 4.0 s (3H); 2.8-2.0 m (2H); 1.9-1.4 m (10H); 1.1 d (6H); |
| 113 | 1 | H | $C_2H_5$ | $(CH_2)_5$ | 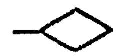 | H | $CH_2-CH_2-C_6H_5$ | 7.2 s (5H); 4.7-4.3 m (1H); 3.8-2.8 m (6H); 4.2 q (2H); 2.8-2.0 m (2H); 1.9-1.4 m (16H); 1.3 t (3H) |
| 114 | 1 | H | H | $(CH_2)_5$ | 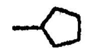 | H | $CH_2-CH_2-C_6H_5$ | 7.2 s (5H); 4.7-4.4 m (1H); 3.8-2.8 m (6H); 2.8-2.0 m (2H); 1.9-1.4 m (18H) |
| 115 | 1 | H | H | $(CH_2)_5$ | $CH_2-CH=CH-CH_3$ | H | $CH_2-CH_2-$ 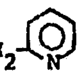 | 8.6-7.2 m (4H); 5.8-5.4 m (2H); 4.9-4.2 m (5H); 3.8-2.9 m (5H); 2.8-2.1 m (2H); 1.9-1.4 m + s (13H) |
| 116 | 1 | H | $C_2H_5$ | $(CH_2)_5$ | $CH_2-CH(CH_3)_2$ | H | $CH_2-CH_2-C_6H_5$ | 7.2 s (5H); 4.7-4.4 m (1H); 4.2 q (2H) 3.8-3.1 m (7H); 2.9-2.4 m (2H); 1.9-1.4 m (11H); 1.1 d (6H) |
| 117 | 1 | H | H | $(CH_2)_5$ | $CH_3$ | $CH_3$ | $\underline{n}-C_4H_9$ | 4.7-4.4 m (1H); 3.8-3.1 m (4H); 2.2 s (3H); 1.9-1.4 m (14H); 1.0 d + t (3H) |
| 118 | 1 | H | H | $(CH_2)_5$ | $C_2H_5$ | H | $CH_2-CH_2-CH(CH_3)_2$ | 4.7-4.4 m (1H); 3.8-3.1 m (7H); 1.9-1.4 m (13H); 1.0 d + t (9H) |
| 119 | 1 | H | $C_2H_5$ | $(CH_2)_5$ | $CH(CH_3)_2$ | H | $CH_2-SO_2-C_6H_5$ | 7.4-6.8 m (5H); 4.7-4.4 m (1H); 4.2 q (2H); 3.8-3.0 m (8H); 1.9-1.4 m (8H); 1.3 t (3H); 1.1 d (6H) |

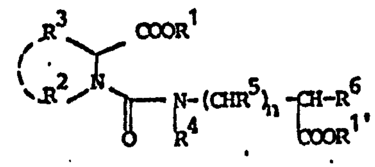

| | n | $R^1$ | $R^{1'}$ | $R^2$ — $R^3$ | $R^4$ | $R^5$ | $R^6$ | |
|---|---|---|---|---|---|---|---|---|
| 120 | 1 | H | H | 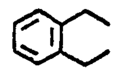 | $CH_3$ | H | $CH_2CH_2C_6H_4$-4-F | 7.3-6.9 m (8H); 4.7-4.2 m (3H); 3.5-2.9 m (3H); 2.8-2.3 m (4H); 1.9-1.4 m (2H); 2.2 s (3H) |
| 121 | 1 | H | $C_2H_5$ | " | $CH_3$ | H | $CH_2CH_2C_6H_4$-4-F | 7.3-6.9 m (8H); 4.7-4.3 m (3H); 4.2 q (2H); 3.5-2.9 m (3H); 2.8-2.3 m (4H); 1.9-1.4 m (2H); 2.2 s (3H); 1.2 t (3H) |
| 122 | 1 | H | H | " | $C_2H_5$ | H | $CH_2CH_2$-$C_6H_4$-4-$OCH_3$ | 7.2-6.5 m (8H); 4.7-4.3 m (3H); 3.8-2.9 m (5H); 4.0 s (3H); 2.8-2.3 m (4H) 1.9-1.4 m (2H); 1.1 t (3H) |
| 123 | 1 | H | $C_2H_5$ | " | $C_2H_5$ | H | $CH_2CH_2$-$C_6H_4$-4-$OCH_3$ | 7.2-6.5 m (8H); 4.7-4.3 m (3H); 4.2 q (2H); 3.8-2.9 m (5H); 4.0 s (3H); 2.8-2.3 m (4H); 1.9-1.4 m (2H); 1.3 t (3H); 1.1 t (3H) |
| 124 | 1 | H | H | " | $CH(CH_3)_2$ | H | $CH_2CH_2C_6H_4$-2-$CH_3$ | 7.2-6.8 m (8H); 4.7-4.3 m (3H); 4.0-2.9 m (4H); 2.8-2.3 m (4H); 2.3 s (3H); 1.9-1.4 m (2H); 1.0 d (6H) |
| 125 | 1 | H | $C_2H_5$ | " | $CH(CH_3)_2$ | H | $CH_2CH_2$-$C_6H_4$-2-$CH_3$ | 7.2-6.8 m (8H); 4.7-4.3 m (3H); 4.2 q (2H); 4.0-2.9 m (4H); 2.8-2.3 m (4H); 2.3 s (3H); 1.9-1.4 m (2H); 1.3 t (3H); 1.0 d (6H) |
| 126 | 1 | H | H | " | $CH_2$-CH=$CH_2$ | H | $CH_2CH_2$-$CH(CH_3)_2$ | 7.2 s (4H); 5.8 m (1H); 5.0-4.3 m (7H); 3.5-2.9 m (3H); 2.8-2.3 m (2H); 1.9-1.4 m (5H); 1.0 d (6H) |

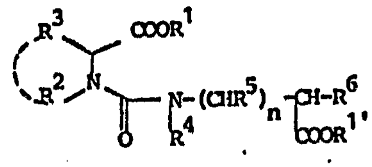

| | n | $R^1$ | $R^{1'}$ | $R^2$ — $R^3$ | $R^4$ | $R^5$ | $R^6$ | |
|---|---|---|---|---|---|---|---|---|
| 127 | 1 | H | H | 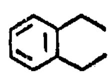 | $CH_3$ | H | $CH_3$ | 7.2 s (4H); 4.7-4.3 m (3H); 3.5-2.9 m (3H); 2.8-2.3 m (2H); 2.3 s (3H); 1.1 d (6H) |
| 128 | 1 | H | H | " | 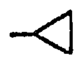 | H | $CH_2-S-C_6H_5$ | 7.3-6.9 m (9H); 4.7-4.3 m (3H); 3.5-2.9 m (6H); 1.1-0.6 m (4H) |
| 129 | 1 | H | H | " | $C_2H_5$ | H | $CH_2-CH_2-C_6H_4-4-N(CH_3)_2$ | 7.2-6.4 m (8H); 4.7-4.3 m (3H); 3.5-2.9 m (5H); 2.9 s (6H); 2.8-2.3 m (4H); 1.9-1.4 m (2H); 1.1 t (3H) |
| 130 | 1 | H | $C_2H_5$ | " | $n-C_4H_9$ | $CH_3$ | $CH_2-CH_2-C_6H_3-3-CN$ | 7.6-7.0 m (8H); 4.7-4.3 m (3H); 4.2q (2H); 3.8-2.9 m (5H); 2.8-2.3 m (4H); 1.9-1.4 m (6H); 1.3 t (3H); 1.0 t (3H) |
| 131 | 1 | H | H | " | $CH_2-CH_2-CH_3$ | H | $CH_2-CH_2-$ 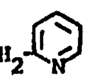 | 8.6-7.2 m (8H); 4.7-4.3 m (3H); 3.8-2.9 m (5H); 2.8-2.3 m (4H); 1.9-1.4 m (4H); 1.0 t (3H) |
| 132 | 1 | H | H | " | $CH_3$ | H | $CH_2-CH_2-$ 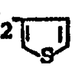 | 7.3-6.8 m (8H); 4.7-4.3 m (3H); 3.5-2.9 m (3H); 2.3 s (3H); 2.8-2.3 m (4H); 1.9-1.4 m (2H) |
| 133 | 1 | H | H | " | 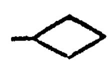 | H | $CH_2-CH_2-$ 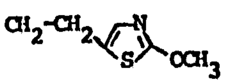 | 8.0 s (1H); 7.2 s (4H); 4.7-4.3 m (3H); 4.0-2.9 m (4H); 2.9-2.3 m (4H); 3.9 s (3H); 1.9-1.2 m (8H) |
| 134 | 1 | H | $C_2H_5$ | " | $C_2H_5$ | H | $CH_2-CH_2-C_6H_3-(OCH_2O)-3.4$ | 7.2-6.2 m (7H); 5.0 s (2H); 4.7-4.3 m (3H); 4.2 q (2H); 3.9-2.9 m (5H); 2.9-2.3 m (4H); 1.9-1.4 m (2H); 1.3 t (3H) |

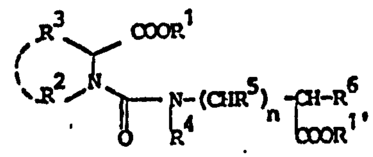

| | n | $R^1$ | $R^{1'}$ | $R^2$ — $R^3$ | $R^4$ | $R^5$ | $R^6$ | |
|---|---|---|---|---|---|---|---|---|
| 135 | 1 | H | H | 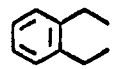 | $CH_3$ | $CH_3$ | $CH_2-CH_2-C_6H_4-O-C_6H_5-4$ | 7.2-6.7 m (13H); 4.7-4.3 m (3H); 4.0-2.9 m (2H); 2.9-2.3 m (2H); 2.4 s (3H); 1.9-1.4 m (2H); 1.1 d (3H) |
| 136 | 0 | H | H | " | $CH_3$ | - | $CH_2-CH_2-C_6H_5$ | 7.2 s (9H); 4.7-4.3 m (4H); 2.9-2.3 m (4H); 2.3 s (3H);1.9-1.4 m (2H) |
| 137 | 0 | H | $C_2H_5$ | " | $CH_3$ | - | $CH_2-CH_2-C_6H_5$ | 7.2 s (9H); 4.7-4.3 m (4H); 4.2 q (2H) 2.9-2.3 m (4H); 2.3 s (3H) 1.9-1.4 m (2H); 1.3 t (3H) |
| 138 | 0 | H | $C_2H_5$ | " | $C_2H_5$ | - | $CH_2-CH_2-C_6H_4-4-F$ | 7.3-6.9 m (8H); 4.7-4.3 m (4H); 4.2 q (2H); 3.6-2.9 m (2H); 2.9-2.3 m (4H); 1.9-1.4 m (2H); 1.3 t (3H); 1.0 t (3H) |
| 139 | 0 | H | H | " | $CH(CH_3)_2$ | - | $CH_2-CH_2-$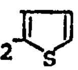 | 7.3-6.8 m (7H); 4.7-4.3 m (4H); 4.0-3.5 m (1H); 2.9-2.3 m (4H); 1.9-1.4 m (2H); 1.1 d (6H) |
| 140 | 0 | H | $C_2H_5$ | " | $CH(CH_3)_2$ | - | $CH_2-CH_2-C_6H_3-(OCH_3)_2-3.4$ | 7.3-6.2 m (7H); 4.7-4.3 m (4H); 4.2 q (2H); 4.0-3.5 m (1H); 4.0 s (6H); 2.9-2.3 m (4H); 1.9-1.4 m (2H); 1.3 t (3H); 1.1 d (6H) |
| 141 | 0 | H | H | " | $CH_2-CH=CH_2$ | - | $CH_2-CH_2-C_6H_4-2-CH_3$ | 7.2 s (8H); 5.8 m (1H); 5.0-4.2 m (6H) 2.9-2.3 m (4H); 2.2 s (3H); 1.9-1.4 m (2H) |
| 142 | 0 | H | $C_2H_5$ | " | 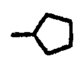 | - | $CH_2CH_2-C_6H_5-2-Cl-4-OCH_3$ | 7.2-6.6 m (7H); 4.6-4.3 m (4H);4.2 q (2H); 4.0-3.5 m (1H); 4.0 s (3H); 2.9-2.3 m (4H); 1.9-1.3 m (10H); 1.3t, (3H) |

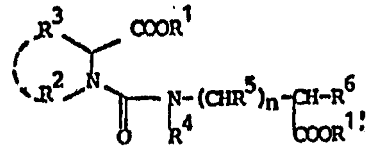

| | n | $R^1$ | $R^{1'}$ | $R^2$ — $R^3$ | $R^4$ | $R^5$ | $R^6$ | |
|---|---|---|---|---|---|---|---|---|
| 143 | 0 | H | H | 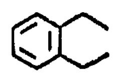 | $C_4H_9$ | - | $CH_2CH_2-C_6H_3-2.6-Cl_2$ | 7.4-6.8 m (7H); 4.7-4.3 m (4H); 3.8-3.1 m (2H); 2.9-2.3 m (4H); 1.9-1.4 m (8H); 1.0 t (3H) |
| 144 | 1 | H | H | 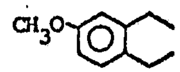 | $CH_3$ | H | $CH_2-CH_2-C_6H_5$ | 7.2-6.5 m (8H); 4.7-4.3 m (3H); 4.0 s (3H); 3.5-2.9 m (3H); 2.8-2.3 m (4H); 1.9-1.4 m (2H); 2.2 s (3H) |
| 145 | 1 | H | $C_2H_5$ | " | $CH_3$ | H | $CH_2-CH_2-C_6H_5$ | 7.2-6.5 m (8H); 4.7-4.3 m (3H); 4.2 q (2H); 4.0 s (3H); 3.6-2.9 m (5H); 2.8-2.3 m (4H); 2.2 s (3H); 1.9-1.4 m (2H); 1.2 t (3H) |
| 146 | 1 | H | H | " | $C_2H_5$ | H | $CH_2CH_2-C_6H_4-4-F$ | 7.3-6.5 m (7H); 4.7-4.3 m (3H); 4.0 s (3H); 3.6-2.9 m (5H); 2.8-2.3 m (4H); 1.9-1.4 m (2H); 1.0 t (3H) |
| 147 | 1 | H | $C_2H_5$ | " | $CH(CH_3)_2$ | H | $CH_2-CH_2-C_6H_4-2-CH_3$ | 7.3-6.5 m (7H); 4.7-4.3 m (3H); 4.2q (2H); 4.0 s (3H); 4.0-2.9 m (4H); 2.8-2.3 m (4H); 2.2 s (3H); 1.3 t (3H); 1.0 d (6H) |
| 148 | 1 | H | H | " | $CH_2-C{\equiv}CH$ | H | $CH_2-CH_2-C_6H_4-4-CH_3$ | 7.3-6.5 m (7H); 4.7-4.0 m (5H); 3.5-2.9 m (3H); 4.0 s (3H); 2.8-2.3 m (4H); 2.2 s (3H); 1.9-1.4 m (3H) |
| 149 | 1 | H | H | " | $CH_2-CH(CH_3)-CH_3$ | $CH_3$ | $CH_2-CH_2-CH(CH_3)_2$ | 6.8-6.4 m (3H); 4.7-4.3 m (3H); 3.8-2.9 m (4H); 4.0 s (3H); 2.8-2.3 m (2H); 1.9-1.4 m (6H); 1.0 d (15H) |

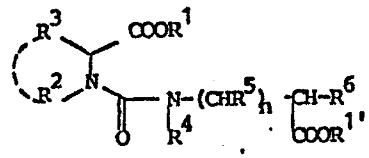

| | n | $R^1$ | $R^{1'}$ | $R^2$ — $R^3$ | $R^4$ | $R^5$ | $R^6$ | |
|---|---|---|---|---|---|---|---|---|
| 150 | 1 | H | H | 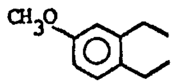 | 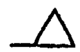 | H | $CH_2$-$CH_2$-$C_6H_4$-4-Cl | 7.3-6.5 m (7H); 4.7-4.3 m (3H); 3,8-2.9 m (4H); 4.0 s (3H); 2.8-2.3 m (4H); 1.9-1.4 m (2H); 1.1-0.6 m (4H) |
| 151 | 1 | H | H | " | $C_2H_5$ | H | 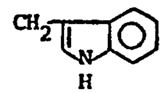 | 8.0 s (1H); 7.6-6.5 m (8H); 4.7-4.3 m (3H); 3.8-2.9 m (5H); 4.0 s (3H); 2.8-2.3 m (4H); 1.2 t (3H) |
| 152 | 1 | H | $C_2H_5$ | " | $C_2H_5$ | H | 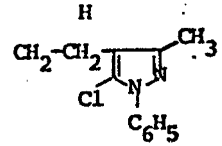 | 7.3-6.5 m (8H); 4.7-4.3 m (3H); 4.2 q (2H); 4.0 s (3H); 3.8-2.9 m (5H); 2.8-2.3 m (4H); 2.2 s (3H); 1.9-1.4 m (2H); 1.3 t (3H); 1.1 t (3H) |
| 153 | 1 | H | H | " | $CH(CH_3)_2$ | H | 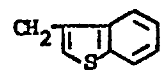 | 7.3-6.5 m (8H); 4.7-4.3 m (3H); 4.0 s (3H); 4.0-2.9 m (4H); 2.8-2.3 m (4H); 1.1 d (6H) |
| 154 | 1 | H | $C_2H_5$ | " | $CH_3$ | H | $CH_2CH_2SC_6H_5$ | 7.3-6.5 m (8H); 4.7-4.3 m (3H); 4.2 q (2H); 4.0 s (3H); 3.7-2.7 m (5H); 2.7-2.3 m (2H); 2.3 s (3H); 1.9-1.4 m (2H); 1.3 t (2H) |
| 155 | 1 | H | H | " | $CH_3$ | H | $CH_2$-$NHCOC_6H_5$ | 7.8-6.5 m (8H); 4.7-4.3 m (3H); 4.0 s (3H); 3.5-2.8 m (5H); 2.7-2.3 m (2H); 2.4 s (3H) |
| 156 | 1 | H | H | 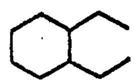 | $CH_3$ | H | $CH_2CH_2$-$C_6H_5$ | 7.2 s (5H); 4.7-4.3 m (1H); 3.5-2.9 m (5H); 2.8-2.4 m (2H); 2.3 s (3H); 1.9-1.3 m (14H) |

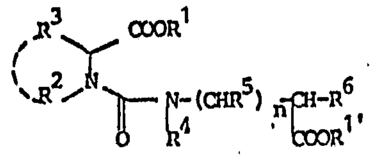

| | n | $R^1$ | $R^{1'}$ | $R^2$ — $R^3$ | $R^4$ | $R^5$ | $R^6$ | |
|---|---|---|---|---|---|---|---|---|
| 157 | 1 | H | $C_2H_5$ | 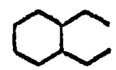 | $CH_3$ | H | $CH_2CH_2-C_6H_5$ | 7.2 s (5H); 4.7-4.3 m (1H); 4.2 q (2H); 3.5-2.9 m (5H); 2.8-2.4 m (2H); 2.3 s (3H); 1.9-1.3 m (14H); 1.2 t (3H) |
| 158 | 1 | H | H | " | $C_2H_5$ | H | $CH_2CH_2-C_6H_4-4-F$ | 7.3-6.9 m (4H); 4.7-4.3 m (1H); 3.8-2.9 m (7H); 2.8-2.4 m (2H); 1.9-1.3 m (14H); 1.1 t (3H) |
| 159 | 1 | H | $C_2H_5$ | " | $C_2H_5$ | H | $CH_2CH_2-C_6H_4-4-F$ | 7.3-6.9 m (4H); 4.7-4.3 m (1H); 4.2 q (2H); 3.8-2.9 m (7H); 2.8-2.4 m (2H) 1.9-1.3 m (14H); 1.3 t (3H);1.1 t (3H) |
| 160 | 1 | H | H | " | $C_2H_5$ | H | $CH_2-CH_2-C_6H_4-4-OCH_3$ | 6.9-6.4 m (4H); 4.7-4.3 m (1H); 3.8-2.9 m (7H);4.0 s (3H); 2.8-2.4 m (2H); 1.9-1.3 m (14H); 1.1 t (3H) |
| 161 | 1 | H | $C_2H_5$ | " | $C_2H_5$ | H | $CH_2-CH_2-C_6H_4-4-OCH_3$ | 6.9-6.4 m (4H); 4.7-4.3 m (1H); 4.2 q (2H); 4.0 s (3H); 3.8-2.9 m (7H); 2.8-2.4 m (2H); 1.9-1.3 m (14H); 1.3t (3H); 1.1 t (3H) |
| 162 | 1 | H | H | " | $CH(CH_3)_2$ | H | $CH_2-CH_2-C_6H_4-2-CH_3$ | 7.2 s (4H); 4.7-4.3 m (1H); 3.9-2.9 m (6H); 2.8-2.4 m (2H); 2.2 s (3H); 1.9-1.3 m (14H); 1.0 d (6H) |
| 163 | 1 | H | $C_2H_5$ | " | $CH(CH_3)_2$ | H | $CH_2-CH_2-C_6H_4-2-CH_3$ | 7.2 s (4H); 4.7-4.3 m (1H); 4.2 q (2H); 3.9-2.9 m (6H); 2.8-2.4 m (2H); 2.2 s (3H); 1.9-1.3 m (14H); 1.2 t (3H); 1.0 d (6H) |

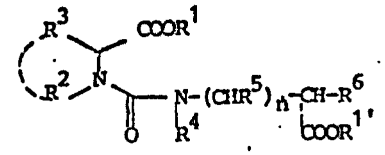

| | n | $R^1$ | $R^{1'}$ | $R^2$ — $R^3$ | $R^4$ | $R^5$ | $R^6$ | |
|---|---|---|---|---|---|---|---|---|
| 164 | 1 | H | H | 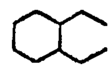 | $CH_2-CH=CH_2$ | H | $CH_2-CH_2-$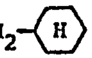 | 5.8 m (1H); 5.0-4.2 m (5H); 3.9-2.8 m (5H); 1.9-1.2 m (27H) |
| 165 | 1 | H | H | " | $CH_3$ | H | $C_2H_5$ | 4.7-4.3 m (1H); 3.5-2.9 m (5H); 2.4 s (3H); 1.9-1.3 m (14H); 0.9 t (3H) |
| 166 | 1 | H | H | " | 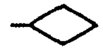 | H | $CH_2SOC_6H_5$ | 7.2-6.8 m (5H); 4.7-4.3 m (1H); 3.8-2.9 m (8H); 1.9-1.3 m (18H) |
| 167 | 1 | H | H | " | $C_2H_5$ | $CH_3$ | $CH_2NHCOC_6H_5$ | 7.7-7.1 m (5H); 4.7-4.3 m (1H); 3.8-2.9 m (8H); 1.9-1.3 m (12H); 1.0 d + t (6H) |
| 168 | 1 | H | $C_2H_5$ | " | $n-C_3H_7$ | H | $CH_2-CH_2-C_6H_3-2-NO_2-4-NHCOCH_3$ | 8.1-7.4 m (3H); 4.7-4.3 m (1H); 4.2 q (2H); 3.8-2.9 m (7H); 2.9-2.4 m (2H); 1.9-1.3 m (16H); 2.3 s (3H); 1.3 t (3H); 0.9 t (3H) |
| 169 | 1 | H | H | " | $CH_3$ | $CH_3$ | $CH_2CH_2-$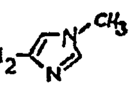 | 7.5-6.7 m (2H); 4.7-4.3 m (1H); 3.9-2.9 m (4H); 3.7 s (3H); 2.8-2.4 m (2H); 1.9-1.4 m (14H); 2.3 s (3H); 1.0 d (3H) |
| 170 | 1 | H | $C_2H_5$ | " | $C_2H_5$ | H | $CH_2C_6H_4-C_6H_5$ | 7.4-7.0 m (9H); 4.7-4.3 m (1H); 4.2 q (2H); 3.8-2.9 m (7H); 2.8-2.4 m (2H); 1.9-1.4 m (12H); 1.3 t (3H); 1.0 t (3H) |

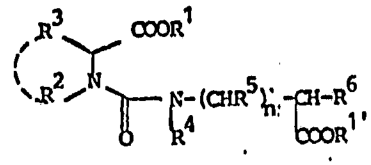

| | n | $R^1$ | $R^{1'}$ | $R^2$ — $R^3$ | $R^4$ | $R^5$ | $R^6$ | |
|---|---|---|---|---|---|---|---|---|
| 171 | 1 | H | H | 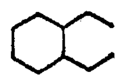 | $CH_3$ | $CH_3$ | $CH_2-CH_2-C_6H_3-(OCH_3)_2-2.5$ | 6.9-6.2 m (3H); 4.7-4.3 m (1H); 4.0 s (6H); 3.8-2.9 m (4H); 2.8-2.4 m (2H); 1.9-1.4 m (14H); 2.3 s (3H); 1.0 d (3H) |
| 172 | 1 | H | H | " | $C_2H_5$ | H | $-CH_2-$ 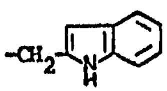 | 8.0 s (1H); 7.6-6.8 m (5H); 4.7-4.3 m (1H); 3.8-2.9 m (7H); 2.8-2.4 m (2H); 1.9-1.4 m (12H); 1.1 t (3H) |
| 173 | 1 | H | H | " | 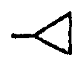 | H | $CH_2-SO_2-C_6H_5$ | 7.6-7.0 m (5H); 4.7-4.3 m (1H); 3.8-2.7 m (8H); 1.9-1.4 m (12H); 1.0-0.6 m (4H) |
| 174 | 0 | H | H | " | $CH_3$ | - | $CH_2CH_2C_6H_6$ | 7.2 s (5H); 4.7-4.3 m (2H); 3.8-2.9 m (2H); 2.8-2.4 m (2H); 1.9-1.4 m (14H); 2.4 s (3H) |
| 175 | 0 | H | $C_2H_5$ | " | $CH_3$ | - | $CH_2CH_2C_6H_5$ | 7.2 s (5H); 4.7-4.3 m (2H); 4.2 q (2H) 3.8-2.9 m (2H); 2.8-2.4 m (2H); 1.9-1.4 m (14H); 2.4 s (3H); 1.3 t (3H); |
| 176 | 0 | H | H | " | $C_2H_5$ | - | $CH_2CH_2C_6H_4-4-F$ | 7.4-6.9 m (4H); 4.7-4.3 m (2H); 3.8-2.9 m (4H); 2.8-2.4 m (2H); 1.9-1.4 m (14H); 1.0 t (3H) |
| 177 | 0 | H | $C_2H_5$ | " | $CH(CH_3)_2$ | - | $CH_2CH_2-$ 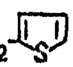 | 7.4-6.8 m (3H); 4.7-4.3 m (2H); 3.9-3.0 m (3H); 4.2q (2H); 2.8-2.4 m (2H); 1.9-1.4 m (14H); 1.3 t (3H); 1.0 d (6H) |

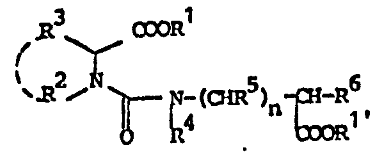

| | n | $R^1$ | $R^{1'}$ | $R^2$ — $R^3$ | $R^4$ | $R^5$ | $R^6$ | |
|---|---|---|---|---|---|---|---|---|
| 178 | 0 | H | H | 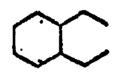 | $CH_2-CH=CH_2$ | - | $CH_2CH_2-C_6H_3(OCH_3)_2$-3.4 | 6.9-6.2 m (3H); 5.8 m (1H); 4.9-4.1 m (6H); 3.7-3.0 m (2H); 4.0 s (6H); 2.8-2.4 m (2H); 1.9-1.4 m (14H) |
| 179 | 0 | H | $C_2H_5$ | " | 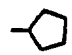 | - | $CH_2-CH_2-C_6H_4$-2-$CH_3$ | 7.2 s (4H); 4.7-4.3 m (1H); 4.2 q (2H); 3.8-2.9 m (3H); 2.8-2.4 m (2H); 2.2 s (3H); 1.9-1.4 m (22H); 1.3 t (3H) |
| 180 | 0 | H | H | " | n-$C_3H_7$ | - | $CH_2-CH_2-C_6H_3$-2.6-$Cl_2$ | 7.4-6.9 m (3H); 4.7-4.3 m (1H); 3.8-2.9 m (4H); 2.8-2.4 m (2H); 1.9-1.4 m (16H); 1.0 t (3H) |
| 181 | 1 | H | H | 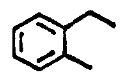 | $CH_3$ | H | $CH_2-CH_2C_6H_5$ | 7.2-6.5 m (9H); 4.9 t (1H); 3.8-3.0 m (3H); 2.9-2.4 m (4H); 2.4 s (3H); 1.9-1.4 m (2H) |
| 182 | 1 | H | $C_2H_5$ | " | $CH_3$ | H | $CH_2-CH_2-C_6H_5$ | 7.2-6.5 m (9H); 4.9 t (1H); 4.2 q (2H) 3.8-3.0 m (3H); 2.9-2.4 m (4H); 2.4 s (3H); 1.9-1.4 m (2H); 1.2 t (3H) |
| 183 | 1 | H | H | " | $C_2H_5$ | H | $CH_2-CH_2-C_6H_4$-4-F | 7.2-6.5 m (8H); 4.9 t (1H); 3.8-3.0 m (5H); 2.9-2.4 m (4H); 1.9-1.4 m (2H); 1.2 t (3H) |
| 184 | 1 | H | $C_2H_5$ | " | $C_2H_5$ | H | $CH_2-CH_2-C_6H_4$-4-F | 7.2-6.5 m (8H); 4.9 t (1H); 3.8-3.0 m (5H); 4.2 q (2H); 2.9-2.4 m (4H); 1.9-1.4 m (2H); 1.2 t (3H); 1.0 t (3H) |
| 185 | 1 | H | H | " | $C_2H_5$ | H | $CH_2-CH_2-C_6H_5$ | 7.2-6.5 m (9H); 4.9 t (1H); 3.8-3.0 m (5H); 2.9-2.4 m (4H); 1.9-1.4 m (2H); 1.1 t (3H) |

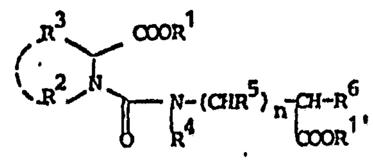

| | n | $R^1$ | $R^{1'}$ | $R^2$ — $R^3$ | $R^4$ | $R^5$ | $R^6$ | |
|---|---|---|---|---|---|---|---|---|
| 186 | 1 | H | $C_2H_5$ | 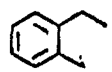 | $C_2H_5$ | H | $CH_2-CH_2C_6H_5$ | 7.2-6.5 m (9H); 4.9 t (1H); 3.8-3.0 m (5H); 4.2 q (2H); 2.9-2.4 m (4H); 1.9-1.4 m (2H); 1.3 t (3H); 1.1 t (3H) |
| 187 | 1 | H | H | " | $C_2H_5$ | H | $CH_2-CH_2-C_6H_4-OCH_3-4$ | 7.2-6.4 m (8H); 4.9 t (1H); 4.0 s (3H) 3.8-3.0 m (5H); 2.9-2.4 m (4H); 1.9-1.4 m (2H); 1.1 t (3H) |
| 188 | 1 | H | $C_2H_5$ | " | $C_2H_5$ | H | $CH_2-CH_2-C_6H_4-OCH_3-4$ | 7.2-6.4 m (8H); 4.9 t (1H); 4.2 q (2H) 4.0 s (3H); 3.8-3.0 m (5H); 2.9-2.4 m (4H); 1.9-1.4 m (2H); 1.3 t (3H); 1.1 t (3H) |
| 189 | 1 | H | H | " | $CH(CH_3)_2$ | H | $CH_2-CH_2-C_6H_4-2-CH_3$ | 7.2-6.5 m (8H); 4.9 t (1H); 3.8-3.0 m (4H); 2.9-2.4 m (4H); 2.2 s (3H); 1.9-1.4 m (2H); 1.0 d (6H) |
| 190 | 1 | H | $C_2H_5$ | " | $CH(CH_3)_2$ | H | $CH_2-CH_2-C_6H_4-2-Cl_3$ | 7.2-6.5 m (8H); 4.9 t (1H); 3.8-3.0 m (4H); 2.9-2.4 m (4H); 2.2 s (3H); 4.0 q (2H); 1.9-1.4 m (2H); 1.2 t (3H); 1.0 d (6H) |
| 191 | 1 | H | H | " | $CH_2-C\equiv CH$ | H | $CH_2-CH_2-CH_3$ | 7.2-6.5 m (4H); 4.9 t(1H); 3.8-3.0 m (5H); 2.9-2.6 m (2H); 1.9-1.4 m (5H); 1.0 t (3H) |
| 192 | 1 | H | $C_2H_5$ | " | $CH_2-CH=CH_2$ | $CH_3$ | $CH_2-CH_2-CH(CH_3)_2$ | 7.2-6.5 m (4H); 5.8 m (1H); 5.0-4.2 m (5H); 3.8-3.0 m (3H); 2.9-2.6 m (2H); 4.2 q (2H); 1.9-1.4 m (5H); 1.2 t (3H); 1.0 d (6H) |

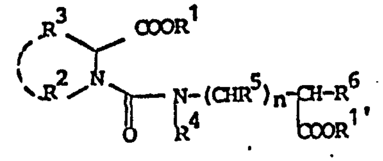

| | n | $R^1$ | $R^{1'}$ | $R^2$ — $R^3$ | $R^4$ | $R^5$ | $R^6$ | |
|---|---|---|---|---|---|---|---|---|
| 193 | 1 | H | H | 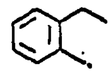 | 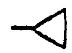 | H | $CH_2SC_6H_5$ | 7.3-6.5 m (9H); 4.9 t (1H); 3.5-3.0 m (4H); 2.9-2.4 m (4H); 1.0-0.6 m (4H) |
| 194 | 1 | H | $C_2H_5$ | " | $CH(CH_3)_2$ | H | $CH_2CH_2OC_6H_5$ | 7.2-6.4 m (9H); 4.9 t (1H); 4.2 q (2H) 3.9-3.0 m (6H); 3.0-2.6 m (2H); 1.9-1.4 m (2H); 1.2 t (3H); 1.0 d (6H) |
| 195 | 1 | H | H | " | $CH_3$ | H | 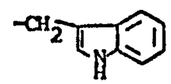 | 8.0 s (1H); 7.5-6.5 m (9H); 4.9 t (1H); 4.0-3.0 m (3H); 3.0-2.6 m (4H); 2.4 s (3H) |
| 196 | 1 | H | $C_2H_5$ | " | $C_2H_5$ | H | 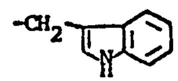 | 8.0 s (1H); 7.5-6.5 m (9H); 4.9 t (1H) 4.2 q (2H); 3.9-2.9 m (5H); 3.0-2.6 m (4H); 1.1 t (3H) |
| 197 | 1 | H | H | " | $C_2H_5$ | H | 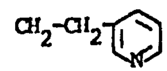 | 8.6-6.5 m (8H); 4.9 t (1H); 3.9-2.9 m (5H); 2.9-2.5 m (4H); 1.9-1.4 (2H); 1.1 t (3H) |
| 198 | 1 | H | H | " | $C_2H_5$ | H | $CH_2CH_2CH_2C_6H_5$ | 7.2-6.5 m (9H); 4.9 t (1H); 3.9-2.9 m (5H); 2.9-2.5 m (4H); 1.9-1.4 m (4H); 1.1 t (3H) |
| 199 | 1 | H | H | " | $C_2H_5$ | H | $CH_2C_6H_5$ | 7.2-6.5 m (9H); 4.9 t (1H); 3.9-2.9 m (5H); 2.9-2.5 m (4H); 1.1 t (3H) |
| 200 | 1 | H | H | " | $CH(CH_3)_2$ | $CH_3$ | $CH_2CH_2C_6H_5$ | 7.2-6.5 m (9H); 4.9 t (1H); 3.9-2.9 m (3H); 2.9-2.5 m (4H); 1.9-1.4 m (2H); 1.0 d + t (9H) |

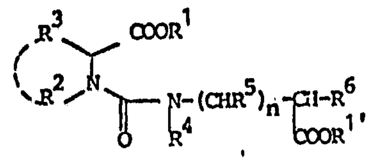

| | n | $R^1$ | $R^{1'}$ | $R^2$ — $R^3$ | $R^4$ | $R^5$ | $R^6$ | |
|---|---|---|---|---|---|---|---|---|
| 201 | O | H | $C_2H_5$ | 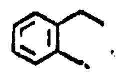 | $CH_3$ | - | $CH_2CH_2C_6H_5$ | 7.2-6.5 m (9H); 4.9-4.4 m (2H); 4.2 q (2H); 2.9-2.5 m (4H); 2.4 s (3H); 1.9-1.4 m (2H); 1.2 t (3H) |
| 202 | O | H | H | " | $CH_3$ | - | $CH_2CH_2C_6H_5$ | 7.2-6.5 m (9H); 4.9-4.4 m (2H); 2.9-2.5 m (4H); 2.4 s (3H); 1.9-1.4 m (2H) |
| 203 | O | H | $C_2H_5$ | " | $C_2H_5$ | - | $CH_2CH_2-C_6H_4-4-F$ | 7.2-6.5 m (8H); 4.9-4.4 m (2H); 3.8-3.1 m (2H); 4.2 q (2H); 2.9-2.5 m (4H); 1.9-1.4 m (2H); 1.2 t (3H); 1.0 t (3H) |
| 204 | O | H | H | " | $C_2H_5$ | - | $CH_2-CH_2-C_6H_4-4-F$ | 7.2-6.5 m (8H); 4.9-4.4 m (2H); 3.8-3.1 m (2H); 2.9-2.5 m (4H); 1.9-1.4 m (2H); 1.0 t (3H) |
| 205 | O | H | $C_2H_5$ | " | $CH(CH_3)_2$ | - | $CH_2CH_2-C_6H_4-4-OCH_3$ | 7.2-6.3 m (8H); 4.9-4.4 m (2H); 4.0-3.6 m (1H); 4.2 q (2H); 4.0 s (3H); 2.9-2.5 m (4H); 1.9-1.4 m (2H); 1.2 t (3H); 1.0 d (6H) |
| 206 | O | H | H | " | $CH(CH_3)_2$ | - | $CH_2CH_2C_6H_4-4-OCH_3$ | 7.2-6.3 m (8H); 4.9-4.4 m (2H); 4.0 s (3H); 4.0-3.6 m (1H); 2.9-2.5 m (4H); 1.9-1.4 m (2H); 1.0 d (6H) |
| 207 | O | H | H | " | $CH_2-CH=CH_2$ | - | $CH_2CH_2-C_6H_3(OCH_2O)-3.4$ | 7.2-6.2 m (7H); 5.8 m (1H); 5.0 s (2H) 4.9-4.0 m (6H); 2.9-2.5 m (2H); 1.9-1.4 m (2H) |

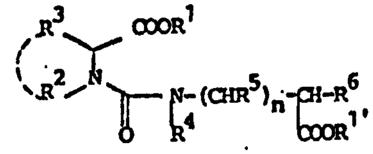

| | n | $R^1$ | $R^{1'}$ | $R^2$ — $R^3$ | $R^4$ | $R^5$ | $R^6$ | |
|---|---|---|---|---|---|---|---|---|
| 208 | 1 | H | H | 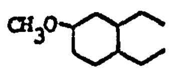 | $C_2H_5$ | H | $CH_2-CH_2-C_6H_5$ | 7.2 s (5H); 4.8-4.4 m (1H); 3.9-3.0 m (8H); 3.2 s (3H); 2.9-2.5 m (2H); 1.9-1.4 m (12H); 1.1 t (3H) |
| 209 | 1 | H | $C_2H_5$ | " | $C_2H_5$ | H | $CH_2-CH_2-C_6H_5$ | 7.2 s (5H); 4.8-4.4 m (1H); 4.2 q (2H); 3.2s (3H); 3.9-3.0 m (8H); 2.9-2.5 m (2H); 1.9-1.4 m (12H); 1.3 t (3H); 1.1 t (3H) |
| 210 | 1 | H | H | " | $CH_3$ | H | $CH_2-CH_2-C_6H_4-4-F$ | 7.4-6.9 m (4H); 4.7-4.4 m (1H); 3.9-3.0 m (6H); 3.2 s (3H); 2.9-2.5 m (2H) 2.3 s (3H); 1.9-1.4 m (12H) |
| 211 | 1 | H | $C_2H_5$ | " | $CH_3$ | H | $CH_2-CH_2-C_6H_4-4-F$ | 7.4-6.9 m (4H); 4.7-4.4 m (1H); 3.9-3.0 m (6H); 3.2 s (3H); 4.2 q (2H); 2.9-2.5 m (2H); 2.3 s (3H); 1.9-1.4 m (12H); 1.2 t (3H) |
| 212 | 1 | H | H | " | $C_2H_5$ | H | $CH_2-CH_2-C_6H_4-2-OCH_3$ | 7.0-6.5 m (4H); 4.7-4.4 m (1H); 3.9 s (3H); 3.9-3.0 m (8H); 3.2 s (3H); 2.9-2.5 m (2H); 1.9-1.4 m (12H); 1.0 t (3H) |
| 213 | 1 | H | $C_2H_5$ | " | $CH(CH_3)_2$ | H | $CH_2-CH_2-C_6H_3-2.6-Cl_2$ | 7.2-6.9 m (3H); 4.7-4.4 m (1H); 4.0-3.1 m (7H); 4.2 q (2H); 3.2 s (3H); 2.9-2.5 m (2H); 1.9-1.4 m (12H) 1.2 t (3H); 1.0 d (6H); |
| 214 | 1 | H | H | " | $CH(CH_3)_2$ | H | $CH_2NHCOC_6H_5$ | 7.8-7.3 m (5H); 4.7-4.4 m (1H); 3.9-3.0 m (9H); 3.2 s (3H); 1.9-1.4 m (10H); 1.0 d (6H) |

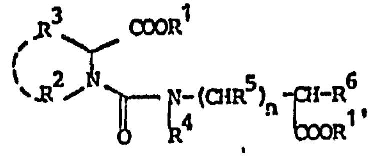

| | n | $R^1$ | $R^{1'}$ | $R^2$ — $R^3$ | $R^4$ | $R^5$ | $R^6$ | |
|---|---|---|---|---|---|---|---|---|
| 215 | 1 | H | $C_2H_5$ | 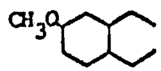 | $CH(CH_3)_2$ | H | $CH_2NHCOCH_3$ | 4.7-4.4 m (1H); 4.0-3.0 m (9H); 3.2 s (3H); 2.4 s (3H); 1.9-1.4 m (10H); 4.2 q (2H); 1.2 t (3H); 1.0 d (6H) |
| 216 | 1 | H | $C_2H_5$ | " | $C_2H_5$ | H | $CH_2$- 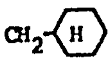 | 4.7-4.4 m (1H); 4.2 q (2H); 3.8-3.0 m (8H); 3.2 s (3H); 1.9-1.4 m (23H); 1.2 t (3H); 1.0 t (3H) |
| 217 | 1 | H | H | " | $CH_2-CH=CH_2$ | H | $CH_2$- 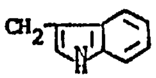 | 8.0 s (1H); 7.6-6.6 m (5H); 5.8 m (1H); 5.0 m (5H); 4.7-4.4 m (1H); 3.9-3.0 m (6H); 2.9-2.4 m (2H); 3.2 s (3H); 1.9-1.4 m (10H) |
| 218 | 1 | H | H | " | 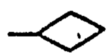 | H | $CH_2SOC_6H_5$ | 7.6-7.0 m (5H); 4.7-4.4 m (1H); 3.9-2.9 m (9H); 3.2 s (3H); 1.9-1.4 m (16H) |
| 219 | 1 | H | H | " | $-CH_2-C\equiv CH$ | H | $CH_2NHC_6H_5$ | 7.2-6.5 m (5H); 4.7-4.0 m (3H); 3.8-2.9 m (6H); 2.9-2.6 m (2H); 3.2 s (3H); 1.9-1.4 m (11H) |
| 220 | 1 | H | $C_2H_5$ | " | $CH_2-C\equiv C-CH_3$ | H | $CH_2CH_2CH_2C_6H_5$ | 7.2 s (5H); 4.7-4.0 m (3H); 4.2 q (2H) 3.8-2.9 m (6H); 3.2 s (3H); 2.9-2.4 m (2H); 1.9-1.3 m + s (17H); 1.2 t (3H) |

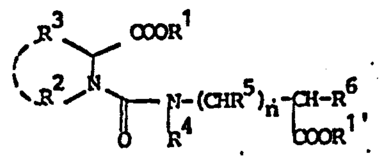

| | n | $R^1$ | $R^{1'}$ | $R^2$ — $R^3$ | $R^4$ | $R^5$ | $R^6$ | |
|---|---|---|---|---|---|---|---|---|
| 221 | 1 | H | H | 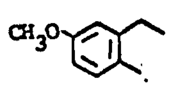 | $C_2H_5$ | H | $CH_2CH_2C_6H_5$ | 7.2-6.3 m (8H); 4.9 t (1H); 3.9 s (3H); 3.9-3.0 m (5H); 2.9-2.4 m (4H); 1.9-1.4 m (2H); 1.1 t (3H) |
| 222 | 1 | H | $C_2H_5$ | " | $C_2H_5$ | H | $CH_2CH_2C_6H_5$ | 7.2-6.3 m (8H); 4.9 t (1H); 4.2 q (2H) 3.9 s (3H); 3.9-3.0 m (5H); 2.9-2.4 m (4H); 1.9-1.4 m (2H); 1.2 t (3H); 1.0 t (3H) |
| 223 | 1 | H | H | " | $CH(CH_3)_2$ | H | $CH_2CH_2C_6H_4$-4-F | 7.2-6.3 m (7H); 4.9 t (1H); 3.9 s (3H); 3.9-3.0 m (4H); 2.9-2.4 m (4H); 1.9-1.4 m (2H); 1.0 d (6H) |
| 224 | 1 | H | $C_2H_5$ | " | $CH(CH_3)_2$ | H | $CH_2CH_2$-$C_6H_4$-4-F | 7.2-6.3 m (7H); 4.9 t (1H); 4.2 q (2H); 3.9 s (3H); 3.9-3.0 m (4H); 2.9-2.4 m (4H); 1.9-1.4 m (2H); 1.3 t (3H); 1.0 d (6H); |
| 225 | 1 | H | H | " | $CH_3$ | $CH_3$ | $CH_2CH_2$- 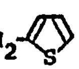 | 7.3-6.5 m (6H); 4.9 t (1H); 3.9 s (3H); 3.9-3.0 m (2H); 2.4 s (3H); 2.9-2.4 m (4H); 1.9-1.4 m (2H), 1.0 d (3H) |
| 226 | 1 | H | H | " | $C_2H_5$ | H | $CH_2$-$CH_2$-$C_6H_3$-2.6-$Cl_2$ | 7.3-6.3 m (6H); 4.9 t (1H); 3.9 s (3H) 3.9-3.0 m (5H); 2.9-2.4 m (4H); 1.9-1.4 m (2H); 1.2 t (3H) |
| 227 | 1 | H | H | " | $CH_2CH_2CH_3$ | H | $CH_2$-$CH_2$-$C_6H_4$-2-$CH_3$ | 7.3-6.3 m (7H); 4.9 t (1H); 3.9 s (3H) 3.9-3.0 m (5H); 2.9-2.4 m (4H); 2.2 s (3H); 1.9-1.4 m (4H); 1.0 t (3H) |

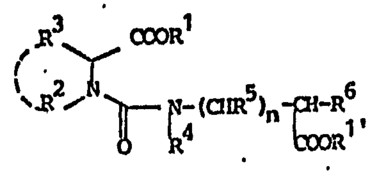

| | n | $R^1$ | $R^{1'}$ | $R^2$ — $R^3$ | $R^4$ | $R^5$ | $R^6$ | |
|---|---|---|---|---|---|---|---|---|
| 228 | 1 | H | H | 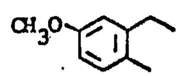 | $CH_2-CH=CH_2$ | H | $CH_2-CH_2-C_6H_3-(OCH_3)_2-3.4$ | 7.4-6.1 m (6H); 5.8 m (1H); 5.0-4.3 m (5H); 3.9 s (9H); 3.9-3.0 m (3H); 2.9-2.4 m (4H); 1.9-1.4 m (2H) |
| 229 | 1 | H | $C_2H_5$ | " | 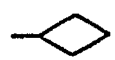 | H | $CH_2CH_2-C_6H_4-2-Cl-4-NO_2$ | 8.2-6.4 m (7H); 4.9 t (1H); 4.2 q (2H) 3.9 s (3H); 3.9-3.0 m (4H); 2.9-2.4 m (4H); 1.9-1.2 m (8H); 1.2 t (3H) |
| 230 | 1 | H | H | " | $C_2H_5$ | H | 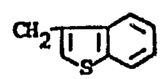 | 7.4-6.3 m (8H); 4.9 t (1H); 3.9 s (3H) 3.9-3.0 m (5H); 2.9-2.4 m (4H); 1.1 t (3H) |
| 231 | 1 | H | H | " | $C_2H_5$ | H | $CH_2-CH_2-CH(CH_3)_2$ | 7.3-6.4 m (8H); 4.9 t (1H); 3.9 s (3H) 3.9-3.0 m (5H); 2.9-2.6 m (2H); 1.9-1.4 m (5H); 1.2 t (3H); 1.0 d (6H) |
| 232 | 1 | H | $C_2H_5$ | " | $CH(CH_3)_2$ | H | $CH_2CH_3$ | 7.3-6.4 m (3H); 4.9 t (1H); 4.2 q (2H); 3.9 s (3H); 3.9-3.0 m (4H); 2.9-2.6 m (2H); 1.9-1.4 m (2H); 1.2 t (3H); 1.0 d + t (9H) |
| 233 | 1 | H | $C_2H_5$ | " | $C_2H_5$ | H | $CH_2CH_2SC_6H_5$ | 7.3-6.4 m (8H); 4.9 t (1H); 4.2 q (2H); 3.9 s (3H); 3.9-3.0 m (5H); 2.9-2.6 m (2H); 1.9-1.4 m (2H); 1.2 t (3H); 1.0 t (3H) |

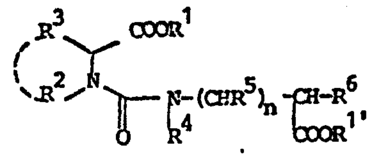

| | n | $R^1$ | $R^{1'}$ | $R^2$ — $R^3$ | $R^4$ | $R^5$ | $R^6$ | |
|---|---|---|---|---|---|---|---|---|
| 234 | 1 | H | H | 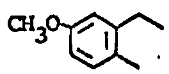 | $CH_2CH_2CH_2CH_3$ | $CH_3$ | $CH_2CH_2NHCOC_6H_5$ | 7.6-6.4 m (8H); 4.9 t (1H); 3.9 s (3H); 3.9-3.0 m (6H); 3.0-2.7 m (2H); 1.9-1.4 m (6H); 1.0 d + t (6H) |
| 235 | 1 | H | $C_2H_5$ | " | $CH_3$ | $CH_3$ | $n-C_6H_{13}$ | 7.3-6.5 m (3H); 4.9 t (1H); 4.2 q (2H); 3.9 s (3H); 3.9-3.0 m (2H); 2.9-2.6 m (2H); 2.4 s (3H); 1.9-1.4 m (10H); 1.2 t (3H); 1.0 d + t (6H) |
| 236 | 0 | H | H | " | $C_2H_5$ | - | $CH_2CH_2C_6H_5$ | 7.2-6.4 m (8H); 4.9-4.4 m (2H); 3.9 s (3H); 3.9-3.0 m (2H); 3.0-2.6 m (4H); 1.9-1.4 m (2H); 1.0 t (3H) |
| 237 | 0 | H | $C_2H_5$ | " | $CH(CH_3)_2$ | - | $CH_2CH_2-C_6H_4-4-F$ | 7.3-6.4 m (7H); 4.9-4.4 m (2H); 3.9 s (3H); 4.2 q (2H); 4.0-3.6 m (1H); 3.0-2.6 m (4H); 1.9-1.4 m (2H); 1.2 t (3H); 1.0 d (6H) |
| 238 | 0 | H | H | " | $CH_2-CH=CH_2$ | - | $CH_2CH_2-C_6H_4-4-OCH_3$ | 7.3-6.2 m (7H); 5.8 m (1H); 5.0-4.2 m (6H); 3.9 s (6H); 3.0-2.6 m (4H); 1.9-1.4 m (2H) |
| 239 | 0 | H | H | " | $CH(CH_3)_2$ | - | $CH_2CH_2$- 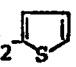 | 7.3-6.4 m (6H); 4.9-4.4 m (2H); 3.9 s (3H); 3.9-3.5 m (1H); 3.0-2.6 m (4H); 1.9-1.4 m (2H); 1.1 d (6H) |
| 240 | 0 | H | $C_2H_5$ | " | 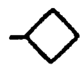 | - | $CH_2CH_2-C_6H_3(OCH_3)_2-2.5$ | 7.3-6.2 m (6H); 4.9-4.4 m (2H); 3.9 s (9H); 3.9-3.5 m (1H); 3.0-2.6 m (4H); 1.9-1.4 m (8H); 4.2 q (2H); 1.2 t (3H) |

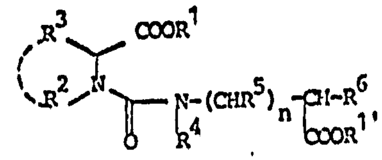

| | n | $R^1$ | $R^{1'}$ | $R^2$ — $R^3$ | $R^4$ | $R^5$ | $R^6$ | |
|---|---|---|---|---|---|---|---|---|
| 241 | 1 | H | H | 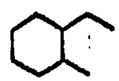 | $CH_3$ | H | $CH_2CH_2C_6H_5$ | 7.2 s (5H); 4.8-4.4 m (1H); 3.9-3.0 m (4H); 2.9-2.6 m (2H); 2.3 s (3H); 1.9-1.4 m (13H) |
| 242 | 1 | H | $C_2H_5$ | " | $CH_3$ | H | $CH_2CH_2C_6H_5$ | 7.2 s (5H); 4.8-4.4 m (1H); 4.2 q (2H) 3.9-3.0 m (4H); 2.9-2.6 m (2H); 2.3 s (3H); 1.9-1.4 m (13H); 1.2 t (3H) |
| 243 | 1 | H | H | " | $C_2H_5$ | H | $CH_2CH_2C_6H_4$-4-F | 7.3-6.9 m (4H); 4.8-4.4 m (1H); 3.9-3.0 m (6H); 2.9-2.6 m (2H); 1.9-1.4 m (13H); 1.0 t (3H) |
| 244 | 1 | H | $C_2H_5$ | " | $C_2H_5$ | H | $CH_2CH_2C_6H_4$-4-F | 7.3-6.9 m (4H); 4.8-4.4 m (1H); 4.2 q (2H); 3.9-3.0 m (6H); 2.9-2.6 m (2H); 1.9-1.4 m (13H); 1.2 t(3H); 1.0 t (3H) |
| 245 | 1 | H | H | " | $CH(CH_3)_2$ | H | $CH_2CH_2C_6H_4$-4-F | 7.3-6.9 m (4H); 4.8-4.4 m (1H); 3.9-3.0 m (5H); 2.9-2.6 m (2H); 1.9-1.4 m (13H); 1.0 d (6H) |

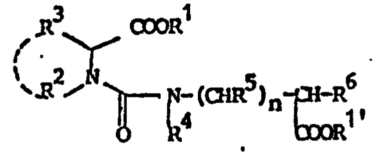

| | n | $R^1$ | $R^{1'}$ | $R^2$ — $R^3$ | $R^4$ | $R^5$ | $R^6$ | |
|---|---|---|---|---|---|---|---|---|
| 246 | 1 | H | $C_2H_5$ | 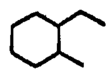 | $CH(CH_3)_2$ | H | $CH_2-CH_2-C_6H_4-4-F$ | 7.3-6.9 m (4H); 4.8-4.4 m (1H); 4.2 q (2H); 3.9-3.0 m (5H); 2.9-2.6 m (2H); 1.9-1.4 m (13H); 1.2 t (3H); 1.0 d (6H) |
| 247 | 1 | H | H | " | $CH_2-CH=CH_2$ | H | $CH_2-CH_2-C_6H_4-4-OCH_3$ | 7.0-6.3 m (4H); 5.8 m (1H); 5.0-4.2 m (5H); 3.9-3.0 m (4H); 3.9 s (3H); 2.9-2.6 m (2H); 1.9-1.4 m (13H) |
| 248 | 1 | H | $C_2H_5$ | " | $CH_2-CH=CH_2$ | H | $CH_2-CH_2-C_6H_4-4-OCH_3$ | 7.0-6.3 m (4H); 5.8 m (1H); 5.0-4.2 m (5H); 4.2 q (2H); 3.9-3.0 m (4H); 3.9 s (3H); 2.9-2.6 m (2H); 1.9-1.4 m (13H); 1.2 t (3H) |
| 249 | 1 | H | H | " | $C_2H_5$ | H | $CH_2-CH_2-C_6H_4-4-OCH_3$ | 7.0-6.3 m (4H); 4.8-4.4 m (1H); 3.9-3.0 m (6H); 2.9-2.4 m (4H); 1.9-1.4 m (13H); 1.0 t (3H) |
| 250 | 1 | H | $C_2H_5$ | " | $C_2H_5$ | H | $CH_2-CH_2-C_6H_4-4-OCH_3$ | 7.0-6.3 m (4H); 4.8-4.4 m (1H); 4.2 q (2H); 3.9-3.0 m (6H); 2.9-2.4 m (4H); 1.9-1.4 m (13H); 1.2 t (2H); 1.0 t (3H) |
| 251 | 1 | H | H | " | $C_2H_5$ | H | $CH_2-CH_2-C_6H_4-2-CH_3$ | 7.2 s (4H); 4.8-4.4 m (1H); 3.9-3.0 m (6H); 2.9-2.4 m (4H); 2.1 s (3H); 1.9-1.4 m (13H); 1.0 t (3H) |
| 252 | 1 | H | $C_2H_5$ | " | $C_2H_5$ | H | $CH_2-CH_2-C_6H_4-2-CH_3$ | 7.2 s (4H); 4.8-4.4 m (1H); 4.2 q (2H); 3.9-3.0 m (6H); 2.9-2.4 m (4H); 2.1 s (3H); 1.9-1.4 m (13H); 1.2 t (3H); 1.0 t (3H) |

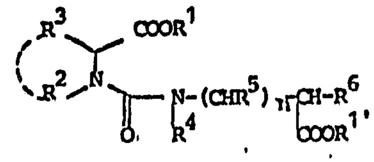

| | n | $R^1$ | $R^{1'}$ | $R^2$ — $R^3$ | $R^4$ | $R^5$ | $R^6$ | |
|---|---|---|---|---|---|---|---|---|
| 253 | 1 | H | H | 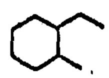 | $CH_2-C{\equiv}CH$ | H | $CH_2-CH_2-C_6H_4-2-CH_3$ | 7.2 s (4H); 4.8-4.0 m (3H); 3.9-3.0 m (4H); 2.9-2.4 m (4H); 2.1 s (3H); 1.9-1.4 m (14H); 1.0 t (3H) |
| 254 | 1 | H | $C_2H_5$ | " | $CH_2-C{\equiv}CH$ | H | $CH_2CH_2-C_6H_4-2-CH_3$ | 7.2 s (4H); 4.8-4.0 m (5H); 4.2 q (2H) 3.9-3.0 m (4H); 2.9-2.4 m (4H); 2.1 s (3H); 1.9-1.4 m (14H); 1.2 t (3H); 1.0 t (3H) |
| 255 | 1 | H | H | " | $C_2H_5$ | H | $CH_2SC_6H_5$ | 7.2 s (5H); 4.8-4.4 m (1H); 3.9-3.0 m (6H); 2.9-2.4 m (4H); 1.9-1.4 m (11H); 1.1 t (3H) |
| 256 | 1 | H | $C_2H_5$ | " | $C_2H_5$ | H | $CH_2SOC_6H_5$ | 7.5-7.0 m (5H); 4.8-4.4 m (1H); 4.2 q (2H); 3.9-3.0 m (6H); 2.9-2.4 m (4H); 1.9-1.4 m (11H); 1.2 t (3H); 1.0 t (3H) |
| 257 | 1 | H | $C_2H_5$ | " | $C_2H_5$ | H | $CH_2SO_2C_6H_5$ | 7.5 -7.0 m (5H); 4.8-4.4 m (1H); 4.2 (2H); 3.9-3.0 m (6H); 2.9-2.4 m (4H); 1.9-1.4 m (11H); 1.2 t (3H); 1.0 t (3H) |
| 258 | 1 | H | H | " | $C_2H_5$ | H | $CH_2NHCOC_6H_5$ | 7.7-7.2 m (5H); 4.7-4.4 m (1H); 3.9-3.0 m (8H); 2.9-2.4 m (2H); 1.9-1.4 m (11H); 1.0 t (3H) |
| 259 | 1 | H | H | " | $C_2H_5$ | H | $CH_2CH_2NHC_6H_5$ | 7.1-6.5 m (5H); 4.7-4.4 m (1H); 3.9-3.0 m (6H); 2.9-2.4 m (4H); 1.9-1.4 m (13H); 1.0 t (3H) |

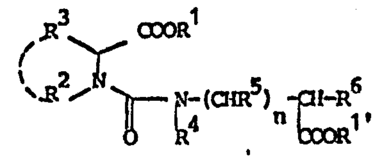

| | n | $R^1$ | $R^{1'}$ | $R^2$ — $R^3$ | $R^4$ | $R^5$ | $R^6$ | |
|---|---|---|---|---|---|---|---|---|
| 260 | 1 | H | H | 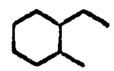 | $C_2H_5$ | H | $CH_2CH_2$- 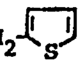 | 7.2-6.8 m (3H); 4.7-4.4 m (1H); 3.9-3.0 m (6H); 2.9-2.4 m (4H); 1.9-1.4 m (13H); 1.0 t (3H) |
| 261 | 1 | H | H | " | $C_2H_5$ | H | $CH_2CH_2CH_2CH_3$ | 4.7-4.4 m (1H); 3.9-3.0 m (6H); 2.9-2.4 m (2H); 1.9-1.4 m (17H); 1.0 t (6H) |
| 262 | 1 | H | H | " | $C_2H_5$ | H | $CH_2CH_2C_6H_3(OCH_3)_2$-3.4 | 6.9-6.2 m (3H); 4.7-4.4 m (1H); 3.9 s (6H); 3.9-3.0 m (6H); 2.9-2.4 m (4H); 1.9-1.4 m (13H); 1.0 t (3H) |
| 263 | 1 | H | H | " | $C_2H_5$ | H | $CH_2OC_6H_5$ | 7.0-6.5 m (5H); 4.7-4.4 m (1H); 3.9-3.0 m (8H); 2.9-2.4 m (2H); 1.9-1.4 m (11H); 1.0 t (3H) |
| 264 | 1 | H | H | " | $C_2H_5$ | H | $CH_2CH_2OC_6H_5$ | 7.0-6.5 m (5H); 4.7-4.4 m (1H); 3.9-3.0 m (8H); 2.9-2.4 m (2H); 1.9-1.4 m (13H); 1.0 t (3H) |
| 265 | 1 | H | H | " | $CH(CH_3)_2$ | H | $CH_2$ 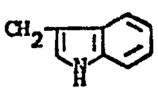 | 8.0 s (1H); 7.6-6.8 m (5H); 4.7-4.4 m (1H); 3.9-3.0 m (5H); 2.9-2.4 m (4H); 1.9-1.4 m (11H); 1.0 d (6H) |
| 266 | 0 | H | H | " | $C_2H_5$ | H | $CH_2CH_2C_6H_5$ | 7.2 s (5H); 4.8-4.3 m (2H); 3.9-3.0 m (3H); 2.9-2.4 m (2H); 1.9-1.4 m (13H); 1.0 t (3H) |
| 267 | 0 | H | $C_2H_5$ | " | $CH(CH_3)_2$ | - | $CH_2CH_2$-$C_6H_4$-4-F | 7.2-6.8 m (4H); 4.8-4.3 m (2H); 4.2q (2H); 3.9-3.0 m (2H); 2.9-2.4 m (2H); 1.9-1.4 m (13H); 1.2 t (3H); 1.0 d (6H) |

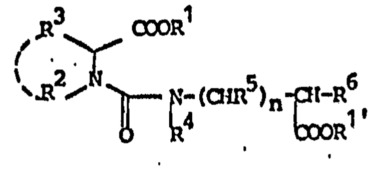

| | n | $R^1$ | $R^{1'}$ | $R^2$ — $R^3$ | $R^4$ | $R^5$ | $R^6$ | |
|---|---|---|---|---|---|---|---|---|
| 268 | 0 | H | H | 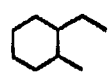 | $CH_2-CH=CH_2$ | - | $CH_2-CH_2-C_6H_5-(OCH_2O)-3.4$ | 6.9-6.2 m (3H); 5.8 m (1H); 5.0 s (2H); 5.0-4.2 m (6H); 3.9-3.4 m (1H); 2.9-2.4 m (2H); 1.9-1.4 m (13H) |
| 269 | 0 | H | $C_2H_5$ | " | $CH_2CH_2CH_3$ | - | $CH_2CH_2-C_6H_4-OCH_3-4$ | 7.0-6.4 m (4H); 4.8-4.3 m (2H); 4.2 q (2H); 3.9-3.0 m (3H); 3.9 s (3H); 2.9-2.5 m (2H); 1.9-1.4 m (15H); 1.2 t (3H); 1.0 t (3H) |
| 270 | 0 | H | H | " | 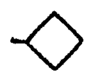 | - | $CH_2CH_2C_6H_4-2-CH_3$ | 7.2 s (4H); 4.8-4.3 m (2H); 3.9-3.1 m (2H); 2.9-2.5 m (2H); 2.1 s (3H); 1.9-1.4 m (19H) |
| 271 | 1 | H | H | 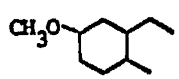 | $C_2H_5$ | H | $CH_2CH_2C_6H_5$ | 7.2 s (5H); 4.8-4.4 m (1H); 3.9-3.0 m (7H); 3.2 s (3H); 2.9-2.4 m (2H); 1.9-1.4 m (12H); 1.0 t (3H) |
| 272 | 1 | H | $C_2H_5$ | " | $C_2H_5$ | H | $CH_2CH_2C_6H_5$ | 7.2 s (5H); 4.8-4.4 m (1H); 4.2 q (2H); 3.9-3.0 m (7H); 3.2 s (3H); 2.9-2.4 m (2H); 1.9-1.4 m (12H); 1.2 t (3H); 1.0 t (3H) |
| 273 | 1 | H | $C_2H_5$ | " | $CH_3$ | H | $CH_2CH_2C_6H_5$ | 7.2 s (5H); 4.8-4.4 m (1H); 4.2 q (2H); 3.9-3.0 m (5H); 3.2 s (3H); 2.9-2.4 m (2H); 2.3 s (3H); 1.9-1.4 m (12H); 1.2 t (3H) |
| 274 | 1 | H | H | " | $CH_3$ | H | $CH_2CH_2C_6H_5$ | 7.2 s (5H); 4.8-4.4 m (1H); 3.9-3.0 m (5H); 3.2 s (3H); 2.9-2.4 m (2H); 2.3 s (3H); 1.9-1.4 m (12H) |

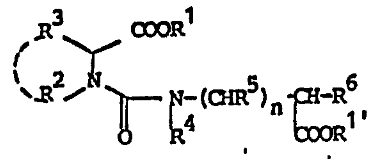

| | n | $R^1$ | $R^{1'}$ | $R^2$ — $R^3$ | $R^4$ | $R^5$ | $R^6$ | |
|---|---|---|---|---|---|---|---|---|
| 275 | 1 | H | H | 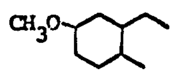 | $C_2H_5$ | H | $CH_2CH_2-C_6H_4-4-F$ | 7.4-6.9 m (4H); 4.8-4.4 m (1H); 3,9-3.0 m (7H); 3.2 s (3H); 2.9-2.4 m (2H); 1.9-1.4 m (12H); 1.0 t (3H) |
| 276 | 1 | H | $C_2H_5$ | " | $C_2H_5$ | H | $CH_2CH_2-C_6H_4-4-F$ | 7.4-6.9 m (4H); 4.8-4.4 m (1H); 3.9-3.0 m (7H); 4.2 q (2H); 3.2 s (3H); 2.9-2.4 m (2H); 1.9-1.4 m (12H); 1.2 t (3H); 1.0 t (3H) |
| 277 | 1 | H | H | " | $CH(CH_3)_2$ | H | $CH_2CH_2-C_6H_4-4-F$ | 7.4-6.9 m (4H); 4.8-4.4 m (1H); 3.9-3.0 m (6H); 3.2 s (3H); 2.9-2.4 m (2H); 1.9-1.4 m (12H); 1.0 d (6H) |
| 278 | 1 | H | $C_2H_5$ | " | $CH(CH_3)_2$ | H | $CH_2CH_2-C_6H_4-4-F$ | 7.4-6.9 m (4H); 4.8-4.4 m (1H); 3.9-3.0 m (6H); 4.2 q (2H); 3.2 s (3H); 2.9-2.4 m (2H); 1.9-1.4 m (12H); 1.2 t (3H); 1.0 d (6H) |
| 279 | 1 | H | $C_2H_5$ | " | $CH_2-CH=CH_2$ | H | $CH_2CH_2C_6H_4-4-OCH_3$ | 7.0-6.4 m (4H); 5.8 m (1H); 5.0-4.2 m (5H); 4.2 q (2H); 3.9 s (3H); 3.9-3.0 m (5H); 3.2 s (3H); 2.9-2.4 m (2H); 1.9-1.4 m (12H); 1.2 t (3H) |
| 280 | 1 | H | H | " | $CH_2-CH=CH_2$ | H | $CH_2CH_2-C_6H_4-4-OCH_3$ | 7.0-6.4 m (4H); 5.8 m (1H); 5.0-4.2 m (5H); 3.9 s (3H); 3.9-3.0 m (5H); 3.2 s (3H); 2.9-2.4 m (2H); 1.9-1.4 m (12H) |
| 281 | 1 | H | H | " | $C_2H_5$ | $CH_3$ | $CH_2CH_2-C_6H_4-4-OCH_3$ | 7.0-6.4 m (4H); 4.8-4.4 m (1H); 3.9-3.0 m (6H); 3.9 s (3H); 3.2 s (3H); 2.9-2.4 m (2H); 1.9-1.4 m (12H); 1.0 d + t (6H) |

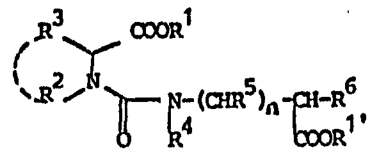

| | n | $R^1$ | $R^{1'}$ | $R^2$ — $R^3$ | $R^4$ | $R^5$ | $R^6$ | |
|---|---|---|---|---|---|---|---|---|
| 282 | 1 | H | $C_2H_5$ | 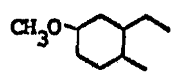 | $C_2H_5$ | $CH_3$ | $CH_2CH_2-C_6H_4-4-OCH_3$ | 7.0-6.4 m (4H); 4.8-4.4 m (1H); 4.2 q (2H); 3.9-3.0 m (6H); 3.9s (3H); 3.2 s (3H); 2.9-2.4 m (2H); 1.9-1.4 m (12H); 1.2 t (3H); 1.0 d + t (6H) |
| 283 | 1 | H | H | " | $C_2H_5$ | H | $CH_2CH_2-C_6H_4-2-CH_3$ | 7.2 s (4H); 4.8-4.4 m (1H); 3.9-3.0 m (7H); 3.2 s (3H); 2.9-2.4 m (2H); 2.1 s (3H); 1.9-1.4 m (12H); 1.0 t (3H) |
| 284 | 1 | H | $C_2H_5$ | " | $C_2H_5$ | H | $CH_2CH_2-C_6H_4-2-CH_2$ | 7.2 s (4H); 4.8-4.4 m (1H); 4.2 q (2H); 3.9-3.0 m (7H); 3.2 s (3H); 2.9-2.4 m (2H); 2.1 s (3H); 1.9-1.4 m (12H); 1.2 t (3H); 1.0 t (3H) |
| 285 | 1 | H | H | " | $CH_2-C\equiv CH$ | H | $CH_2CH_2-C_6H_4-2-CH_3$ | 7.2 s (4H); 4.8-4.4 m (1H); 4.3-3.0 m (7H); 3.2 s (3H); 2.9-2.4 m (2H); 2.1 s (3H); 1.9-1.4 m (13H) |
| 286 | 1 | H | $C_2H_5$ | " | $CH_2-C\equiv CH$ | H | $CH_2CH_2-C_6H_4-2-CH_3$ | 7.2 s (4H); 4.8-4.4 m (1H); 4.3-3.0 m (9H); 3.2 s (3H); 2.9-2.4 m (2H); 2.1 s (3H); 1.9-1.4 m (13H); 1.2 t (3H) |
| 287 | 1 | H | H | " | $C_2H_5$ | H | $CH_2SC_6H_5$ | 7.2 s (5H); 4.8-4.4 m (1H); 3.9-3.0 m (7H); 3.2 s (3H); 2.9-2.4 m (2H); 1.9-1.4 m (10H); 1.0 t (3H) |
| 288 | 1 | H | $C_2H_5$ | " | $C_2H_5$ | H | $CH_2SO\ C_6H_5$ | 7.4-7.0 m (5H); 4.8-4.4 m (1H); 4.2 q (2H); 3.9-3.0 m (7H); 3.2 s (3H); 2.9-2.4 m (2H); 1.9-1.4 m (10H); 1.2 t (3H); 1.0 t (3H) |

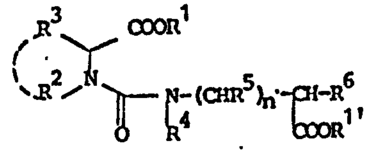

| | n | $R^1$ | $R^{1'}$ | $R^2$ — $R^3$ | $R^4$ | $R^5$ | $R^6$ | |
|---|---|---|---|---|---|---|---|---|
| 289 | 1 | H | $C_2H_5$ | 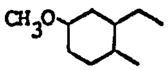 | $C_2H_5$ | H | $CH_2SO_2C_6H_5$ | 7.6-7.0 m (5H); 4.8-4.4 m (1H); 4.2 q (2H); 3.9-3.0 m (7H); 3.2 s (3H); 3.1-2.6 m (2H); 1.9-1.4 m (10H); 1.2 t (3H); 1.0 t (3H) |
| 290 | 1 | H | H | " | $C_2H_5$ | H | $CH_2NHCOC_6H_5$ | 7.9-7.3 m (5H); 4.8-4.4 m (1H); 3.9-2.8 m (9H); 3.2 s (3H); 1.9-1.4 m (10H); 1.0 t (3H) |
| 291 | 1 | H | H | " | $C_2H_5$ | H | $CH_2CH_2NHC_6H_5$ | 7.0-6.4 m (5H); 4.8-4.4 m (1H); 3.9-2.8 m (9H); 3.2 s (3H); 1.9-1.4 m (12H); 1.0 t (3H) |
| 292 | 1 | H | H | " | $C_2H_5$ | H | $CH_2CH_2$ 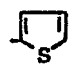 | 7.2-6.7 m (3H); 4.8-4.4 m (1H); 3.9-3.1 m (7H); 3.2 s (3H); 2.9-2.5 m (2H) 1.9-1.4 m (12H); 1.0 t (3H) |
| 293 | 1 | H | H | " | $C_2H_5$ | H | $CH_2CH_2CH_3$ | 4.8-4.4 m (1H); 3.9-3.1 m (7H); 3.2 s (3H); 1.9-1.4 m (14H); 1.0 t (6H) |
| 294 | 1 | H | H | " | $C_2H_5$ | $CH_3$ | $CH_2CH_2-CH(CH_3)_2$ | 4.8-4.4 m (1H); 3.9-3.1 m (6H); 3.2 s (3H); 1.9-1.4 m (15H); 1.0 t + d (12H) |
| 295 | 1 | H | H | " | 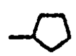 | H | $CH_2CH_2-C_6H_3(OCH_3)_2-3.4$ | 6.9-6.2 m (3H); 3.9 s (6H); 3.9-3.1 m (6H); 3.2 s (3H); 2.9-2.4 m (2H); 1.9-1.4 m (20H) |
| 296 | 1 | H | H | " | $CH(CH_3)_2$ | H | $CH_2OC_6H_5$ | 7.0-6.6 m (5H); 3.9-3.1 m (8H); 3.2 s (3H); 1.9-1.4 m (10H); 1.0 d (6H) |

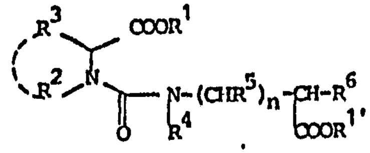

| | n | $R^1$ | $R^{1'}$ | $R^2$ — $R^3$ | $R^4$ | $R^5$ | $R^6$ | |
|---|---|---|---|---|---|---|---|---|
| 297 | 1 | H | H | 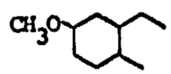 | $C_2H_5$ | H | $CH_2CH_2OC_6H_5$ | 7.1-6.6 m (5H); 4.8-4.4 m (1H); 3.9-3.1 m (9H); 3.2 s (3H); 2.0-1.3 m (12H); 1.0 t (3H) |
| 298 | 1 | H | H | " | $CH_3$ | H | 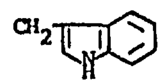 | 8.0 s(1H); 7.6-6.7 m (5H); 4.8-4.4 m (1H); 3.9-3.1 m (5H); 3.2 s (3H); 2.9-2.4 m (2H); 2.3 s (3H); 1.9-1.4 m (10H) |
| 299 | 1 | H | H | " | $CH(CH_3)_2$ | H | 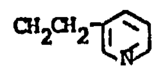 | 8.6-7.4 m (4H); 4.8-4.4 m (1H); 3.9-3.1 m (6H); 3.2 s (3H); 2.9-2.4 m (2H); 1.9-1.4 m (12H); 1.0 d (6H) |
| 300 | 1 | H | H | " | $CH_3$ | H | 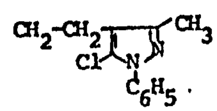 | 7.3 s (5H); 4.8-4.4 m (1H); 3.9-3.1 m (5H); 3.2 s (3H); 2.9-2.4 m (2H); 2.3 s (3H); 2.1 s (3H); 1.9-1.4 m (12H) |
| 301 | 0 | H | H | " | $C_2H_5$ | - | $CH_2CH_2C_6H_5$ | 7.2 s (5H); 4.8-4.3 m (2H); 3.9-3.1 m (4H); 3.2 s (3H); 2.9-2.4 m (2H); 1.9-1.4 m (12H); 1.0 t (3H) |
| 302 | 0 | H | $C_2H_5$ | " | $C_2H_5$ | - | $CH_2CH_2C_6H_5$ | 7.2 s (5H); 4.8-4.3 m (2H); 4.2 q (2H); 3.9-3.1 m (4H); 3.2 s (3H); 2.9-2.4 m (2H); 1.9-1.4 m (12H); 1.2 t (3H); 1.0 t (3H) |
| 303 | 0 | H | H | " | $C_2H_5$ | - | $CH_2CH_2-C_6H_4-4-F$ | 7.4-6.9 m (4H); 4.8-4.3 m (2H); 3.9-3.1 m (4H); 3.2 s (3H); 2.9-2.4 m (2H); 1.9-1.4 m (12H); 1.0 t (3H) |

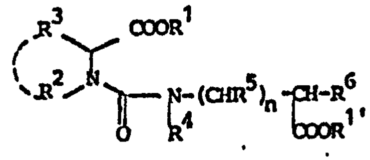

| | n | $R^1$ | $R^{1'}$ | $R^2$ — $R^3$ | $R^4$ | $R^5$ | $R^6$ | |
|---|---|---|---|---|---|---|---|---|
| 304 | 0 | H | $C_2H_5$ | 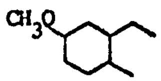 | $CH(CH_3)_2$ | - | $CH_2CH_2C_6H_3(OCH_2O)$-3.4 | 6.9-6.2 m (3H); 5.0 s (2H); 4.8-4.3 m (2H); 4.2 q (2H); 3.9-3.1 m (3H); 3.2 s (3H); 2.9-2.4 m (2H); 1.9-1.4 m (12H); 1.0 d (6H) |
| 305 | 0 | H | H | " | $CH_2$-CH=$CH_2$ | - | $CH_2$-$CH_2$-$C_6H_3$-2.6-Cl | 7.4-7.0 m (3H); 5.8 m (1H); 5.0-4.1 m (6H); 3.9-3.1 m (2H); 3.2 s (3H); 2.9-2.4 m (2H); 1.9-1.4 m (12H) |
| 306 | 0 | H | $C_2H_5$ | " | $CH_2CH_2CH_3$ | - | $CH_2CH_2$-$C_6H_4$-$OCH_3$- | 7.0-6.4 m (4H); 4.8-4.3 m (2H); 4.2 q (2H); 3.9-3.1 m (4H); 3.9 s (3H); 3.2 s (3H); 2.9-2.4 m (2H); 1.9-1.4 m (14H); 1.2 t (3H); 1.0 t (3H) |
| 307 | 0 | H | H | " | $CH_2$-C≡CH | - | $CH_2CH_2$- 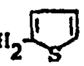 | 7.2-6.7 m (3H); 4.8-4.0 m (4H); 3.9-3.1 m (2H); 3.2 s (3H); 2.9-2.4 m (2H); 1.9-1.4 m (13H) |
| 308 | 0 | H | H | " | 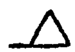 | - | $CH_2CH_2C_6H_3$-Cl-4-CN | 7.9-7.4 m (3H); 4.8-4.3 m (2H); 3.9-3.1 m (3H); 2.9-2.4 m (2H); 1.9-1.4 m (12H); 1.1-0.6 m (4H) |
| 309 | 0 | H | H | " | $CH_3$ | - | $CH_2CH_2$- 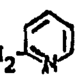 | 8.6-7.4 m (4H); 4.8-4.3 m (2H); 3.9-3.1 m (2H); 2.9-2.4 m (2H); 2.3 s (3H); 1.9-1.4 m (12H) |
| 310 | 1 | H | H | 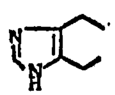 | $CH_3$ | H | $CH_2CH_2C_6H_5$ | 13.4 s (1H); 7.7 s (1H); 7.2 s (5H); 4.7-4.3 m (3H); 3.9-3.0 m (3H); 2.9-2.4 m (4H); 2.2 s (3H); 1.9-1.4 m (2H) |

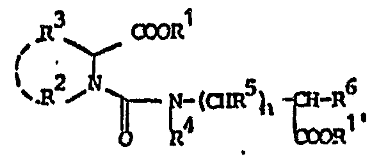

| | n | $R^1$ | $R^{1'}$ | $R^2$ — $R^3$ | $R^4$ | $R^5$ | $R^6$ | |
|---|---|---|---|---|---|---|---|---|
| 311 | 1 | H | $C_2H_5$ | 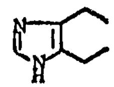 | $CH_3$ | H | $CH_2CH_2C_6H_5$ | 13.4 s (1H); 7.7 s (1H); 7.2 s (5H); 4.7-4.3 m (3H); 4.2 q (2H); 3.9-3.0 m (3H); 2.9-2.4 m (4H); 2.2 s (3H); 1.9-1.4 m (2H); 1.2 t (3H) |
| 312 | 1 | H | H | " | $C_2H_5$ | H | $CH_2CH_2C_6H_5$ | 13.4 s (1H); 7.7 s (1H); 7.2 s (5H); 4.7-4.3 m (3H); 3.9-3.1 m (5H); 2.9-2.4 m (4H); 1.9-1.4 m (2H); 1.0 t (3H) |
| 313 | 1 | H | $C_2H_5$ | " | $C_2H_5$ | H. | $CH_2CH_2C_6H_5$ | 13.4 s (1H); 7.7 s (1H); 7.2 s (5H); 4.7-4.3 m (3H); 4.2 q (2H); 3.9-3.1 m (5H); 2.9-2.4 m (4H); 1.9-1.4 m (2H); 1.2 t (3H); 1.0 t (3H) |
| 314 | 1 | H | H | " | $CH(CH_3)_2$ | H | $CH_2CH_2C_6H_5$ | 13.4 s (1H); 7.7 s (1H); 7.2 s (5H); 4.7-4.3 m (3H); 3.9-3.1 m (4H); 2.9-2.4 m (4H); 1.9-1.4 m (2H); 1.0 d (6H) |
| 315 | 1 | H | $C_2H_5$ | " | $CH(CH_3)_2$ | H | $CH_2CH_2C_6H_5$ | 13.4 s (1H); 7.7 s (1H); 7.2 s (5H); 4.7-4.3 m (3H); 4.2 q (2H); 3.9-3.0 m (4H); 2.9-2.4 m (4H); 1.9-1.4 m (2H); 1.2 t (3H); 1.0 d (6H) |
| 316 | 1 | H | H | " | $CH_2CH_2CH_3$ | H | $CH_2CH_2C_6H_4$-4-F | 13.4 s (1H); 7.7 s (1H); 7.4-6.9 m (4H); 4.7-4.3 m (3H); 3.9-3.0 m (5H); 2.9-2.4 m (4H); 1.9-1.4 m (4H); 1.0 t (3H) |

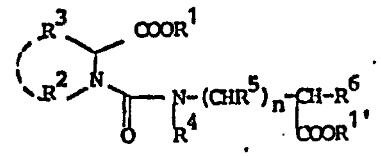

| | n | $R^1$ | $R^{1'}$ | $R^2$ — $R^3$ | $R^4$ | $R^5$ | $R^6$ | |
|---|---|---|---|---|---|---|---|---|
| 317 | 1 | H | H | 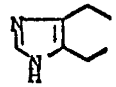 | $CH_2CH_2CH_2CH_3$ | H | $CH_2CH_2C_6H_3-(OCH_3)_2-3.4$ | 13.4 s (1H); 7.7 s (1H); 6.9-6.2 m (3H); 4.7-4.3 m (3H); 3.9-3.0 m (5H); 3.9 s (6H); 2.9-2.4 m (4H); 1.9-1.4 m (6H); 1.0 t (3H) |
| 318 | 1 | H | H | " | 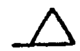 | H | $CH_2CH_2CH_2CH_3$ | 13.4 s (1H); 7.7 s (1H); 4.7-4.3 m (3H); 3.9-3.0 m (4H); 2.9-2.4 m (2H); 1.9-1.4 m (6H); 1.1-0.5 t + m (7H) |
| 319 | 1 | H | H | " | $CH_2-C{\equiv}CH$ | H | $CH_2-S-C_6H_5$ | 13.4 s (1H); 7.7 s (1H); 7.2 s (5H); 4.8-4.0 m (5H); 3.9-3.0 m (3H); 2.9-2.4 m (4H); 1.8 s (1H) |
| 320 | 1 | H | H | " | $CH_2-CH=CH_2$ | $CH_3$ | $CH_2CH_2-C_6H_4-4-OCH_3$ | 13.4 s (1H); 7.7 s (1H); 6.8-6.3 m (4H); 5.8 m (1H); 5.0-4.1 m (7H); 3.9 s (3H); 3.9-3.0 m (2H); 2.9-2.4 m (4H); 1.0 d (3H) |
| 321 | 1 | H | $C_2H_5$ | " | $C_2H_5$ | H | $CH_2NH-COC_6H_5$ | 13.4 s (1H);7.9-7.4 m (6H); 4.7-4.3 m (3H); 4.2 q (2H); 3.9-3.1 m (7H); 2.9-2.5 m (2H); 1.3 t (3H); 1.0 t (3H) |
| 322 | 1 | H | H | " | $C_2H_5$ | H | $CH_2CH_2OC_6H_5$ | 13.4 s (1H); 7.7 s (1H); 7.1-6.6 m (5H); 4.7-4.3 m (3H); 3.9-3.1 m (7H); 2.9-2.5 m (2H); 1.9-1.4 m (2H); 1.0 t (3H) |

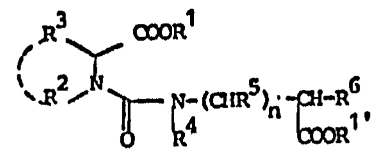

| | n | $R^1$ | $R^{1'}$ | $R^2$ — $R^3$ | $R^4$ | $R^5$ | $R^6$ | |
|---|---|---|---|---|---|---|---|---|
| 323 | 1 | H | H | 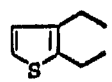 | $CH_3$ | H | $CH_2CH_2C_6H_5$ | 7.3-6.9 m (7H); 4.9-4.4 m (3H); 3.9-3.1 m (3H); 3.0-2.4 m (4H); 2.3 s (3H); 1.9-1.4 m (2H) |
| 324 | 1 | H | $C_2H_5$ | " | $CH_3$ | H | $CH_2CH_2C_6H_5$ | 7.3-6.9 m (7H); 4.9-4.4 m (3H); 4.2 q (2H); 3.9-3.1 m (3H); 3.0-2.4 m (4H); 2.3 s (3H); 1.9-1.4 m (2H); 1.2 t (3H) |
| 325 | 1 | H | H | " | $C_2H_5$ | H | $CH_2CH_2C_6H_5$ | 7.3-6.9 m (7H); 4.9-4.4 m (3H); 3.9-3.1 m (5H); 3.0-2.4 m (4H); 1.9-1.4 m (2H); 1.0 t (3H) |
| 326 | 1 | H | $C_2H_5$ | " | $C_2H_5$ | H | $CH_2CH_2C_6H_5$ | 7.3-6.9 m (7H); 4.9-4.4 m (3H); 4.2 q (2H); 3.9-3.1 m (5H); 3.0-2.4 m (4H); 1.9-1.4 m (2H); 1.2 t (3H); 1.0 t (3H) |
| 327 | 1 | H | H | " | $CH(CH_3)_2$ | H | $CH_2CH_2C_6H_5$ | 7.3-6.9 m (7H); 4.9-4.4 m (3H); 3.9-3.1 m (4H); 3.0-2.4 m (4H); 1.9-1.4 m(2H); 1.0 d (6H) |
| 328 | 1 | H | $C_2H_5$ | " | $CH(CH_3)_2$ | H | $CH_2CH_2C_6H_5$ | 7.3-6.9 m (7H); 4.9-4.4 m (3H); 4.2 q (2H); 3.9-3.1 m (4H); 3.0-2.4 m (4H); 1.9-1.4 m (2H); 1.2 t (3H); 1.0 d (6H) |
| 329 | 1 | H | H | " | $CH_2-CH=CH_2$ | H | $CH_2CH_2C_6H_5$ | 7.3-6.9 m (7H); 5.8 m (1H); 5.0-4.2 m (7H); 3.9-3.1 m (3H); 3.0-2.4 m (4H); 1.9-1.4 m (2H) |

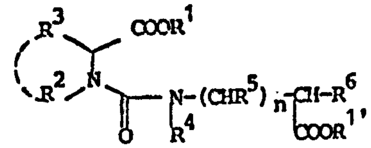

| | n | $R^1$ | $R^{1'}$ | $R^2$ — $R^3$ | $R^4$ | $R^5$ | $R^6$ | |
|---|---|---|---|---|---|---|---|---|
| 330 | 1 | H | H | 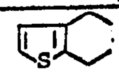 | $CH_2-C\equiv CH$ | H | $CH_2CH_2C_6H_5$ | 7.3-6.9 m (7H); 5.0-4.2 m (5H); 3.9-3.1 m (3H); 3.0-2.4 m (4H); 1.9-1.4 m (3H) |
| 331 | 1 | H | H | " | 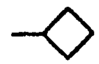 | H | $CH_2CH_2C_6H_5$ | 7.3-6.9 m (7H); 4.8-4.3 m (3H); 3.9-3.1 m (4H); 3.0-2.4 m (4H); 1.9-1.2 m (8H) |
| 332 | 1 | H | H | 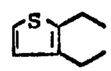 | $CH_3$ | H | $CH_2CH_2C_6H_5$ | 7.3-6.9 m (7H); 4.9-4.4 m (3H); 3.9-3.0 m (3H); 3.0-2.4 m (4H); 2.3 s (3H); 1.9-1.4 m (2H) |
| 333 | 1 | H | H | " | $C_2H_5$ | H | $CH_2CH_2C_6H_5$ | 7.3-6.9 m (7H); 4.9-4.4 m (3H); 3.9-3.1 m (5H); 3.0-2.4 m (4H); 1.9-1.4 m (2H); 1.0 t (3H) |
| 334 | 1 | H | $C_2H_5$ | " | $CH_3$ | H | $CH_2CH_2C_6H_5$ | 7.3-6.9 m (7H); 4.9-4.4 m (3H); 4.2 q (2H); 3.9-3.1 m (3H); 3.0-2.4 m (4H); 2.3 s (3H); 1.9-1.4 m (2H); 1.2 t(3H) |
| 335 | 1 | H | $C_2H_5$ | " | $C_2H_5$ | H | $CH_2CH_2C_6H_5$ | 7.3-6.9 m (7H); 4.9-4.4 m (3H); 4.2 q (2H); 3.9-3.1 m (5H); 3.0-2.4 m (4H); 1.9-1.4 m (2H); 1.2 t (3H); 1.0 t (3H) |

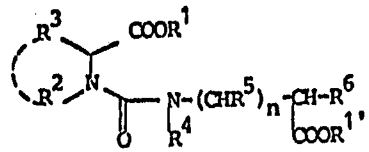

| | n | $R^1$ | $R^{1'}$ | $R^2$ — $R^3$ | $R^4$ | $R^5$ | $R^6$ | |
|---|---|---|---|---|---|---|---|---|
| 336 | 1 | H | H | 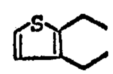 | $CH(CH_3)_2$ | H | $CH_2CH_2C_6H_5$ | 7.3-6.9 m (7H); 4.9-4.4 m (3H); 3.9-3.1 m (4H); 3.0-2.4 m (4H); 1.9-1.4 m (2H); 1.0 d (6H) |
| 337 | 1 | H | $C_2H_5$ | " | $CH(CH_3)_2$ | H | $CH_2CH_2C_6H_5$ | 7.3-6.9 m (7H); 4.9-4.4 m (3H); 4.2 q (2H); 3.9-3.1 m (4H); 3.0-2.4 m (4H); 1.9-1.4 m (2H); 1.2 t (3H); 1.0 d (6H) |
| 338 | 1 | H | H | " | $CH_2-CH=CH_2$ | H | $CH_2CH_2C_6H_5$ | 7.3-6.9 m (7H); 5.8 m (1H); 5.0-4.2 m (7H); 3.9-3.1 m (3H); 3.0-2.4 m (4H); 1.9-1.4 m (2H) |
| 339 | 1 | H | H | " | $CH_2-C\equiv CH$ | H | $CH_2CH_2C_6H_5$ | 7.3-6.9 m (7H); 5.0-4.2 m (5H); 3.9-3.1 m (4H); 3.0-2.4 m (4H); 1.9-1.4 m (3H) |
| 340 | 1 | H | H | " | 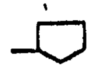 | H | $CH_2CH_2C_6H_5$ | 7.3-6.9 m (7H); 4.8-4.3 m (3H); 3.9-3.1 m (4H); 3.0-2.4 m (4H); 1.9-1.4 m (10H) |
| 341 | 1 | H | H | 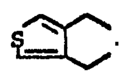 | $CH_3$ | H | $CH_2CH_2C_6H_5$ | 7.2 s (7H); 4.8-4.4 m (3H); 3.9-3.1 m (3H); 3.0-2.4 m (4H); 2.3 s (3H); 1.9-1.4 m (2H) |
| 342 | 1 | H | $C_2H_5$ | " | $CH_3$ | H | $CH_2CH_2C_6H_5$ | 7.2 s (7H); 4.8-4.3 m (3H); 4.2 q (2H); 3.9-3.1 m (3H); 3.0-2.4 m (4H); 2.3 s (3H); 1.9-1.4 m (2H); 1.2 t (3H) |

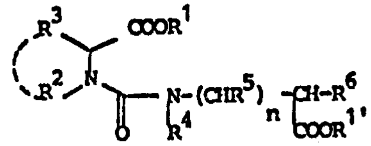

| | n | $R^1$ | $R^{1'}$ | $R^2$ — $R^3$ | $R^4$ | $R^5$ | $R^6$ | |
|---|---|---|---|---|---|---|---|---|
| 343 | 1 | H | H | 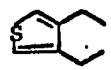 | $C_2H_5$ | H | $CH_2CH_2C_6H_5$ | 7.2 s (7H); 4.8-4.3 m (3H); 3.9-3.1 m (5H); 3.0-2.4 m (4H); 1.9-1.4 m (2H); 1.0 t (3H) |
| 344 | 1 | H | $C_2H_5$ | " | $C_2H_5$ | H | $CH_2CH_2C_6H_5$ | 7.2 s (7H); 4.8-4.3 m (3H); 4.2 q (2H); 3.9-3.1 m (5H); 3.0-2.4 m (4H); 1.9-1.4 m (2H); 1.2 t (3H); 1.0 t (3H) |
| 345 | 1 | H | H | " | $CH(CH_3)_2$ | H | $CH_2CH_2C_6H_5$ | 7.2 s (7H); 4.8-4.4 m (3H); 3.9-3.1 m (4H); 3.0-2.4 m (4H); 1.9-1.4 m (2H); 1.0 d (6H) |
| 346 | 1 | H | $C_2H_5$ | " | $CH(CH_3)_2$ | H | $CH_2CH_2C_6H_5$ | 7.2 s (7H); 4.8-4.4 m (3H); 4.2 q (2H); 3.9-3.1 m (4H); 3.0-2.4 m (4H); 1.9-1.4 m (2H); 1.3 t (3H); 1.0 d (6H) |
| 347 | 1 | H | H | " | n-$C_3H_7$ | H | $CH_2CH_2C_6H_5$ | 7.2 s (7H); 4.8-4.3 m (3H); 3.9-3.1 m (5H); 3.0-2.4 m (4H); 1.9-1.4 m (4H); 1.05 t (3H) |

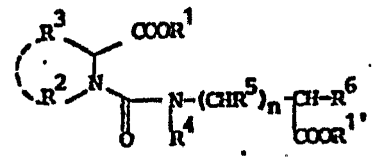

| | n | $R^1$ | $R^{1'}$ | $R^2$ — $R^3$ | $R^4$ | $R^5$ | $R^6$ | |
|---|---|---|---|---|---|---|---|---|
| 348 | H | H | H | $CH_2$-CH($OCH_3$)-$CH_2$ | $CH_3$ | H | H | 4.8-4.3 m (1H); 3.6-3.0 m+s (7H); 2.3 t (2H); 2.2 s (3H) 1.9-1.3 m (2H) |
| 349 | H | H | $C_2H_5$ | $CH_2$-CH ($OCH_3$)-$CH_2$ | $CH_3$ | H | H | 4.8-4.3 m (1H); 4.2 q (2H); 3.6-3.0 m+s (7H); 2.3 t (2H); 2.2 s (3H); 1.9-1.4 m (2H); 1.2 t (3H) |
| 350 | H | H | H | $CH_2$-CH($OCH_3$)-$CH_2$ | $C_2H_5$ | H | H | 4.8-4.3 m (1H); 3.8-3.0 m+s (9H); 2.3 t (2H); 1.9-1.4 m (2H); 1.2 t (3H) |
| 351 | H | H | H | $CH_2$-CH($OCH_3$)-$CH_2$ | CH($CH_3$)$_2$ | H | $CH_3$ | 4.8-4.3 m (1H); 3.8-2.9 m+s (10H) 1.9-1.4 m (2H); 1.0 3d (9H) |
| 352 | H | H | $C_2H_5$ | $CH_2$-CH($OCH_3$)-$CH_2$ | $C_2H_5$ | H | $CH_3$ | 4.8-4.3 m (1H); 4.2 q (2H); 3.8-2.9 m+s (11H); 1.9-1.4 m (2H); 1.2 t (3H); 1.0 d + t (6H) |
| 353 | H | H | H | $CH_2$-CH=CH | $C_2H_5$ | H | H | 6.4-5.5 m(3H); 4.3-3.1 m (6H); 2.3 t (2H); 1.0 t (3H) |
| 354 | H | H | $C_2H_5$ | $CH_2$-CH=CH | $CH_2$-CH=$CH_2$ | H | H | 6.4-5.5 m (4H); 4.9-3.1 m (10H); 4.2 q (2H); 2.3 t (2H); 1.2 t (3H) |
| 355 | H | H | H | $CH_2$-CH=CH | $C_2H_5$ | H | $CH_3$ | 6.5-5.5 m (3H); 4.3 - 3.0 m (7H); 1.0 d+t (6H) |

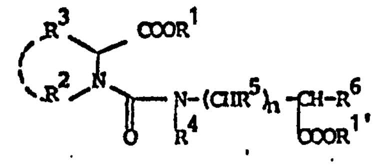

| | n | $R^1$ | $R^{1'}$ | $R^2$ — $R^3$ | $R^4$ | $R^5$ | $R^6$ | |
|---|---|---|---|---|---|---|---|---|
| 356 | H | H | H | $CH(CH_3)-CH_2-CH_2$ | $CH_3$ | H | H | 4.8-4.2 m (1H); 3.6-2.9 m (4H); 2.4 s (3H); 2.3 t (2H); 1.9-1.4 m (3H); 1.0 d (3H) |
| 357 | H | H | $C_2H_5$ | $CH(CH_3)-CH_2-CH_2$ | $C_2H_5$ | H | H | 4.8-4.3 m (1H); 4.2 q (2H); 3.8-3.0 m (6H); 2.3 t (2H); 1.9-1.4 m (3H); 1.2 t (3H); 0.95 d+t (6H) |
| 358 | H | H | H | $CH(CH_3)-CH_2-CH_2$ | $CH_2CH_2CH_3$ | H | $CH_3$ | 4.8-4.3 m (1H); 3.8-2.9 m (7H); 1.9-1.4 m (5H); 1.0 t+2d (9H) |
| 359 | H | H | $C_2H_5$ | $CH(CH_3)-CH_2-CH_2$ | $CH_2-C\equiv CH$ | H | $CH_3$ | 4.8-4.0 m (5H); 3.8-2.9 m (5H); 1.9-1.4 m (4H); 1.0 2d (6H) |
| 360 | H | H | H | $CH_2CH(C_6H_5)-CH_2$ | $CH_3$ | H | H | 7.3-6.9 m (5H); 4.8-4.3 m (1H); 3.6-2.9 m (5H); 2.3 t (2H); 2.2 s (3H); 1.9-1.4 m (2H) |
| 361 | H | H | H | $CH_2CH(C_6H_5)-CH_2$ | $C_2H_5$ | H | $CH_3$ | 7.3-6.9 m (5H); 4.8-4.3 m (1H); 3.6-2.8 m (8H); 1.9-1.4 m (2H); 1.05d (3H) |
| 362 | 1 | H | H | $CH(C_6H_5)-CH_2-CH_2$ | $C_2H_5$ | H | H | 7.3-6.9 m (5H); 4.8-4.3 m (1H); 3.8-2.9 m (7H); 2.3 t (2H) 1.9-1.4 m (2H); 1.0 t (2H) |

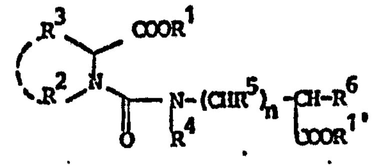

| | n | $R^1$ | $R^{1'}$ | $R^2$ — $R^3$ | $R^4$ | $R^5$ | $R^6$ | |
|---|---|---|---|---|---|---|---|---|
| 363 | 1 | H | H | $CH(C_6H_5)-CH_2-CH_2$ | $CH(CH_3)_2$ | H | H | 7.3-6.9 m (5H); 4.8-4.3 m (1H); 3.8-2.9 m (6H); 2.3 t (2H) 1.9-1.4 m (2H); 1.0d (6H) |
| 364 | 1 | H | H | $CH(C_6H_5)-CH_2-CH_2$ | 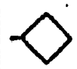 | H | $CH_3$ | 7.3-6.9 m (5H); 4.8-4.3 m (1H); 3.7-2.8 m (7H); 1.9-1.3 m (8H); 1.0d (3H) |
| 365 | 1 | H | $C_2H_5$ | $(CH_2)_4$ | $CH_3$ | H | H | 4.8-4.3 m (1H); 3.6-2.8 m (4H); 4.2 q (2H); 2.4s (3H); 2.3 t (2H); 1.9-1.4 m (6H); 1.2 t (3H) |
| 366 | 1 | H | H | $(CH_2)_4$ | $CH_3$ | H | $CH_3$ | 4.8-4.3 m (1H); 3.6-2.8 m (5H); 2.4 s (3H); 1.9-1.4 m (6H); 1.1 d (3H) |
| 367 | 1 | H | H | $(CH_2)_4$ | $C_2H_5$ | H | H | 4.8-4.3 m (1H); 3.6-2.8 m (6H); 2.3 t (2H); 1.9-1.4 m (6H); 1.1 t (3H) |
| 368 | 1 | H | H | $(CH_2)_4$ | $CH(CH_3)_2$ | H | $CH_3$ | 4.8-4.3 m (1H); 3.8-2.8 m (6H); 1.9-1.4 m (6H); 1.0 d (9H) |
| 369 | 1 | H | $C_2H_5$ | $(CH_2)_4$ | CH-C≡CH | H | H | 4.8-4.2 m (3H); 3.8-2.9 m (4H); 4.2 q (2H); 2.3 t (2H); 1.9-1.4 m (7H); 1.2 t (3H) |
| 370 | 1 | H | H | $(CH_2)_4$ | $C_4H_9$ | H | $CH_3$ | 4.8-4.3 m (1H); 3.8-2.9 m (7H); 1.9-1.4 m (10H); 1.0 d+t (6H) |

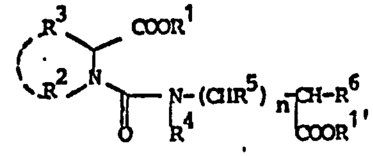

| | n | $R^1$ | $R^{1'}$ | $R^2$ — $R^3$ | $R^4$ | $R^5$ | $R^6$ | |
|---|---|---|---|---|---|---|---|---|
| 377 | 1 | H | H | $(CH_2)_5$ | $CH_3$ | H | H | 4.8-4.3 m (1H); 3.6-2.8 m (4H); 2.4 s (3H); 2.3 t (2H); 1.9-1.4 m (8H) |
| 378 | 1 | H | $C_2H_5$ | $(CH_2)_5$ | $CH_3$ | H | H | 4.8-4.0 m (3H); 3.6-2.8 m (4H); 2.4 s (3H); 2.3 t (2H); 1.9-1.4 m (8H); 1.2 t (3H) |
| 379 | 1 | H | H | $(CH_2)_5$ | $C_2H_5$ | H | H | 4.8-4.3 m (1H); 3.8-2.8 m (6H); 2.3 t (2H); 1.9-1.4 m (8H); 1.0 t (3H) |
| 380 | 1 | H | $C_2H_5$ | $(CH_2)_5$ | $C_2H_5$ | H | H | 4.8-4.3 m (1H); 4.2 q (2H); 3.8-2.8 m (6H); 2.3 t (2H); 1.9-1.4 m (8H); 1.2 t (3H); 1.0 t (3H) |
| 381 | 1 | H | H | $(CH_2)_5$ | $CH(CH_3)_2$ | H | $CH_3$ | 4.8-4.3 m (1H); 3.8-2.8 m (6H); 1.9-1.4 m (8H); 1.0 3d (9H) |
| 382 | 1 | H | H | $(CH_2)_5$ | $CH_2-CH=CH_2$ | H | $CH_3$ | 5.8m (1H); 5.0-4.1 m (5H); 3.8-2.9 m (5H); 1.9-1.4 m (8H); 1.0 d (3H) |
| 383 | 1 | H | $C_2H_5$ | $(CH_2)_5$ | 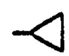 | H | $CH_3$ | 4.7-4.3 m (1H); 4.2 q (2H); 3.7-3.0 m (6H); 1.9-1.4 m (8H); 1.0-0.5 d+m (7H); 1.2 t (3H) |

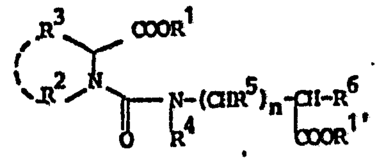

| | n | $R^1$ | $R^{1'}$ | $R^2$ — $R^3$ | $R^4$ | $R^5$ | $R^6$ | |
|---|---|---|---|---|---|---|---|---|
| 384 | 1 | H | H | $(CH_2)_5$ | $\underline{n}-C_4H_9$ | H | H | 4.7-4.3 m (1H); 3.9-3.0 m (6H); 2.3 t (2H); 1.9-1.4 m (12H); 1.0 t (3H) |
| 385 | 1 | H | H | 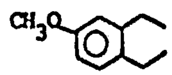 | $CH_3$ | H | H | 7.1-6.6 m (3H); 4.8-4.3 m (3H); 3.9 s (3H); 3.8-3.0 m (2H); 2.9-2.4 m (4H) 2.3 s (3H) |
| 386 | 1 | H | $C_2H_5$ | " | $C_2H_5$ | H | H | 7.1-6.6 m (3H); 4.8-4.3 m (3H); 4.2 q (2H); 3.9 s (3H); 3.8-3.0 m (4H); 2.9-2.4 m (4H); 1.2 t (3H); 1.0 t (4H) |
| 387 | 1 | H | H | " | $C_2H_5$ | H | $CH_3$ | 7.1-6.6 m (3H); 4.8-4.3 m (3H); 3.9 s (3H); 3.8-3.0 m (5H); 2.9-2.4 m (2H); 1.0 d+t (6H) |
| 388 | 1 | H | $C_2H_5$ | " | $CH(CH_3)_2$ | H | $CH_3$ | 7.1-6.6 m (3H); 4.8-4.3 m (3H); 3.9 s (3H); 3.9-3.1 m (4H); 4.2 q (2H); 2.9-2.4 m (2H); 1.2 t (3H); 1.0 d (9H) |
| 389 | 1 | H | H | " | $CH_2-CH=CH_2$ | H | $CH_3$ | 7.1-6.6 m (3H); 5.8 m (1H); 5.0-4.3 (5H); 3.9 s (3H); 3.8-2.9 m (3H); 2.8-2.4 m (2H); 1.05 d (3H) |
| 390 | 1 | H | H | 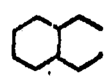 | $CH_3$ | H | H | 4.7-4.3 m (1H); 3.6-2.9 m (4H); 2.3 t (2H); 2.2 s (3H); 1.9-1.4 m (12H) |
| 391 | 1 | H | H | " | $C_2H_5$ | H | H | 4.7-4.3 m (1H); 3.8-2.9 m (6H); 2.3 t (2H); 1.9-1.4 m (12H); 1.1 t (3H) |

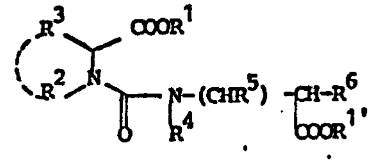

| | n | $R^1$ | $R^{1'}$ | $R^2$ — $R^3$ | $R^4$ | $R^5$ | $R^6$ | |
|---|---|---|---|---|---|---|---|---|
| 392 | 1 | H | $C_2H_5$ | " | $C_2H_5$ | H | H | 4.7-4.3 m (1H); 4.2 q (2H); 3.8-2.9 m (6H); 2.3 t (2H); 1.9-1.4 m (12H); 1.3 t (3H); 1.1 t (3H) |
| 393 | 1 | H | H | " | $CH(CH_3)_2$ | H | H | 4.7-4.3 m (1H); 4.0-3.1 m (5H); 2.3 t (2H); 1.9-1.4 m (12H); 1.0 d (6H) |
| 394 | 1 | H | H | " | $CH_2-CH=CH_2$ | H | $CH_3$ | 5.8 q (1H); 5.0-4.1 m (5H); 3.8-2.9 m (3H); 1.9-1.4 m (12H); 1.0 d (3H) |
| 395 | 1 | H | H | " | 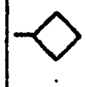 | | H | 4.7-4.3 m (1H); 3.9-2.9 m (5H); 2.3 t (2H); 1.9-1.4 m (18H) |
| 396 | 1 | H | H | 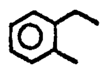 | $CH_3$ | H | H | 7.2-6.6 m (4H); 4.9 t (1H); 3.8-3.1 m (2H); 2.9-2.2 m (4H); 2.3 s (3H) |
| 397 | 1 | H | H | " | $C_2H_5$ | H | H | 7.2-6.6 m (4H); 4.9 t (1H); 3.8-3.1 m (4H); 2.9-2.2 m (4H); 1.1 t (3H) |
| 398 | 1 | H | H | " | $C_2H_5$ | H | $CH_3$ | 7.2-6.6 m (4H); 4.9 t (1H); 3.8-2.8 m (3H); 2.9-2.2 m (2H); 1.0 t+d (6H) |
| 399 | 1 | H | H | " | $CH(CH_3)_2$ | H | H | 7.2-66 m (4H); 4.9 t (1H); 3.9-2.9 m (3H); 2.9-2.2 m (4H); 1.0 d (6H) |

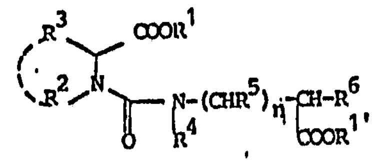

| | n | $R^1$ | $R^{1'}$ | $R^2$ — $R^3$ | $R^4$ | $R^5$ | $R^6$ | |
|---|---|---|---|---|---|---|---|---|
| 400 | 1 | H | H | " | $CH_2C\equiv CH$ | H | H | 7.2-6.6 m (4H); 4.9 t (1H); 4.3-3.2 m (4H); 2.9-2.2 m (4H); 1.8 s (1H) |
| 401 | 1 | H | H | " | $CH_2CH=CH_2$ | H | $CH_3$ | 7.2-6.6 m (4H); 5.8 m (1H); 5.0-4.2 m (5H); 3.9-2.9 m (3H); 1.0 d (3H) |
| 402 | 1 | H | H | 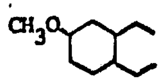 | $CH_3$ | H | H | 4.8-4.3 m (1H); 3.8-2.9 m+s (8H); 2.3 t (2H); 2.2 s (3H); 1.9-1.4 m (10H) |
| 403 | 1 | H | H | " | $C_2H_5$ | H | H | 4.8-4.3 m (1H); 3.8-2.9 m+s (10H); 2.3 t (2H); 1.9-1.4 m (10H); 1.0 t (3H) |
| 404 | 1 | H | H | " | $C_2H_5$ | H | $CH_3$ | 4.8-4.3 m (1H); 3.8-2.8 m+s (11H); 1.9-1.4 m (10H); 1.0 d+t (6H) |
| 405 | 1 | H | H | " | $CH(CH_3)_2$ | H | H | 4.8-4.3 m (1H); 3.8-2.9 m+s (9H); 2.3 t (2H); 1.9-1.4 m (10H); 1.0 d (6H) |
| 406 | 1 | H | H | " | 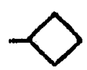 | H | $CH_3$ | 4.8-4.3 m (1H); 3.8-2.9 m+s (10H); 1.9-1.4 m (16H); 1.0 d (6H) |

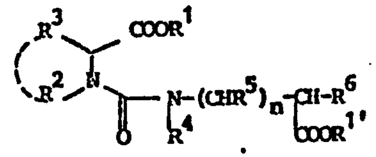

| | n | $R^1$ | $R^{1'}$ | $R^2$ — $R^3$ | $R^4$ | $R^5$ | $R^6$ | |
|---|---|---|---|---|---|---|---|---|
| 407 | 1 | H | H | 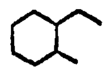 | $CH_3$ | H | H | 4.9-4.4 m(1H); 4.0-3.1 m (3H); 2.3 t (2H); 2.2 s (3H); 1.9-1.4 m (11H) |
| 408 | 1 | H | H | " | $C_2H_5$ | H | H | 4.9-4.4 m (1H); 4.0-3.1 m (5H); 2.3 t (2H); 1.9-1.4 m (11H) 1.1 t (3H) |
| 409 | 1 | H | H | " | $CH(CH_3)_2$ | H | H | 4.9-4.4 m (1H); 4.0-3.1 m (4H); 2.3 t (2H); 1.9-1.4 m (11H); 1.0 d (6H) |
| 410 | 1 | H | H | " | $C_2H_5$ | H | $CH_3$ | 4.9-4.4 m (1H); 4.0-3.0 m (7H); 1.9-1.4 m (11H); 1.0 d+t (6H) |
| 411 | 1 | H | H | " | $CH_2-CH=CH_2$ | H | $CH_3$ | 5.8 m (1H); 5.1-4.3 m (5H); 4.0-3.2 m (4H); 1.9-1.4 m (11H); 1.0 d (3H) |
| 412 | 1 | H | H | 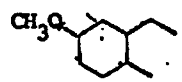 | $CH_3$ | H | H | 4.9-4.4 m (1H); 4.0-3.1 m+s (7H); 2.4 s (3H); 2.3 t (2H); 1.9-1.4 m (9H) |
| 413 | 1 | H | H | " | $C_2H_5$ | H | H | 4.9-4.4 m (1H); 4.0-3.1 m+s (9H); 2.3 t (2H); 1.9-1.4 m (9H); 1.0 t (3H) |
| 414 | 1 | H | H | " | $CH(CH_3)_2$ | H | H | 4.9-4.4 m (1H); 4.0-3.1 m+s (8H); 2.3 t (2H); 1.9-1.4 m (9H); 1.0 d (6H) |
| 415 | 1 | H | H | " | $C_2H_5$ | H | $CH_3$ | 4.9-4.4 m (1H); 4.0-3.0 m+s (11H); 1.9-1.4 m (9H); 1.0 d+t (6H) |
| 416 | 1 | H | H | " | $CH(CH_3)_2$ | H | $CH_3$ | 4.9-4.4 m (1H); 4.0-3.0 m+s (10H); 1.9-1.4 m (9H); 1.0 d (9H) |

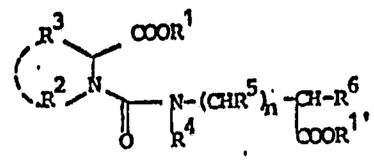

| | n | $R^1$ | $R^{1'}$ | $R^2$ — $R^3$ | $R^4$ | $R^5$ | $R^6$ | |
|---|---|---|---|---|---|---|---|---|
| 417 | 1 | H | H | 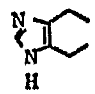 | $CH_3$ | H | H | 13.4 s (1H); 7.7 s (1H); 4.9-4.3 m (3H); 3.9-3.1 m (2H); 2.3 t (3H); 2.4 s (3H); 2.9-2.4 m (2H) |
| 418 | 1 | H | H | " | $C_2H_5$ | H | H | 13.4 s (1H); 7.7 s (1H); 4.8-4.3 m (3H); 3.8-3.1 m (4H); 2.9-2.5 m (2H); 2.3 t (2H); 1.0 t (3H) |
| 419 | 1 | H | H | " | $CH(CH_3)_2$ | H | H | 13.4 s (1H); 7.7 s (1H); 4.8-4.3 m (3H); 4.0-3.1 m (3H); 2.9-2.5 m(2H); 2.3 t (2H); 1.0 d (6H) |
| 420 | 1 | H | H | " | $C_2H_5$ | H | $CH_3$ | 13.4 s (1H); 7.7 s (1H); 4.8-4.3 m (3H); 4.0-3.0 m (5H); 2.9-2.5 m (2H); 1.0 d+t (6H) |
| 421 | 1 | H | H | " | 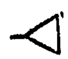 | H | H | 13.4 s (1H); 7.7 s (1H); 4.8-4.3 m (3H); 3.7-3.0 m (3H); 2.9-2.5 m (2H); 2.3 t (2H); 1.0-0.5 m (4H) |
| 422 | 1 | H | H | 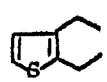 | $CH_3$ | H | H | 7.3-6.9 m (2H); 4.9-4.4 m (3H); 3.9-3.1 m (2H), 2.3 t (2H); 2.2 s (3H); 2.9-2.2 m (2H) |
| 423 | 1 | H | H | " | $C_2H_5$ | H | H | 7.3-6.9 m (2H); 4.9-4.4 m (3H); 3.9-3.1 m (4H); 2.3 t (2H); 2.9-2.4 m (2H); 1.1 t (3H) |
| 424 | 1 | H | H | " | $C_2H_5$ | H | H | 7.3-6.9 m (2H); 4.9-4.4 m (3H); 3.9-3.0 m (5H); 2.9-2.4 m (2H); 1.1 t+d (6H) |

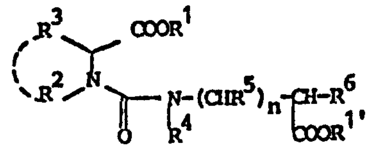

| | n | $R^1$ | $R^{1'}$ | $R^2$ — $R^3$ | $R^4$ | $R^5$ | $R^6$ | |
|---|---|---|---|---|---|---|---|---|
| 425 | 1 | H | H | 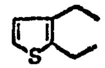 | $CH(CH_3)_2$ | H | $CH_3$ | 7.3-6.9 m (2H); 4.9-4.4 m (3H); 3.9-3.0 m (4H); 2.9-2.4 m (2H); 1.0 d (9H) |
| 426 | 1 | H | H | " | $CH(CH_3)_2$ | H | H | 7.3-6.9 m (2H); 4.9-4.4 m (3H); 3.9-3.0 m (3H); 2.9-2.4 m (2H); 2.3 t (2H); 1.0 d (6H) |
| 427 | 1 | H | H | 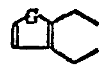 | $CH_3$ | H | H | 7.3-6.9 m (2H); 4.9-4.4 m (3H); 3.9-3.1 m (2H); 2.3 t (2H); 2.2 s (3H); 2.9-2.2 m (2H) |
| 428 | 1 | H | H | " | $C_2H_5$ | H | H | 7.3-6.9 m (2H); 4.9-4.4 m (3H); 3.9-3.1 m (4H); 2.3 t (2H); 2.9-2.4 m (2H); 1.1 t (3H) |
| 429 | 1 | H | H | " | $C_2H_5$ | H | $CH_3$ | 7.3-6.9 m (2H); 4.9-4.3 m (3H); 3.9-3.0 m (5H); 2.9-2.4 m (2H); 1.1 t+d (6H) |
| 430 | 1 | H | H | " | $CH(CH_3)_2$ | H | H | 7.3-6.9 m (2H); 4.8-4.4 m (3H); 3.8-3.0 m (3H); 2.9-2.4 m (2H); 2.3 t (2H); 1.0 d (6H) |
| 431 | 1 | H | H | " | $CH(CH_3)_2$ | H | $CH_3$ | 7.3-6.9 m (2H); 4.9-4.4 m (3H); 3.9-3.1 m (4H); 2.9-2.4 m (2H); 1.0 d (9H). |

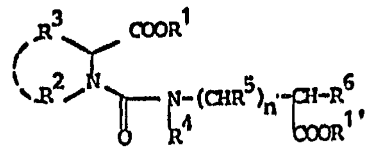

| | n | $R^1$ | $R^{1'}$ | $R^2$ — $R^3$ | $R^4$ | $R^5$ | $R^6$ | |
|---|---|---|---|---|---|---|---|---|
| 432 | 1 | H | H | 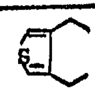 | $C_2H_5$ | H | H | 7.2 s (2H); 4.9-4.4 m (3H); 3.9-3.1 m (4H); 2.3 t (2H); 2.9-2.4 m (2H); 1.0 t (3H) |
| 433 | 1 | H | H | " | $C_2H_5$ | H | $CH_3$ | 7.2s (2H); 4.9-4.4 m (3H); 3.9-3.0 m (5H); 2.9-2.4 m (2H); 1.1 t+d (6H) |
| 434 | 1 | H | H | " | $CH(CH_3)_2$ | H | H | 7.2 s (2H); 4.9-4.4 m (3H); 3.9-3.2 m (3H); 2.9-2.4 m (2H); 2.3 t (2H); 1.0 d (6H) |
| 435 | 1 | H | H | " | $CH(CH_3)_2$ | H | $CH_3$ | 7.2 s (2H); 4.9-4.4 m (3H); 3.9-3.1 m (4H); 2.9-2.4 m (2H); 1.1 d (9H) |
| 436 | 1 | H | H | 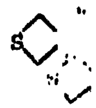 | $CH_3$ | H | H | 4.8-4.2 m (3H); 3.6-3.0 m (2H); 2.7-2.2 m (4H); 2.4 s (3H) |
| 437 | 1 | H | $C_2H_5$ | " | $CH_3$ | H | H | 4.8-4.2 m (5H); 3.6-3.0 m (2H); 2.7-2.2 m (4H); 2.4 s (3H); 1.2 t (3H) |
| 438 | 1 | H | H | " | $C_2H_5$ | H | $CH_3$ | 4.8-4.2 m (3H); 3.7-3.0 m (5H); 2.7-2.4 m (2H); 1.0 t+d (6H) |

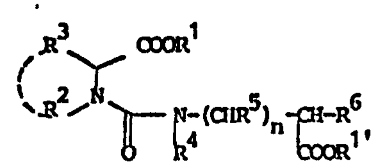

| | n | $R^1$ | $R^{1'}$ | $R^2$ — $R^3$ | $R^4$ | $R^5$ | $R^6$ | |
|---|---|---|---|---|---|---|---|---|
| 439 | 1 | H | $C_2H_5$ | 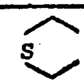 | $CH(CH_3)_2$ | H | H | 4.8-4.4 m (3H); 4.2 q (2H); 3.9-3.0 m (4H); 2.7-2.4 m (2H); 1.0 d (9H); 1.2 t (3H) |
| 440 | 1 | H | H | " | $CH_3$ | H | $CH_2CH_2C_6H_5$ | 7.2 s (5H); 4.8-4.2 m (3H); 3.9-3.0 m (3H); 2.9-2.2 m (4H); 1.9-1.4 m (2H); 2.3 s (3H); |
| 441 | 1 | H | $C_2H_5$ | " | $C_2H_5$ | H | $CH_2$ $CH_2C_6H_5$ | 7.2 s (5H); 4.8-4.3 m (3H) 4.2 q (2H); 3.9-3.0 m (5H); 2.9-2.2 m (4H); 1.9-1.4 m (2H); 1.2 t (3H); 1.0 t (3H) |
| 442 | 1 | H | H | " | $CH_2CH{=}CH_2$ | H | $CH_2CH_2C_6H_4$-4-F | 7.4-6.9 m (4H); 5.8 m (1H); 5.1-4.2 (5H); 3.9-3.0 m(3H); 2.9-2.2 m (4H); 1.9-1.4 m (2H) |
| 443 | 1 | H | $C_2H_5$ | " | $CH_2{-}C{\equiv}CH$ | H | $CH_2CH_2C_6H_4$-4-$OCH_3$ | 6.9-6.3 m (4H); 4.8-4.0 m (5H); 3.9-3.1 m (3H); 3.9 s (3H); 2.9-2.2 m (4H); 1.9-1.4 m (3H); 1.2 t (3H); 4.2 q (2H) |
| 444 | 1 | H | H | " | 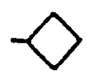 | $CH_3$ | $CH_2$ 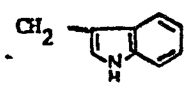 | 8.0 s (1H); 7.6-6.8 m (5H); 4.8-4.0 m (3H); 3.8-3.1 m (3H); 2.9-2.2 m (4H); 1.9-1.4 m (6H); 1.0 d (3H) |
| 445 | 1 | H | H | 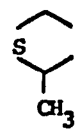 | $CH_3$ | H | $CH_3$ | 5.0-4.3 m (2H); 3.6-3.0 m (3H); 2.9-2.2 m (2H); 2.3 s (3H); 1.2 d (3H); 1.0 d (3H) |

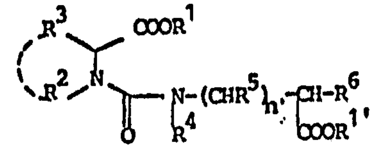

| | n | $R^1$ | $R^{1'}$ | $R^2$ — $R^3$ | $R^4$ | $R^5$ | $R^{6'}$ | |
|---|---|---|---|---|---|---|---|---|
| 446 | 1 | H | $C_2H_5$ | 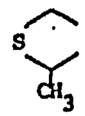 | $C_2H_5$ | H | $C_2H_5$ | 5.0-4.3 m (2H); 4.2 q (2H); 3.9-3.1 m (5H); 2.9-2.2 (2H); 1.9-1.4 m (2H); 1.2 d+t (6H); 1.0 t (6H) |
| 447 | 1 | H | H | " | $CH(CH_3)_2$ | H | $CH_2CH_2C_6H_5$ | 7.2 s (5H); 5.0-4.3 m (2H); 3.8-3.0 m (4H); 2.9-2.2 m (4H); 1.9-1.4 m (2H); 1.2 d (3H); 1.0 d (6H) |
| 448 | 1 | H | H | " | $C_2H_5$ | H | $CH_2CH_2C_6H_5$ | 7.2 s (5H); 5.0-4.3 m (2H); 3.8-3.0 m (5H); 2.9-2.2 m (4H); 1.9-1.4 m (2H); 1.2 d (3H); 1.0 t (3H) |
| 449 | 1 | H | H | " | $CH_3$ | H | $CH_2CH_2C_6H_4$-4-F | 7.4-6.9 m (4H); 5.0-4.3 m (2H); 3.8-3.0 m (3H); 2.9-2.2 m (4H); 2.3 s (3H); 1.9-1.4 m (2H); 1.2 d (3H) |
| 450 | 1 | H | H | " | $C_2H_5$ | H | H | 5.0-4.3 (2H); 3.8-3.0 m (4H); 2.9-2.2 m (4H); 1.2 d (3H); 1.0 t (3H); |
| 451 | 1 | H | H | " | $CH(CH_3)_2$ | H | H | 5.0-4.3 m (2H); 3.9-3.1 m (3H); 2.9-2.2 m (4H); 1.2 d (3H); 0.9 d (6H) |
| 452 | 1 | H | $C_2H_5$ | " | $CH_3$ | $CH_3$ | $CH_3$ | 5.0-4.3 m (2H); 4.2 q (2H); 3.9-3.1 m (2H); 2.9-2.2 m (2H); 2.4 s (3H); 1.2 d+t (6H); 1.0 d (6H) |

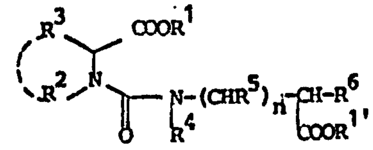

| | n | $R^1$ | $R^{1'}$ | $R^2$ — $R^3$ | $R^4$ | $R^5$ | $R^6$ | |
|---|---|---|---|---|---|---|---|---|
| 453 | 1 | H | H | 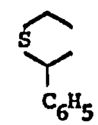 | $CH_3$ | H | H | 7.4-6.8 m (5H); 6.0 s (1H); 4.7-4.3 m (1H); 3.6-3.0 t (2H); 2.9-2.2 m (4H); 2.4 s (3H) |
| 454 | 1 | H | $C_2H_5$ | " | $C_2H_5$ | H | $CH_3$ | 7.4-6.8 m (5H); 6.0 s (1H); 4.7-4.3 (1H); 4.2 q (2H); 3.6-3.0 m (5H); 2.9-2.2 m (2H); 1.2 t (3H); 1.0 d + t (3H) |
| 455 | 1 | H | H | " | $C_2H_5$ | H | $CH_2CH_2C_6H_5$ | 7.4-6.8 m (10H); 6.0 s (1H); 4.7-4.3 m (1H); 3.8-3.0 m (5H); 2.9-2.2 m (4H); 1.9-1.4 m (2H); 1.0 t (3H) |
| 456 | 1 | H | H | " | $CH(CH_3)_2$ | H | $CH_2CH_2C_6H_5$ | 7.4-6.8 m (10H); 6.0 s (1H); 4.7-4.3 m (1H); 3.8-3.0 m (4H); 2.9-2.2 m (4H); 1.9-1.4 m (2H); 1.0 d (6H) |
| 457 | 1 | H | $C_2H_5$ | " | $CH_3$ | H | $CH_2CH_2C_6H_4$-4-F | 7.5-6.8 m (9H); 6.0 s (1H); 4.7-4.3 m (1H); 4.2 q (2H); 3.8-3.0 m (3H); 2.9-2.2 m (4H); 2.3 s (3H); 1.9-1.4 m (2H); 1.2 t (3H) |
| 458 | 1 | H | H | " | $CH_2CH_2CH_3$ | H | $CH_2CH_2$-$C_6H_4$-2-$CH_3$ | 7.5-6.9 m (9H); 6.0 s (1H); 4.7-4.3 m (1H); 3.8-3.0 m (5H); 2.9-2.2 m (4H); 2.1 s (3H); 1.9-1.4 m (4H); 1.0 t (3H) |
| 459 | 1 | H | $C_2H_5$ | " | 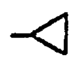 | H | $CH_2$ 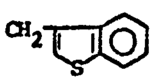 | 7.5-6.9 m (10H); 6.0 s (1H); 4.7-4.3 m (1H); 3.8-3.0 m (4H); 4.2 q (2H); 2.9-2.2 m (4H); 1.2 t (3H); 1.0-0.5 m (4H) |

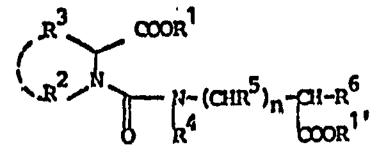

| | n | $R^1$ | $R^{1'}$ | $R^2$ — $R^3$ | $R^4$ | $R^5$ | $R^6$ | |
|---|---|---|---|---|---|---|---|---|
| 460 | 1 | H | H | 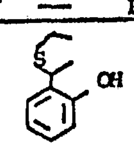 | $CH_3$ | H | H | 7.1-6.4 m (4H); 6.0 s (1H); 4.7-4.3 m (1H); 3.6-3.0 t (2H); 2.9-2.2 m (4H); 2.4 s (3H) |
| 461 | 1 | H | $C_2H_5$ | " | $CH_3$ | H | H | 7.1-6.4 m (4H); 6.0 s (1H); 4.7-4.3 m (1H); 4.2 q (2H); 3.6-3.0 t (2H); 2.9-2.2 m (4H); 2.4 s (3H); 1.2 t (3H) |
| 462 | 1 | H | H | " | $C_2H_5$ | H | $CH_2CH_2C_6H_5$ | 7.3-6.4 m (9H); 6.0 s (1H); 4.7-4.3 m (1H); 3.8-3.0 m (5H); 2.9-2.2 m (4H); 1.9-1.4 m (2H); 1.0 t (3H) |
| 463 | 1 | H | $C_2H_5$ | " | $CH_3$ | H | $CH_2CH_2C_6H_5$ | 7.3-6.4 m (9H); 6.0 s (1H); 4.7-4.3 m (1H); 4.2 q (2H); 3.8-3.0 m (3H); 2.9-2.2 m (4H); 1.9-1.4 m (2H); 2.2 s (3H); 1.2 t (3H) |
| 464 | 1 | H | H | " | $CH(CH_3)_2$ | H | $CH_2CH_2C_6H_5$ | 7.3-6.4 m (9H); 6.0 s (1H); 4.7-4.3 m (1H); 3.8-2.9 m (4H); 2.9-2.2 m (4H); 1.9-1.4 m (2H); 1.0 d (6H) |
| 465 | 1 | H | H | " | $CH_2-CH=CH_2$ | H | $CH_2CH_2C_6H_4-4-F$ | 7.3-6.4 m (8H); 6.0-5.6 m (2H); 5.0-4.1 m (5H); 3.6-3.1 m (3H); 2.9-2.2 m (4H); 1.9-1.4 m (2H) |
| 466 | 1 | H | $C_2H_5$ | " | $CH_2CH_2CH_3$ | H | $CH_2CH_2-C_6H_4-4-OCH_3$ | 7.1-6.3 m (8H); 6.0 s (1H); 4.7-4.3 m (1H); 4.2 q (2H); 3.9 s (3H); 3.8-3.0 m (5H); 2.9-2.2 m (4H); 1.9-1.4 m (2H); 1.2 t (3H); 1.0 t (3H) |

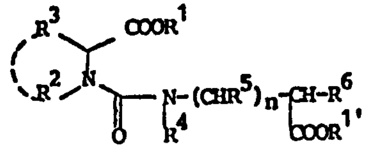

| | n | $R^1$ | $R^{1'}$ | $R^2$ — $R^3$ | $R^4$ | $R^5$ | $R^6$ | |
|---|---|---|---|---|---|---|---|---|
| 467 | 1 | H | H | 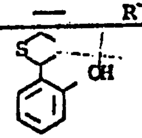 | $C_2H_5$ | H | $C_2H_5$ | 7.1-6.5 m (4H); 6.0 s (1H); 4.7-4.3 m (1H); 3.8-3.0 m (5H); 2.9-2.4 m (2H); 1.9-1.4 m (2H); 1.0 t (6H) |
| 468 | 1 | H | H | " | $C_2H_5$ | H | $n-C_4H_9$ | 7.1-6.5 m (4H); 6.0 s (1H); 4.7-4.3 m (1H); 3.8-3.0 m (5H); 2.9-2.4 m (2H); 1.9-1.4 m (6H); 1.0 t (6H) |
| 469 | 1 | H | H | " | $C_2H_5$ | H | $-CH_2CH_2-$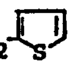 | 7.3-6.5 m (7H); 6.0 s (1H); 4.7-4.3 m (1H); 3.8-3.0 m (5H); 2.9-2.4 m (4H); 1.9-1.4 m (2H); 1.0 t (3H) |
| 470 | 1 | H | H | " | $C_2H_5$ | H | $-CH_2CH_2-C_6H_3-2.6-Cl_2$ | 7.4-6.5 m (7H); 6.0 s (1H); 4.7-4.3 m (1H); 3.8-3.0 m (5H); 2.9-2.4 m (4H); 1.9-1.4 m (2H); 1.0 t (3H) |
| 471 | 1 | H | $C_2H_5$ | " | 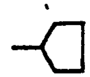 | $CH_3$ | $-CH_2CH_2-$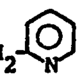 | 8.6-6.5 m (8H); 6.0 s (1H); 4.7-4.3 m (1H); 4.2 q (2H); 3.9-3.1 m (4H); 2.9-2.2 m (4H); 1.9-1.4 m (10H); 1.2 t (3H); 1.0 d (3H) |
| 472 | 1 | H | H | " | $CH_3$ | H | $CH_3$ | 7.1-6.5 m (4H); 6.0 s (1H); 4.7-4.3 m (1H); 3.6-3.1 m (3H); 2.9-2.4 m (2H); 2.3 s (3H); 1.0 d (3H) |
| 473 | 1 | H | $C_2H_5$ | " | $CH_3$ | H | $CH_3$ | 7.1-6.5 m (4H); 6.0 s (1H); 4.7-4.3 m (1H); 4.2 q (2H); 3.6-3.1 m (3H); 2.9-2.4 m (2H); 2.3 s (3H); 1.0 d (3H) |

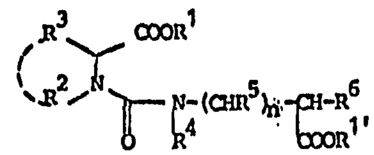

| | n | $R^1$ | $R^{1'}$ | $R^2$ — $R^3$ | $R^4$ | $R^5$ | $R^6$ | |
|---|---|---|---|---|---|---|---|---|
| 474 | 1 | H | $C_2H_5$ | 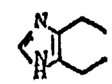 | $C_2H_5$ | H | H | 13.4 s (1H); 7.7 s (1H); 4.9-4.3 m (3H); 4.2 q (2H); 3.9-3.1 m (4H); 2.9-2.5 m (2H); 2.3 t (2H); 1.2 t (3H); 1.0 t (3H) |
| 475 | 1 | H | $C_2H_5$ | 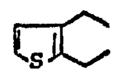 | $C_2H_5$ | H | H | 7.3-6.9 m (2H); 4.9-4.4 m (3H); 4.2 q (2H); 3.9-3.1 m (4H); 2.3 t (2H); 2.9-2.4 m (2H); 1.2 t (3H); 1.0 t (3H) |
| 476 | 1 | H | $C_2H_5$ | 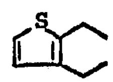 | $C_2H_5$ | H | H | 7.3-6.9 m (2H); 4.8-4.4 m (3H); 4.2 q (2H); 3.9-3.1 m (4H) 2.9-2.5 m (2H); 2.3 t (2H); 1.2 t (3H); 1.0 t (3H) |
| 477 | 1 | H | $C_2H_5$ | 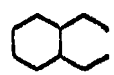 | $CH_3$ | H | H | 4.7-4.3 m (1H); 4.2 q (2H); 3.6-2.9m (4H); 2.3 t (2H); 2.2 s (3H); 1.9-1.4 m (12H); 1.1 t (3H) |
| 478 | 1 | H | $C_2H_5$ | " | $C_2H_5$ | H | $CH_3$ | 4.7-4.3 m (1H); 4.2 q (2H); 3.8-2.9m (7H); 1.9-1.4 m (12H); 1.2 t (3H); 1.1 d + t (6H) |
| 479 | 1 | H | $C_2H_5$ | " | $CH(CH_3)_2$ | H | H | 4.7-4.3 m (1H); 4.2 q (2H); 4.0-3.1m (5H); 2.3 t (2H); 1.9-1.4 m (12H); 1.2 t (3H); 1.0 d (6H) |
| 480 | 1 | H | $C_2H_5$ | " | $CH_3$ | H | $CH_3$ | 4.7-4.3 m (1H); 4.2 q (2H); 3.8-2.9m (5H); 2.2 s (3H); 1.9-1.4 m (12H); 1.2 t (3H); 1.1 d (3H) |

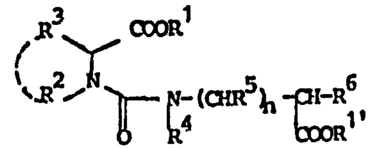

| | n | $R^1$ | $R^{1'}$ | $R^2$ — $R^3$ | $R^4$ | $R^5$ | $R^6$ | |
|---|---|---|---|---|---|---|---|---|
| 481 | 1 | H | $C_2H_5$ | 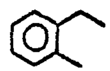 | $CH_3$ | H | H | 7.2-6.6 m (4H); 4.9 t (1H); 4.2 q (2H); 3.8-3.1 m (2H); 2.9-2.2 m (4H) 2.3 s (3H); 1.2 t (3H) |
| 482 | 1 | H | $C_2H_5$ | " | $C_2H_5$ | H | H | 7.2-6.6 m (4H); 4.9 t (1H); 4.2 q (2H); 3.8-3.1 m (4H); 2.9-2.2 m (4H) 1.2 t (3H); 1.0 t (3H) |
| 483 | 1 | H | $C_2H_5$ | " | $CH(CH_3)_2$ | H | H | 7.2-6.6 m (4H); 4.9 t (1H); 4.2 q (2H); 3.9-2.9 m (3H); 2.9-2.2 m (4H); 1.2 t (3H); 1.0 d (6H) |
| 484 | 1 | H | $C_2H_5$ | " | $CH_3$ | H | $CH_3$ | 7.2-6.6 m (4H); 4.9 t (1H); 4.2 q (2H); 3.8-3.1 m (2H); 2.9-2.3 m (3H) 2.3 s (3H); 1.2 t (3H); 1.0 d (3H) |
| 485 | 1 | H | $C_2H_5$ | 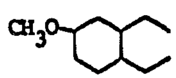 | $CH_3$ | H | H | 4.8-4.3 m (1H); 4.2 q (2H); 3.8-2.9m + s (8H); 2.3 s (3H); 2.2 t (2H); 1.9-1.4 m (10H); 1.2 t (3H) |
| 486 | 1 | H | $C_2H_5$ | " | $C_2H_5$ | H | H | 4.8-4.3 m (1H); 4.2 q (2H); 3.8-2.9 m + s (10H); 2.2 t (2H); 1.9-1.4 m (10H); 1.2 t (3H); 1.0 t (3H) |
| 487 | 1 | H | $C_2H_5$ | " | $CH(CH_3)_2$ | H | H | 4.8-4.3 m (1H); 4.2 q (2H); 3.9-2.9 m + s (9H); 2.2 t (2H); 1.9-1.4 m (10H); 1.2 t (3H); 0.9 d (6H) |

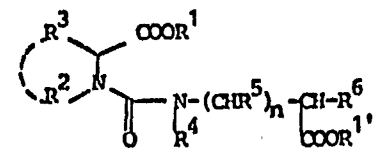

| | n | $R^1$ | $R^{1'}$ | $R^2$ — $R^3$ | $R^4$ | $R^5$ | $R^6$ | |
|---|---|---|---|---|---|---|---|---|
| 488 | 1 | H | $C_2H_5$ | 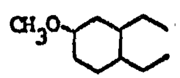 | $CH_3$ | H | $CH_3$ | 4.8-4.3 m (1H); 4.2 q (2H); 3.9-2.9m + s (9H); 2.3 s (3H); 1.9-1.4 m (10H); 1.2 t (3H); 1.0 d (3H) |
| 489 | 1 | H | $C_2H_5$ | 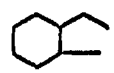 | $CH_3$ | H | H | 4.9-4.4 m (1H); 4.2 q (2H); 4.0-3.1m (3H); 2.3 t (2H); 2.2 s (3H); 1.9-1.4 m (11H); 1.2 t (3H) |
| 490 | 1 | H | $C_2H_5$ | " | $C_2H_5$ | H | H | 4.9-4.4 m (1H); 4.2 q (2H); 4.0-3.1m (5H); 2.3 t (2H); 1.9-1.4 m (11H); 1.2 t (3H); 1.0 t (3H) |
| 491 | 1 | H | $C_2H_5$ | " | $CH(CH_3)_2$ | H | H | 4.9-4.4 m (1H); 4.2 q (2H); 4.0-3.1 m (4H); 2.3 t (2H); 1.9-1.4 m (11H); 1.2 t (3H); 1.05 d (6H) |
| 492 | 1 | H | $C_2H_5$ | " | $CH_3$ | H | $CH_3$ | 4.9-4.4 m (1H); 4.2 q (2H); 4.0-3.0m (4H); 2.2 s (3H); 1.9-1.4 m (11H); 1.2 t (3H); 1.0 d (3H) |
| 493 | 1 | H | $C_2H_5$ | 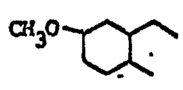 | $CH_3$ | H | H | 4.9-4.4 m (1H); 4.2 q (2H); 4.0-3.1m + s (7H); 2.4 s (3H); 2.3 t (2H); 1.9-1.4 m (9H); 1.2 t (3H) |
| 494 | 1 | H | $C_2H_5$ | " | $C_2H_5$ | H | H | 4.9-4.4 m (1H); 4.2 q (2H); 4.0-3.1 m + s (9H); 2.3 t (2H); 1.9-1.4 m (9H); 1.2 t (3H); 1.0 t (3H) |

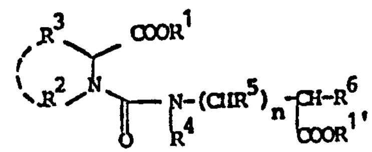

| | n | $R^1$ | $R^{1'}$ | $R^2$ — $R^3$ | $R^4$ | $R^5$ | $R^6$ | |
|---|---|---|---|---|---|---|---|---|
| 495 | 1 | H | $C_2H_5$ | 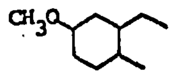 | $CH(CH_3)_2$ | H | H | 4.9-4.4 m (1H); 4.2 q (2H); 4.0-3.1 m + s (8H); 2.3 t (2H); 1.9-1.4 (9H) 1.2 t (3H); 0.9 d (6H) |
| 496 | 1 | H | $C_2H_5$ | " | $CH_3$ | H | $CH_3$ | 4.9-4.4 m (1H); 4.2 q (2H); 4.0-2.9 m + s (8H); 2.3 s (3H); 1.9-1.4 m (9H); 1.2 t (3H); 1.0 d (3H) |

Patentansprüche:

1. Verbindung der Formel I

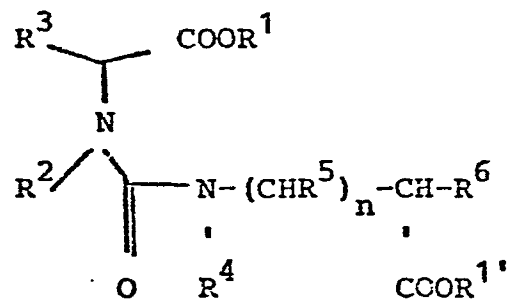

I

in welcher bedeuten:

n eine ganze Zahl zwischen 0 und 3 inclusiv,

$R^1$ und $R^{1'}$, gleich oder verschieden, Wasserstoff; Alkyl oder Alkenyl mit 1 - 8 C-Atomen; Phenyl oder Benzyl, jedes gewünschtenfalls mit Methyl, Halogen, Methoxy oder Nitro substituiert;

$R^2$ Wasserstoff, Alkyl oder Alkenyl mit 1 - 8 C-Atomen;

$R^3$ Wasserstoff; Alkyl mit 1 - 10 C-Atomen; Hydroxyalkyl, Alkoxyalkyl oder Aminoalkyl mit je 1 - 5 C-Atomen; Alkanoylaminoalkyl mit 1 - 7 C-Atomen; Guanidinoalkyl, Imidazolylalkyl, Indolylalkyl, Mercaptoalkyl oder Alkylthioalkyl mit je 1 - 6 Alkyl-C-Atomen; Phenylalkyl mit 1 - 5 Alkyl-C-Atomen; Hydroxyphenylalkyl mit 1 - 5 Alkyl-C-Atomen; Phenoxyalkyl oder Phenylthioalkyl mit je 1 - 4 Alkyl-C-Atomen oder $R^2$ und $R^3$ gemeinsam mit den sie tragenden C- und N-Atomen ein gesättigtes oder ungesättigtes 4 - 8 gliedriges monocyclisches oder 8 - 10 gliedriges bicyclisches Ringsystem bilden, das 1 - 2 Sauerstoff-, 1 - 2 Schwefel- und/oder 1 - 4 Stickstoffatome enthalten und durch Hydroxy, Alkoxy mit 1 - 3 C-Atomen, Alkyl

mit 1 - 3 C-Atomen oder Phenyl mono- oder disubstituiert sein kann;

$R^4$ Wasserstoff;

Alkyl, Alkenyl, Alkadienyl, Alkinyl, Alkeninyl oder Alkadiinyl mit 1 - 8 C-Atomen;

Cycloalkyl mit 3 - 6 C-Atomen;

Phenyl, Benzyl, Phenethyl oder Phenylpropyl, deren jedes durch Halogen, Hydroxy, Acetoxy, Carboxy, Carbonamido, Sulfonamido, Nitro, Methyl, Ethyl, Methoxy, Ethoxy oder Methylendioxy mono- oder disubstituiert sein kann;

$R^5$ Wasserstoff oder

Alkyl mit 1 - 5 C-Atomen, Hydroxy, Alkoxy mit 1 - 3 C-Atomen;

$R^6$ Wasserstoff;

Alkyl mit 1 - 12 C-Atomen;

Cycloalkyl mit 3 - 12 C-Atomen;

Alkenyl mit 1 - 12 C-Atomen;

Phenyl oder Naphthyl, deren jedes durch Halogen, Hydroxy, Acetoxy, Carboxy, Carbonamido, Sulfonamido, Nitro, Methyl, Ethyl, Methoxy, Ethoxy oder Methylendioxy mono- oder disubstituiert sein kann;

durch Halogen, Hydroxy, Alkoxy mit 1 - 3 C-Atomen, Phenoxy, Amino, Dialkylamino mit 1 - 6 C-Atomen, Alkanoyl-amino mit 1 - 3 C-Atomen, Mercapto, Alkylthio mit 1 - 3 C-Atomen, Phenylthio, Phenylsulfinyl, Phenylsulfonyl, Phenyl, Biphenylyl, Naphthyl oder Heteroaryl substituiertes Alkyl mit 1 - 6 C-Atomen, wobei das Phenyl oder Naphthyl seinerseits mit Halogen, Methyl, Ethyl, Methoxy, Ethoxy, Nitro, Amino, Alkylamino, Dialkylamino, Acetylamino, Cyano, Methylendioxy oder Sulfonamido mono- oder disubstituiert und das Heteroaryl durch die genannten Substituenten und zusätzlich durch Phenyl substituiert sein kann
und deren Salze.

2. Verbindung der Formel I gemäß Anspruch 1, dadurch gekennzeichnet, daß die Substituenten
n = 0 bis 2,
$R^1$ und $R^{1'}$ Wasserstoff, Alkyl oder Alkenyl mit 1 bis 4 C-Atomen, Benzyl, ggf. im Phenylkern mit Methyl, Halogen, Methoxy- oder Nitro substituiert;
$R^2$ Wasserstoff, Alkyl, Alkenyl oder Alkinyl mit 1 bis 5 C-Atomen;
$R^3$ der Rest einer natürlichen Aminosäure, Acetylaminobutyl, Methoxymethyl, Methoxyethyl, Phenoxymethyl, Methylthiomethyl, Methylthioethyl oder Phenylthiomethyl;
$R^2$ und $R^3$ können gemeinsam mit dem sie tragenden Kohlenstoff- bzw. Stickstoffatom Teil eines gesättigten oder ungesättigten 4 bis 8-gliedrigen monocyclischen bzw. 8 bis 10-gliedrigen bicyclischen Ringsystems bedeuten, das außer Kohlenstoff auch noch jeweils ein Sauerstoff-, Schwefel und/oder 1 bis 3 Stickstoffatome enthalten kann,
$R^4$ Wasserstoff, geradkettiges oder verzweigtes Alkyl, Alkenyl oder Alkinyl mit 1 bis 5 C-Atomen, Cycloalkyl mit 3 bis 6 C-Atomen, Phenyl, Benzyl, Phenethyl;
$R^5$ Wasserstoff, Methyl, Ethyl, Hydroxy, Methoxy, Benzyl;
$R^6$ Wasserstoff, Alkyl mit 1 bis 8 Kohlenstoffatomen oder Phenyl, das durch Methyl, Halogen, Methoxy, Acetoxy, Nitro mono- oder disubstituiert sein kann;
mit Halogen, Hydroxy, Methoxy, Ethoxy, Phenoxy, Amino, Methylamino, Dimethylamino, Anilino, Acetylamino, Benzamido, Mercapto, Phenylthio, Phenylsulfinyl,

Phenylsulfonyl; ggf. durch Halogen, Methyl, Ethyl, Methoxy, Ethoxy, Nitro, Amino, Methylamino, Dimethylamino, Acetylamino, Cyano, Methylendioxy, Sulfonamido mono- oder disubstituiertem Phenyl, Biphenylyl, ggf. durch Halogen, Methyl, Methoxy und Phenyl substituiertem Heteroaryl substituiertes Alkyl mit 1 - 4 C-Atomen

bedeuten.

3. Verbindung der Formel I gemäß Anspruch 1, dadurch gekennzeichnet, daß die Substituenten

n = 0 oder 1,

$R^1$ und $R^{1'}$ Wasserstoff, Methyl, Ethyl, n-Butyl, t-Butyl, Benzyl, p-Nitrophenyl

$R^2$ Wasserstoff, Methyl, Ethyl, n-Butyl

$R^3$ der Rest einer natürlichen Aminosäure oder Acetylaminobutyl, Methoxymethyl, Methoxyethyl, Phenoxymethyl, Methylthiomethyl, Methylthioethyl, Phenylthiomethyl;

$R^2$ und $R^3$ können gemeinsam mit dem sie tragenden Kohlenstoff- bzw. Stickstoffatom Teil eines gesättigten oder ungesättigten 5 bis 7-gliedrigen monocyclischen bzw. 8 bis 10-gliedrigen bicyclischen Ringsystems bedeuten, daß außer Kohlenstoff- auch noch jeweils ein Sauerstoff- oder Schwefelatom und/oder 1 bis 2 Stickstoffatome enthalten kann;

$R^4$ Methyl, Ethyl, n-Propyl, n-Butyl, Isopropyl, Isobutyl, Cycloprcpyl, Cyclobutyl, Allyl, Butenyl, Propargyl, Butinyl, tert.-Butyl;

$R^5$ Wasserstoff, Methyl, Benzyl;

$R^6$ Wasserstoff, geradkettiges oder verzweigtes Alkyl oder Alkenyl mit 1 bis 6 C-Atomen oder Cycloalkyl mit 3 bis 6 C-Atomen;

mit Phenoxy, Ethoxy, Methoxy, Dimethylamino, Anilino, Benzamido, Phenylthio, Phenylsulfinyl, Phenylsulfonyl; ggf. durch Halogen, Methyl, Methoxy, Nitro, Amino, Methylamino, Dimethylamino, Acetylamino, Cyano, Methylendioxy mono- oder disubstituiertem Phenyl,

Biphenylyl; ggf. durch Chlor, Methyl, Methoxy oder Phenyl substituiertem Heteroaryl substituiertes Alkyl mit 1 - 3 C-Atomen
bedeuten.

4. Verbindung der Formel I gemäß den Ansprüchen 1 bis 3, dadurch gekennzeichnet, daß das Kohlenstoffatom, das den Substituenten $R^3$ trägt, die (S)-Konfiguration aufweist.

5. Verbindung der Formel I gemäß Anspruch 1, dadurch gekennzeichnet, daß n = 1, $R^1$ Wasserstoff, $R^2$ und $R^3$ gemeinsam mit den sie tragenden C- und N-Atomen das 1,2,3,4-Tetrahydroisochinolin-System, $R^4$ Ethyl, $R^5$ Wasserstoff und $R^6$ ß-Phenylethyl bedeuten.

6. Verbindung der Formel I gemäß Anspruch 1, dadurch gekennzeichnet, daß n = 1, $R^1$ Wasserstoff, $R^2$ und $R^3$ gemeinsam mit den sie tragenden C- und N-Atomen das Octahydroindol-System, $R^4$ Ethyl, $R^5$ Wasserstoff und $R^6$ ß-Phenylethyl bedeuten.

7. Verbindung der Formel I gemäß Anspruch 1, dadurch gekennzeichnet, daß n = 1, $R^1$ Wasserstoff, $R^2$ und $R^3$ gemeinsam mit den sie tragenden C- und N-Atomen das 2-Azabicyclo[3.3.0]octan-System, $R^4$ Ethyl, $R^5$ Wasserstoff und $R^6$ ß-Phenylethyl bedeuten.

8. Verfahren zur Herstellung einer Verbindung gemäß Anspruch 1, dadurch gekennzeichnet, daß man einen Aminosäureester der Formel II

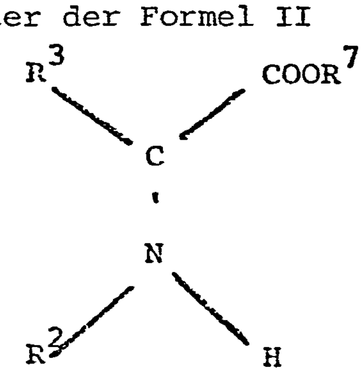

II

in der $R^7$ die gleiche Bedeutung wie $R^1$ hat, jedoch nicht Wasserstoff ist, mit Phosgen und danach mit einer Verbindung der Formel IV

$$R^4-NH-(CHR^5)_n-\underset{\displaystyle COOR^8}{\underset{|}{CHR^6}} \qquad IV$$

in der $R^8$ eine der Bedeutungen von $R^7$ hat, umsetzt,

oder eine Verbindung der Formel IV mit Phosgen und danach mit einer Verbindung der Formel II umsetzt,

und gegebenenfalls das erhaltene Produkt einer Hydrolyse unterwirft.

9. Mittel enthaltend eine Verbindung gemäß Anspruch 1.

10. Verwendung einer Verbindung gemäß Anspruch 1 als Heilmittel.

11. Verbindung gemäß Anspruch 1 zur Verwendung als Heilmittel.

0074070
HOE 81/F 228

Patentansprüche für Österreich:

1. Verfahren zur Herstellung einer Verbindung der Formel I

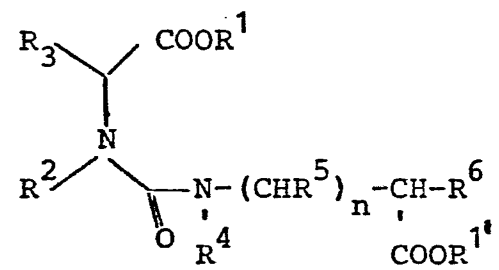

in welcher bedeuten:

n eine ganze Zahl zwischen 0 und 3 inklusiv,

$R^1$ und $R^{1'}$, gleich oder verschieden Wasserstoff; Alkyl oder Alkenyl mit 1 bis 8 C-Atomen; Phenyl oder Benzyl, jedes gewünschtenfalls mi- Methyl, Halogen, Methoxy oder Nitro substituiert;

$R^2$ Wasserstoff, Alkyl oder Alkenyl mit 1 bis 8 C-Atomen;

$R^3$ Wasserstoff; Alkyl mit 1 bis 10 C-Atomen; Hydroxyalkyl, Alkoxyalkyl oder Aminoalkyl mit je 1 bis 5 C-Atomen; Alkanoylaminoalkyl mit 1 bis 7 C-Atomen; Guanidinoalkyl, Imidazolylalkyl, Indolylalkyl, Mercaptoalkyl oder Alkylthioalkyl mit je 1 bis 6 Alkyl-C-Atomen; Phenylalkyl mit 1 bis 5 Alkyl-C-Atomen; Hydroxyphenylalkyl mit 1 bis 5 Alkyl-C-Atomen; Phenoxyalkyl oder Phenylthioalkyl mit je 1 bis 4 Alkyl-C-Atomen oder $R^2$ und $R^3$ gemeinsam mit den sie tragenden C- und N-Atomen ein gesättigtes oder ungesättigtes 4- bis 8-gliedriges monocyclisches oder 8- bis 10-gliedriges bicyclisches Ringsystem bilden, das 1 bis 2 Sauerstoff-, 1 bis 2 Schwefel- und/oder 1 bis 4 Stickstoffatome enthalten und durch Hydroxy, Alkoxy mit 1 bis 3 C-Atomen, Alkyl mit 1 bis 3 C-Atomen oder Phenyl mono- oder disubstituiert sein kann;

$R^4$ Wasserstoff;
Alkyl, Alkenyl, Alkadienyl, Alkinyl, Alkeninyl oder Alkadiinyl mit 1 bis 8 C-Atomen; Cycloalkyl mit 3 bis 6 C-Atomen;
Phenyl, Benzyl, Phenethyl oder Phenylpropyl, deren jedes durch Halogen, Hydroxy, Acetoxy, Carboxy, Carbonamido, Sulfonamido, Nitro, Methyl, Ethyl, Methoxy, Ethoxy oder Methylendioxy mono- oder disubstituiert sein kann;

$R^5$ Wasserstoff oder
Alkyl mit 1 bis 5 C-Atomen, Hydroxy, Alkoxy mit 1 bis 3 C-Atomen;

$R^6$ Wasserstoff;
Alkyl mit 1 bis 12 C-Atomen;
Cycloalkyl mit 3 bis 12 C-Atomen;
Alkenyl mit 1 bis 12 C-Atomen;
Phenyl oder Naphthyl, deren jedes durch Halogen, Hydroxy, Acetoxy, Carboxy, Carbonamido, Sulfonamido, Nitro, Methyl, Ethyl, Methoxy, Ethoxy oder Methylendioxy mono- oder disubstituiert sein kann;
durch Halogen, Hydroxy, Alkoxy mit 1 bis 3 C-Atomen, Phenoxy, Amino, Dialkylamino mit 1 bis 6 C-Atomen; Alkanoylamino mit 1 bis 3 C-Atomen, Mercapto, Alkylthio mit 1 bis 3 C-Atomen, Phenylthio, Phenylsulfinyl, Phenylsulfonyl, Phenyl, Biphenylyl, Naphthyl oder Heteroaryl substituiertes Alkyl mit 1 bis 6 C-Atomen, wobei das Phenyl oder Naphthyl seinerseits mit Halogen, Methyl, Ethyl, Methoxy, Ethoxy, Nitro, Amino, Alkylamino, Dialkylamino, Acetylamino, Cyano, Methyldioxy oder Sulfonamido mono- oder disubstituiert und das Heteroaryl durch die genannten Substituenten und zusätzlich durch Phenyl substituiert sein kann

und deren Salze, dadurch gekennzeichnet, daß man einen Aminosäureester der Formel II

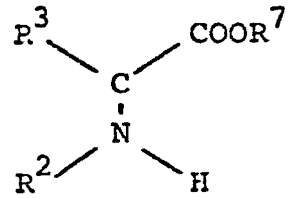 II

in der $R^7$ die gleiche Bedeutung wie $R^1$ hat, jedoch nicht Wasserstoff ist, mit Phosgen und danach mit einer Verbindung der Formel IV

$$R^4-NH-(CHR^5)_n-\underset{COOR^8}{\underset{|}{CHR^6}} \qquad IV$$

in der $R^8$ eine der Bedeutungen von $R^7$ hat, umsetzt, oder eine Verbindung der Formel IV mit Phosgen und danach mit einer Verbindung der Formel II umsetzt, gegebenenfalls das erhaltene Produkt einer Hydrolyse unterwirft und diese gegebenenfalls in ihre Salze überführt.

2. Verfahren gemäß Anspruch 1, dadurch gekennzeichnet, daß die Substituenten

n = 0 bis 2,

$R^1$ und $R^{1'}$ Wasserstoff, Alkyl oder Alkenyl mit 1 bis 4 C-Atomen, Benzyl, gegebenenfalls im Phenylkern mit Methyl, Halogen, Methoxy oder Nitro substituiert;

$R^2$ Wasserstoff, Alkyl, Alkenyl oder Alkinyl mit 1 bis 5 C-Atomen;

$R^3$ der Rest einer natürlichen Aminosäure, Acetylaminobutyl, Methoxymethyl, Methoxyethyl, Phenoxymethyl, Methylthiomethyl, Methylthioethyl oder Phenylthiomethyl;

$R^2$ und $R^3$ können gemeinsam mit dem sie tragenden Kohlenstoff- bzw. Stickstoffatom Teil eines gesättigten oder ungesättigten 4 bis 8-gliedrigen monocyclischen bzw. 8 bis 10-gliedrigen bicyclischen Ringsystems bedeuten, das außer Kohlenstoff auch noch jeweils ein

Sauerstoff-, Schwefel- und/oder 1 bis 3 Stickstoffatome enthalten kann,

$R^4$ Wasserstoff; geradkettiges oder verzweigtes Alkyl, Alkenyl oder Alkinyl mit 1 bis 5 C-Atomen; Cycloalkyl mit 3 bis 6 C-Atomen, Phenyl, Benzyl, Phenethyl;

$R^5$ Wasserstoff, Methyl, Ethyl, Hydroxy, Methoxy, Benzyl;

$R^6$ Wasserstoff, Alkyl mit 1 bis 8 Kohlenstoffatomen oder Phenyl, das durch Methyl, Halogen, Methoxy, Acetoxy, Nitro mono- oder disubstituiert sein kann; mit Halogen, Hydroxy, Methoxy, Ethoxy, Phenoxy, Amino, Methylamino, Dimethylamino, Anilino, Acetylamino, Benzamido, Mercapto, Phenylthio, Phenylsulfinyl, Phenylsulfonyl, ggf. durch Halogen, Methyl, Ethyl, Methoxy, Ethoxy, Nitro, Amino, Methylamino, Dimethylamino, Acetylamino, Cyano, Methylendioxy, Sulfonamido mono- oder disubstituiertem Phenyl, Biphenylyl, ggf. durch Halogen, Methyl, Methoxy und Phenyl substituierten Heteroaryl substituiertes Alkyl mit 1 bis 4 C-Atomen,

bedeuten.

3. Verfahren gemäß Anspruch 1, dadurch gekennzeichnet, daß die Substituenten

n = 0 oder 1,

$R^1$ und $R^{1'}$ Wasserstoff, Methyl, Ethyl, n-Butyl, t.-Butyl, Benzyl, p-Nitrophenyl,

$R^2$ Wasserstoff, Methyl, Ethyl, n-Butyl,

$R^3$ der Rest einer natürlichen Aminosäure oder Acetylaminobutyl, Methoxymethyl, Methoxyethyl, Phenoxymethyl, Methylthiomethyl, Methylthioethyl, Phenylthiomethyl;

$R^2$ und $R^3$ können gemeinsam mit dem sie tragenden Kohlenstoff- bzw. Stickstoffatom Teil eines gesättigten 5 bis 7-gliedrigen monocyclischen bzw. 8 bis 10-gliedrigen bicyclischen Ringsystems bedeuten, daß außer Kohlenstoff- auch noch jeweils ein Sauerstoff-

oder Schwefelatom und/oder 1 bis 2 Stickstoffatome enthalten kann;

$R^4$ Methyl, Ethyl, n-Propyl, n-Butyl, Isopropyl, Isobutyl, Cyclopropyl, Cyclobutyl, Allyl, Butenyl, Propargyl, Butinyl, tert. Butyl;

$R^5$ Wasserstoff, Methyl, Benzyl,

$R^6$ Wasserstoff, geradkettiges oder verzweigtes Alkyl oder Alkenyl mit 1 bis 6 C-Atomen oder Cycloalkyl mit 3 bis 6 C-Atomen; mit Methoxy, Ethoxy, Phenoxy, Dimethylamino, Anilino, Benzamido, Phenylthio, Pheynsulfinyl, Phenylsulfonyl ggf. durch Halogen, Methyl, Methoxy, Nitro, Amino, Methylamino, Dimethylamino, Acetylamino, Cyano, Methylendioxy mono- oder disubstituiertem Phenyl; Biphenylyl; ggf. durch Chlor, Methyl, Methoxy oder Phenyl substituiertem Heteroaryl substituiertes Alkyl mit 1 bis 3 C-Atomen,

bedeuten.

4. Verfahren gemäß den Ansprüchen 1 bis 3, dadurch gekennzeichnet, daß das Kohlenstoffatom, das den Substituenten $R^3$ trägt, die (S)-Konfiguration aufweist.

5. Verfahren gemäß Anspruch 1, dadurch gekennzeichnet, daß n = 1, $R^1$ Wasserstoff, $R^2$ und $R^3$ gemeinsam mit den sie tragenden C- und N-Atomen das 1,2,3,4-Tetrahydroisochinolin-System, $R^4$ Ethyl, $R^5$ Wasserstoff und $R^6$ ß-Phenylethyl bedeuten.

6. Verfahren gemäß Anspruch 1, dadurch gekennzeichnet, daß n = 1, $R^1$ Wasserstoff, $R^2$ und $R^3$ gemeinsam mit den sie tragenden C- und N-Atomen das Octahydroindol-System, $R^4$ Ethyl, $R^5$ Wasserstoff und $R^6$ ß-Phenylethyl bedeuten.

7. Verfahren gemäß Anspruch 1, dadurch gekennzeichnet, daß n = 1, $R^1$ Wasserstoff, $R^2$ und $R^3$ gemeinsam mit den sie tragenden C- und N-Atomen das 2-Azabicyclo[3.3.0]octan-

System, $R^4$ Ethyl, $R^5$ Wasserstoff und $R^6$ ß-Phenylethyl bedeuten.

8. Mittel enthaltend eine Verbindung der Formel I, in welcher n, $R^1$, $R^{1'}$, $R^2$, $R^3$, $R^4$, $R^5$ und $R^6$ die in Anspruch 1 genannten Bedeutungen haben.

0074070 Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 82108019.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | EP - A2 - 0 018 549 (TANABE)<br>* Zusammenfassung; Beispiele *<br>-- | 1,2,4,8,9,11 |
| P,A | EP - A1 - 0 049 605 (WARNER-LAMBERT) (14-04-1982)<br>* Zusammenfassung *<br>-- | 1,2,4,9,11 |
| P,A | EP - A1 - 0 049 589 (E.R. SQUIBB) (14-04-1982)<br>* Zusammenfassung *<br>-- | 1,9,11 |
| D,P,A | EP - A2 - 0 037 231 (WARNER-LAMBERT) (07-10-1981)<br>* Zusammenfassung *<br>-- | 1,9,11 |
| A | US - A - 4 284 779 (ONDETTI) (18-08-1981)<br>* Zusammenfassung *<br>-- | 1,9,11 |
| A | DE - A1 - 2 907 601 (E.R. SQUIBB)<br>* Patentansprüche 1,15; Seite 4, Zeile 35 - Seite 5, Zeile 13 *<br>-- | 1,8,9,11 |

**KLASSIFIKATION DER ANMELDUNG (Int Cl.³)**

C 07 D 217/26
C 07 D 209/42
C 07 D 209/52
C 07 D 209/20
C 07 C 127/19
C 07 C 127/15
C 07 C 149/437
C 07 D 333/24
C 07 D 213/55
C 07 D 213/40
C 07 D 231/12
C 07 D 409/12
C 07 D 403/12
C 07 D 207/16
C 07 D 207/22

**RECHERCHIERTE SACHGEBIETE (Int. Cl.³)**

C 07 D 217/00
C 07 D 209/00
C 07 C 127/00
C 07 C 149/00
C 07 D 333/00
C 07 D 213/00
C 07 D 409/00
C 07 D 403/00
C 07 D 207/00
C 07 D 211/00
C 07 D 401/00
C 07 D 417/00
C 07 D 223/00
C 07 D 471/00
C 07 D 495/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9 und 11
Unvollständig recherchierte Patentansprüche:
Nicht recherchierte Patentansprüche: 10
Grund für die Beschränkung der Recherche:

(Verfahren zur therapeutischen Behandlung des menschlichen oder tierischen Körpers, Artikel 52(4) EPÜ)

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| Recherchenort | Abschlußdatum der Recherche | Prüfer |
|---|---|---|
| WIEN | 03-12-1982 | ONDER |

EPA Form 1505.1 06.78

Europäisches Patentamt

**EUROPÄISCHER TEILRECHERCHENBERICHT**

Nummer der Anmeldung 0074070

EP 82108019.9

| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe, soweit erforderlich, der maßgeblichen Teile | betrifft Anspruch | |
| A | DE - A1 - 2 914 059 (SANTEN)<br>* Patentansprüche 1,5,7 *<br>-- | 1,8,9,11 | C 07 D 211/60<br>C 07 D 401/12<br>C 07 D 417/12<br>C 07 D 223/06<br>C 07 D 471/04<br>C 07 D 495/04<br>C 07 D 277/06<br>A 61 K 31/47<br>A 61 K 31/40<br>A 61 K 31/17<br>A 61 K 31/38<br>A 61 K 31/44<br>A 61 K 31/415<br>A 61 K 31/445<br>A 61 K 31/425 |
| A | DD - A - 135 387 (PARCOR)<br>* Zusammenfassung; Seite 2, Zeilen 6-15 *<br>-- | 1,9,11 | |
| A | EP - A1 - 0 001 813 (F. HOFFMANN-LA ROCHE)<br>* Zusammenfassung letzte und vorletzte Zeile; Seite 2, Formel II *<br>-- | 1 | RECHERCHIERTE SACHGEBIETE (Int. Cl.3) |
| A | DE - A1 - 2 624 094 (SUMITOMO)<br>* Patentansprüche 1,16 *<br>---- | 1,8 | C 07 D 277/00<br>C 07 D 231/00<br>C 07 D 215/00 |